# EUROPEAN PATENT APPLICATION

(11) **EP 4 512 813 A1**
(43) Date of publication of application: **26.02.2025**
(21) Application number: 24171601.8
(22) Date of filing: 22.04.2024
(51) Int. Cl.: C07F 7/08, C07C 211/61, C07C 255/58, H10K 50/15

(54) **FLUORENE-CONTAINING TRIARYLAMINE COMPOUND AND ORGANIC ELECTROLUMINESCENT DEVICE THEREOF**

(30) Priority: 21.08.2023 CN 202311054913
(71) Applicant: Changchun Hyperions Technology Co., Ltd., Bayhood Science and Technology Development Zone Changchun City Jilin 130000 (CN)
(72) Inventor: GUO, Jianhua, Changchun City, Jilin Province, 130000 (CN); HAN, Chunxue, Changchun City, Jilin Province, 130000 (CN); LIU, Xiqing, Changchun City, Jilin Province, 130000 (CN); ZHOU, Wenting, Changchun City, Jilin Province, 130000 (CN)
(74) Representative: Wallinger Ricker Schlotter Tostmann

(57) **Abstract**

Provided are a fluorene-containing triarylamine compound and an organic electroluminescent device thereof, which relate to the technical field of organic electroluminescent materials. The fluorene-containing triarylamine compound has a good hole transporting ability, a proper HOMO energy level, and good film formability and stability. When used in the organic electroluminescent device as a hole transporting material, the fluorene-containing triarylamine compound can balance electrons and holes in the device, improve recombination efficiency of electrons and holes in a light-emitting layer, effectively reduce a drive voltage of the device, improve luminescence efficiency of the device, and prolong a service life of the device and can be applied to fields such as display, lighting, and organic solar cells.

## Description

### TECHNICAL FIELD

The present disclosure relates to the technical field of organic electroluminescent materials and, in particular, to a fluorene-containing triarylamine compound and an organic electroluminescent device thereof.

### BACKGROUND

An organic light-emitting diode (OLED) is a technology for directly converting electrical energy into optical energy by using an organic semiconductor function material. An organic electroluminescent device prepared by using the OLED has many characteristics of an all solid state, a wide material selection range, a low operating temperature, high luminescence efficiency, a high color contrast, a fast response speed, thinness and lightness, a wide viewing angle, low power consumption, an ability to achieve flexible display, etc., is widely considered as the most promising display technology, and is widely applied to various fields such as display, lighting, and planar light sources.

The luminescence principle of the organic electroluminescent device is that electrons and holes are separately injected into a light-emitting layer from electrodes and moved under the action of an applied electric field to form excitons, and the excitons generate radiation and recombine in the light-emitting layer to emit light. The specific process is that carriers are separately injected into charge transporting layers from a positive electrode and a negative electrode; the carriers are transported in the charge transporting layers; positive and negative carriers are combined into excitons; and the excitons radiate and recombine to emit light. Organic electroluminescent devices manufactured based on this principle mostly adopt a sandwich structure, that is, an organic function layer is located between a cathode and an anode on two sides of a device. An organic layer may include a hole injection layer, a hole transporting layer, a hole assistance layer, a luminescence assistance layer, an electron blocking layer, a light-emitting layer, an electron buffer layer, a hole blocking layer, an electron transporting layer, an electron injection layer, a capping layer, and the like.

Primary functions of the hole transporting layer are to improve transporting efficiency of holes in the device and effectively block electrons in the light-emitting layer to implement the maximum recombination of carriers and meanwhile to reduce an energy barrier of holes in the injection process and improve injection efficiency of holes to improve the brightness, efficiency, and lifetime of the device. A good hole transporting material for organic electroluminescence not only needs to be able to form a uniform amorphous film without pinholes, which has very good thermal stability, but also needs to have a proper highest occupied molecular orbital (HOMO) energy level to ensure the effective injection and transport of holes from the electrode to the organic layer, in the organic layer, and via an interface between organic layers. Meanwhile, the good hole transporting material also needs to have a very high hole mobility. Conventional hole transporting materials still have the problems of a low hole mobility, energy level mismatching, and low thermal stability, causing an increased drive voltage, reduced luminescence efficiency, and a shortened service lifetime of the organic electroluminescent device. Meanwhile, the device generates Joule heat during operation, which often causes recrystallization of the material, and the crystallization destroys the uniformity of the film and also destroys good interface contact between the hole transporting layer and the anode and between the organic layers, resulting in the reduced luminescence efficiency and service lifetime of the device.

To further improve the performance of the OLED and solve the problems of the organic electroluminescent device, an organic electroluminescent material with better performance needs to be developed, where the hole transporting material is a priority.

### SUMMARY

In terms of the problems in the related art, the present disclosure provides a fluorene-containing triarylamine compound and an organic electroluminescent device thereof.

The present disclosure provides a fluorene-containing triarylamine compound, which is represented by the following Formula 1: 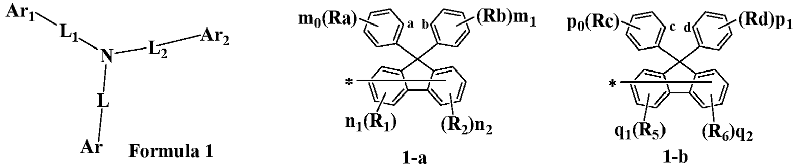 where Ar₁ is selected from a group represented by Formula 1-a;
"* " represents a linkage site to L₁;
Rₐ and R_{b} are each identically or differently selected from one of hydrogen, halogen, cyano, nitro, substituted or unsubstituted C1 to C25 alkyl, substituted or unsubstituted C3 to C25 cycloalkyl, and substituted or unsubstituted C6 to C60 aryl or a combination thereof, or adjacent two Rₐ or two R_{b} are bonded to each other to form a substituted or unsubstituted ring;
at least one of Rₐ and R_{b} is selected from substituted or unsubstituted C1 to C25 alkyl;
carbon atoms corresponding to a and b are separate, connected via a single bond, or bonded into a ring;
R₁ and R₂ are each identically or differently selected from one of hydrogen, halogen, cyano, nitro, substituted or unsubstituted C1 to C25 alkyl, substituted or unsubstituted silyl, substituted or unsubstituted C3 to C25 cycloalkyl, and substituted or unsubstituted C6 to C60 aryl, or adjacent two R₁ or two R₂ are bonded to each other to form a substituted or unsubstituted ring;
m₀ is selected from 1, 2, 3, 4, or 5; m₁ is selected from 1, 2, 3, 4, or 5;
n₁ is selected from 0, 1, 2, 3, or 4; n₂ is selected from 0, 1, 2, 3, or 4;
Ar₂ is selected from a group represented by Formula 1-b;
R_{c} and R_{d} are each identically or differently selected from one of hydrogen, halogen, cyano, nitro, substituted or unsubstituted C1 to C25 alkyl, substituted or unsubstituted C3 to C25 cycloalkyl, and substituted or unsubstituted C6 to C60 aryl or a combination thereof, or adjacent two R_{c} or two R_{d} are bonded to each other to form a substituted or unsubstituted ring;
at least one of R_{c} and R_{d} is selected from substituted or unsubstituted C1 to C25 alkyl;
carbon atoms corresponding to c and d are separate or bonded into a ring;
R₅ and R₆ are each identically or differently selected from one of hydrogen, halogen, cyano, nitro, substituted or unsubstituted C1 to C25 alkyl, substituted or unsubstituted silyl, substituted or unsubstituted C3 to C25 cycloalkyl, and substituted or unsubstituted C6 to C60 aryl, or adjacent two R₅ or two R₆ are bonded to each other to form a substituted or unsubstituted ring;
p₀ is selected from 1, 2, 3, 4, or 5; p₁ is selected from 1, 2, 3, 4, or 5;
q₁ is selected from 0, 1, 2, 3, or 4; q₂ is selected from 0, 1, 2, 3, or 4;
Ar is selected from one of substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, substituted or unsubstituted triphenylenyl, substituted or unsubstituted pyrenyl, substituted or unsubstituted 9,9-dimethylfluorenyl, substituted or unsubstituted 9,9-diphenylfluorenyl, substituted or unsubstituted 9,9-spirobifluorenyl, and substituted or unsubstituted spiroanthrafluorenyl; the substituent in the "substituted or unsubstituted" is selected from one of cyano, nitro, substituted or unsubstituted C1 to C25 alkyl, substituted or unsubstituted C3 to C25 cycloalkyl, and substituted or unsubstituted C6 to C30 aryl, or adjacent two groups are bonded to each other to form a substituted or unsubstituted benzene ring; and
L, L₁, and L₂ are independently selected from one of a single bond and substituted or unsubstituted C6 to C30 arylene.

Additionally, the present disclosure further provides an organic electroluminescent device. The organic electroluminescent device includes an anode, a cathode, and an organic layer, where the organic layer includes the preceding fluorene-containing triarylamine compound of the present disclosure.

Beneficial effects: The fluorene-containing triarylamine compound of Formula 1 in the present disclosure has a good hole transporting ability, a proper HOMO energy level, and a relatively high triplet energy level. When used in the organic electroluminescent device as a hole transporting material, the fluorene-containing triarylamine compound can balance electrons and holes in the device, improve recombination efficiency, effectively reduce a drive voltage of the organic electroluminescent device, and improve luminescence efficiency of the organic electroluminescent device. Additionally, the fluorene-containing triarylamine compound in the present disclosure also has relatively good stability and relatively good film formability and can effectively prolong a service lifetime of the organic electroluminescent device.

### DETAILED DESCRIPTION

The present disclosure is further described below in conjunction with embodiments. It is to be understood that the embodiments described below are intended to describe the present disclosure and not to limit the scope of the present disclosure. After the present disclosure is read, various equivalent modifications made by those skilled in the art to the present disclosure are all within the scope of the present application.

In a compound of the present disclosure, any atom not specifically designated as a particular isotope includes any stable isotope of the atom, and includes atoms presented at both its natural isotope abundance and unnatural abundance.

In the present disclosure, halogen includes fluorine, chlorine, bromine, and iodine.

In the present disclosure, "hydrogen" refers to isotopes with different numbers of neutrons, that is, includes protium, deuterium, and tritium.

In the present disclosure, an "unsubstituted ZZ group" in the "substituted or unsubstituted ZZ group" indicates that no hydrogen atom in the "ZZ group" is substituted with a substituent. For example, "unsubstituted aryl" in "substituted or unsubstituted C6 to C60 aryl" means that no hydrogen atom in "aryl" is substituted with a substituent. The same is true in other cases.

In the present disclosure, "CXX to CYY" in the "substituted or unsubstituted CXX to CYY ZZ group" represents the number of carbon atoms in the unsubstituted "ZZ group" and excludes the number of carbon atoms in a substituent when the "ZZ group" has the substituent. For example, "C6 to C60" in "substituted or unsubstituted C6 to C60 aryl" represents the number of carbon atoms in unsubstituted "aryl" and excludes the number of carbon atoms in a substituent when "aryl" has the substituent. The same is true in other cases.

In the present disclosure, when the position of a substituent on a ring is unfixed, it means that the substituent may be linked to any of the corresponding optional sites of the ring. For example, 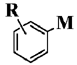 may represent 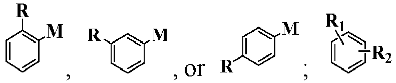 may represent 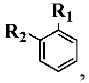 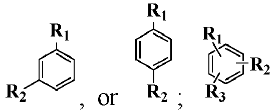 may represent 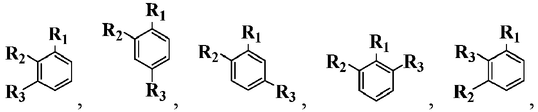 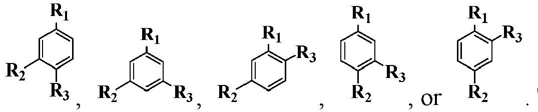 The same is true in other cases.

In the present disclosure, the expression that "adjacent two groups are bonded into a ring" means that adjacent groups are bonded to each other and optionally aromatized to form a substituted or unsubstituted hydrocarbon ring. The hydrocarbon ring may be an aromatic hydrocarbon ring. The hydrocarbon ring may be a monocyclic or polycyclic group. This is exemplified by the following formula:

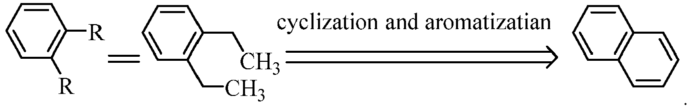

Additionally, a ring formed by bonding adjacent groups may be linked to another ring to form a spiro structure. This is exemplified by the following formulas:

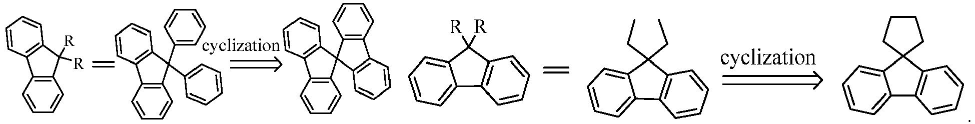

In the present disclosure, a ring formed by linking may be a three-membered ring, a four-membered ring, a five-membered ring, a six-membered ring, a seven-membered ring, an eight-membered ring, a fused ring, a spirocyclic ring, etc., such as, but not limited to, cyclopropane, cyclobutane, cyclopentane, cyclohexane, cycloheptane, cyclopentene, cyclohexene, benzene, naphthalene, phenanthrene, triphenylene, and fluorene.

In the present disclosure, "substituted" in a "substituted or unsubstituted" group means that at least one hydrogen atom in the group is substituted with a substituent. When multiple hydrogens are substituted with multiple substituents, the multiple substituents may be the same or different. The preceding hydrogen substituted with the substituent may be at any position. The substituent represented by "substituted" in "substituted or unsubstituted" includes the following groups: cyano, halogen, nitro, substituted or unsubstituted C1 to C15 alkoxy, substituted or unsubstituted C6 to C20 aryloxy, substituted or unsubstituted C1 to C15 alkyl, substituted or unsubstituted C3 to C15 cycloalkyl, substituted or unsubstituted C6 to C20 aryl, etc. Preferably, the substituent includes the following groups: deuterium, tritium, cyano, halogen, nitro, methyl, ethyl, propyl, butyl, pentyl, hexyl, cyclopropyl, cyclobutyl, cyclopentyl, cyclohexyl, adamantyl, norbornyl, camphanyl, isocamphanyl, fenchyl, phenyl, biphenyl, naphthyl, phenanthryl, triphenylenyl, anthryl, pyrenyl, fluorenyl, spirobifluorenyl, etc. Additionally, each of the above substituents may be substituted or unsubstituted. Adjacent two substituents may be bonded into a ring.

An alkyl group of the present disclosure refers to a hydrocarbyl group formed by removing one hydrogen atom from an alkane molecule. Alkyl may be linear alkyl or branched alkyl. When the number of carbon atoms in a chain alkyl group of the present disclosure is 3 or above, the chain alkyl group includes its isomers. For example, propyl includes n-propyl and isopropyl; butyl includes n-butyl, isobutyl, sec-butyl, and t-butyl; and so on. Examples of the alkyl group include, but are not limited to, the following groups: methyl, ethyl, propyl, butyl, pentyl, hexyl, heptyl, and octyl, which are not limited thereto. The number of carbon atoms in the alkyl group is C1 to C25, preferably C1 to C20, preferably C1 to C15, and more preferably C1 to C10.

"Substituted or unsubstituted silyl" of the present disclosure refers to a -Si(Rₖ)₃ group, where each Rₖ is identically or differently selected from the following groups: hydrogen, deuterium, tritium, cyano, halogen, nitro, substituted or unsubstituted C1 to C30 alkyl, substituted or unsubstituted C1 to C30 alkenyl, substituted or unsubstituted C3 to C30 cycloalkyl, and substituted or unsubstituted C6 to C60 aryl. Preferably, each Rₖ is identically or differently selected from the following groups: hydrogen, cyano, halogen, nitro, substituted or unsubstituted C1 to C30 alkyl, and substituted or unsubstituted C3 to C30 cycloalkyl. The number of carbon atoms in the alkyl group is preferably 1 to 20, preferably 1 to 15, more preferably 1 to 10, and most preferably 1 to 8. The number of carbon atoms in the cycloalkyl group is preferably 3 to 20, preferably 3 to 15, more preferably 3 to 10, and most preferably 3 to 7. The number of carbon atoms in the aryl group is preferably 6 to 20, preferably 6 to 13, more preferably 6 to 12, and most preferably 6 to 10. Preferably, each Rₖ is identically or differently selected from the following groups: hydrogen, cyano, halogen, nitro, substituted or unsubstituted methyl, substituted or unsubstituted ethyl, substituted or unsubstituted propyl, substituted or unsubstituted butyl, substituted or unsubstituted pentyl, substituted or unsubstituted hexyl, substituted or unsubstituted heptyl, substituted or unsubstituted octyl, substituted or unsubstituted cyclopropyl, substituted or unsubstituted cyclobutyl, substituted or unsubstituted cyclohexyl, substituted or unsubstituted cycloheptyl, substituted or unsubstituted adamantyl, substituted or unsubstituted norbornyl, substituted or unsubstituted phenyl, substituted or unsubstituted biphenyl, and substituted or unsubstituted naphthyl. Preferably, substituted silyl specifically includes, but is not limited to, trimethylsilyl, triethylsilyl, triisopropylsilyl, t-butyldimethylsilyl, vinyldimethylsilyl, propyldimethylsilyl, triphenylsilyl, diphenylsilyl, and phenylsilyl. Substituted silyl is preferably trimethylsilyl, triethylsilyl, triphenylsilyl, diphenylmethylsilyl, phenyldimethylsilyl, diphenylmethylsilyl, or phenyldimethylsilyl.

A cycloalkyl group of the present disclosure refers to a hydrocarbyl group formed by removing one hydrogen atom from a cycloalkane molecule. Cycloalkyl includes monocyclic cycloalkyl, polycyclic cycloalkyl, and endocyclic cycloalkyl. Examples of the cycloalkyl group include, but are not limited to, the following groups: cyclopropyl, cyclobutyl, cyclopentyl, cyclohexyl, cycloheptyl, adamantyl, norbornyl, camphanyl, fenchyl, and isocamphanyl, which are not limited thereto. The number of carbon atoms in the cycloalkyl group is C3 to C25, preferably C3 to C20, preferably C3 to C15, and more preferably C3 to C10.

An aryl group of the present disclosure refers to a general term for a monovalent group remaining after one hydrogen atom is removed from the aromatic core carbon of an aromatic compound molecule. Aryl includes monocyclic aryl, polycyclic aryl, fusedcyclic aryl, or a combination thereof. Examples of the aryl group include, but are not limited to, the following groups: phenyl, biphenyl, terphenyl, naphthyl, phenanthryl, anthryl, triphenylenyl, fluorenyl, benzofluorenyl, spirobifluorenyl, spiroanthrafluorenyl, pyrenyl, chrysenyl, fluoranthenyl, which are not limited thereto. The number of carbon atoms in the aryl group is C6 to C60, preferably C6 to C30, preferably C6 to C25, and preferably C6 to C20.

An arylene group of the present disclosure refers to a general term for a monovalent group remaining after one hydrogen atom is removed from the aromatic core carbon of an aromatic compound molecule. Arylene includes monocyclic arylene, polycyclic arylene, fusedcyclic arylene, or a combination thereof. Examples of the arylene group include, but are not limited to, the following groups: phenylene, biphenylylene, terphenylene, naphthylene, phenanthrylene, fluorenylene, benzofluorenylene, dibenzofluorenylene, naphthofluorenylene, and spirobifluorenylene, which are not limited thereto. The number of carbon atoms in the arylene group is C6 to C30, preferably C6 to C25, preferably C6 to C20, and more preferably C6 to C18.

The present disclosure provides a fluorene-containing triarylamine compound, which is represented by the following Formula 1: 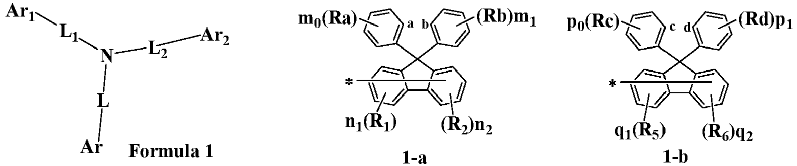 where Ar₁ is selected from a group represented by Formula 1-a;
"* " represents a linkage site to L₁;
Rₐ and R_{b} are each identically or differently selected from one of hydrogen, halogen, cyano, nitro, substituted or unsubstituted C1 to C25 alkyl, substituted or unsubstituted C3 to C25 cycloalkyl, and substituted or unsubstituted C6 to C60 aryl or a combination thereof, or adjacent two Rₐ or two R_{b} are bonded to each other to form a substituted or unsubstituted ring;
at least one of Rₐ and R_{b} is selected from substituted or unsubstituted C1 to C25 alkyl;
carbon atoms corresponding to a and b are separate, connected via a single bond, or bonded into a ring;
R₁ and R₂ are each identically or differently selected from one of hydrogen, halogen, cyano, nitro, substituted or unsubstituted C1 to C25 alkyl, substituted or unsubstituted silyl, substituted or unsubstituted C3 to C25 cycloalkyl, and substituted or unsubstituted C6 to C60 aryl, or adjacent two R₁ or two R₂ are bonded to each other to form a substituted or unsubstituted ring;
m₀ is selected from 1, 2, 3, 4, or 5; m₁ is selected from 1, 2, 3, 4, or 5;
n₁ is selected from 0, 1, 2, 3, or 4; n₂ is selected from 0, 1, 2, 3, or 4;
Ar₂ is selected from a group represented by Formula 1-b;
R_{c} and R_{d} are each identically or differently selected from one of hydrogen, halogen, cyano, nitro, substituted or unsubstituted C1 to C25 alkyl, substituted or unsubstituted C3 to C25 cycloalkyl, and substituted or unsubstituted C6 to C60 aryl or a combination thereof, or adjacent two R_{c} or two R_{d} are bonded to each other to form a substituted or unsubstituted ring;
at least one of R_{c} and R_{d} is selected from substituted or unsubstituted C1 to C25 alkyl;
carbon atoms corresponding to c and d are separate or bonded into a ring;
R₅ and R₆ are each identically or differently selected from one of hydrogen, halogen, cyano, nitro, substituted or unsubstituted C1 to C25 alkyl, substituted or unsubstituted silyl, substituted or unsubstituted C3 to C25 cycloalkyl, and substituted or unsubstituted C6 to C60 aryl, or adjacent two R₅ or two R₆ are bonded to each other to form a substituted or unsubstituted ring;
p₀ is selected from 1, 2, 3, 4, or 5; p₁ is selected from 1, 2, 3, 4, or 5;
q₁ is selected from 0, 1, 2, 3, or 4; q₂ is selected from 0, 1, 2, 3, or 4;
Ar is selected from one of substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, substituted or unsubstituted triphenylenyl, substituted or unsubstituted pyrenyl, substituted or unsubstituted 9,9-dimethylfluorenyl, substituted or unsubstituted 9,9-diphenylfluorenyl, substituted or unsubstituted 9,9-spirobifluorenyl, and substituted or unsubstituted spiroanthrafluorenyl; the substituent in the "substituted or unsubstituted" is selected from one of cyano, nitro, substituted or unsubstituted C1 to C25 alkyl, substituted or unsubstituted C3 to C25 cycloalkyl, and substituted or unsubstituted C6 to C30 aryl, or adjacent two groups are bonded to each other to form a substituted or unsubstituted benzene ring; and
L, L₁, and L₂ are independently selected from one of a single bond and substituted or unsubstituted C6 to C30 arylene.

Preferably, the fluorene-containing triarylamine compound is selected from one of Formulas 1-1 to 1-5: 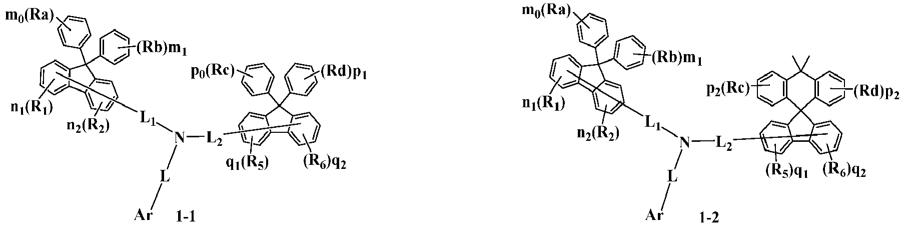 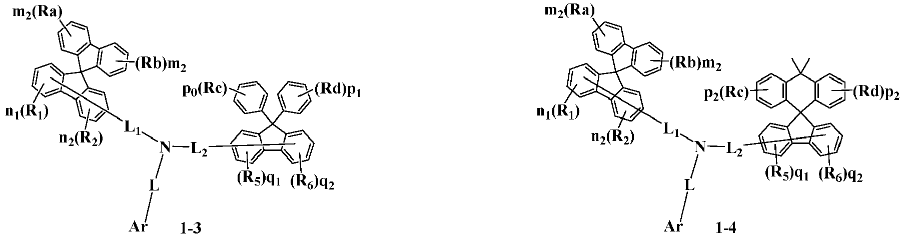 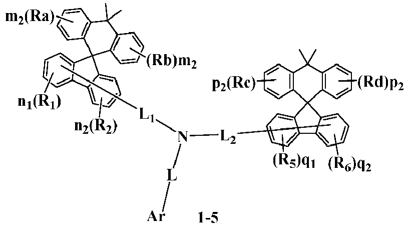 where m₀ is selected from 1, 2, 3, 4, or 5; m₁ is selected from 1, 2, 3, 4, or 5; m₂ is selected from 1, 2, 3, or 4; p₀ is selected from 1, 2, 3, 4, or 5; p₁ is selected from 1, 2, 3, 4, or 5; and p₂ is selected from 1, 2, 3, or 4.

Preferably, Ar₁ is selected from one of the following groups: 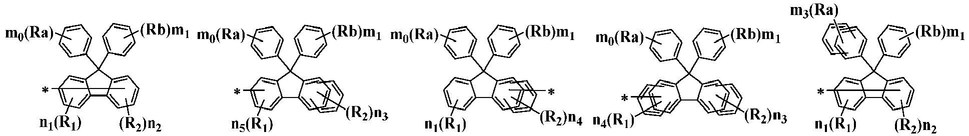 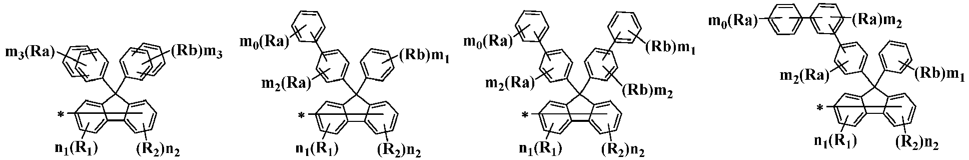 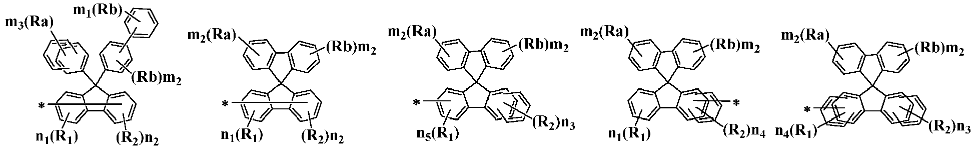 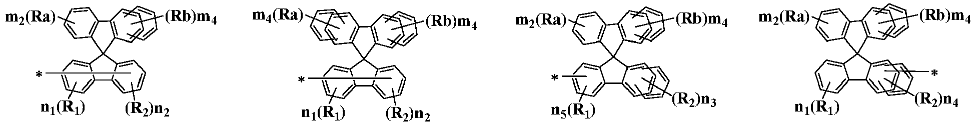 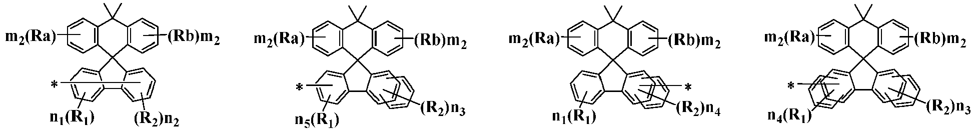 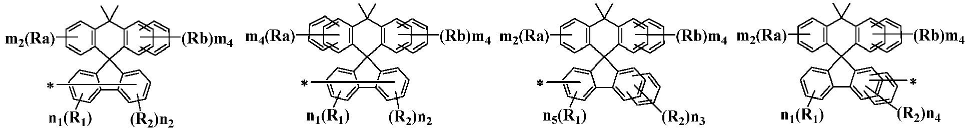 where m₀ is selected from 1, 2, 3, 4, or 5; m₁ is selected from 1, 2, 3, 4, or 5; m₂ is selected from 1, 2, 3, or 4; m₃ is selected from 1, 2, 3, 4, 5, 6, or 7; m₄ is selected from 1, 2, 3, 4, 5, or 6; n₁ is selected from 0, 1, 2, 3, or 4; n₂ is selected from 0, 1, 2, 3, or 4; n₃ is selected from 0, 1, 2, 3, 4, 5, or 6; n₄ is selected from 0, 1, 2, 3, 4, or 5; and n₅ is selected from 0, 1, 2, or 3.

More preferably, Ar₁ is selected from one of the following groups: 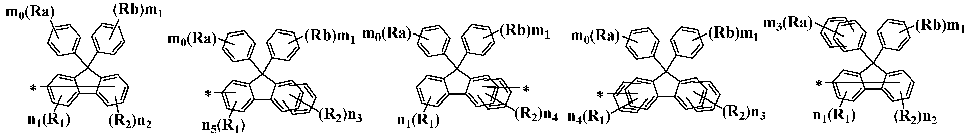 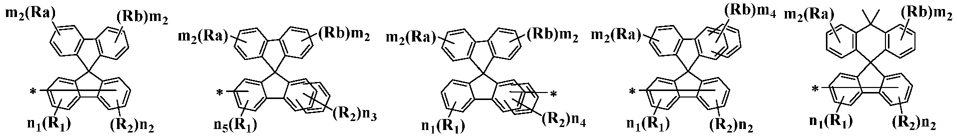 where m₀ is selected from 1, 2, 3, 4, or 5; m₁ is selected from 1, 2, 3, 4, or 5; m₂ is selected from 1, 2, 3, or 4; m₃ is selected from 1, 2, 3, 4, 5, 6, or 7; m₄ is selected from 1, 2, 3, 4, 5, or 6; n₁ is selected from 0, 1, 2, 3, or 4; n₂ is selected from 0, 1, 2, 3, or 4; n₃ is selected from 0, 1, 2, 3, 4, 5, or 6; n₄ is selected from 0, 1, 2, 3, 4, or 5; and n₅ is selected from 0, 1, 2, or 3.

In the present disclosure, Rₐ and R_{b} are each identically or differently selected from one of hydrogen, halogen, cyano, nitro, substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, substituted or unsubstituted decyl, substituted or unsubstituted cyclopropyl, substituted or unsubstituted cyclobutyl, substituted or unsubstituted cyclopentyl, substituted or unsubstituted cyclohexyl, substituted or unsubstituted adamantyl, substituted or unsubstituted norbornyl, substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, and substituted or unsubstituted triphenylenyl, or adjacent two Rₐ or two R_{b} are bonded to each other to form a substituted or unsubstituted benzene ring; and
R₁ and R₂ are each identically or differently selected from one of hydrogen, halogen, cyano, nitro, substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, substituted or unsubstituted decyl, substituted or unsubstituted cyclopropyl, substituted or unsubstituted cyclobutyl, substituted or unsubstituted cyclopentyl, substituted or unsubstituted cyclohexyl, substituted or unsubstituted adamantyl, substituted or unsubstituted norbornyl, substituted or unsubstituted trimethylsilyl, substituted or unsubstituted triethylsilyl, substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, and substituted or unsubstituted triphenylenyl, or adjacent two R₁ or two R₂ are bonded to each other to form a substituted or unsubstituted benzene ring.

Further preferably, R₁ and R₂ are each identically or differently selected from one of hydrogen, halogen, cyano, nitro, trifluoromethyl, methyl, ethyl, n-propyl, isopropyl, n-butyl, isobutyl, sec-butyl, *t*-butyl, pentyl, hexyl, heptyl, octyl, nonyl, decyl, cyclopropyl, cyclobutyl, cyclopentyl, cyclohexyl, adamantyl, norbornyl, trimethylsilyl, triethylsilyl, phenyl, biphenyl, terphenyl, naphthyl, anthryl, phenanthryl, and triphenylenyl.

Preferably, Ar₂ is selected from one of the following groups: 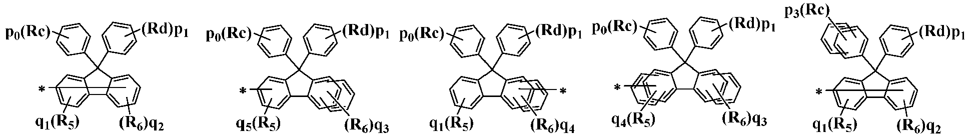 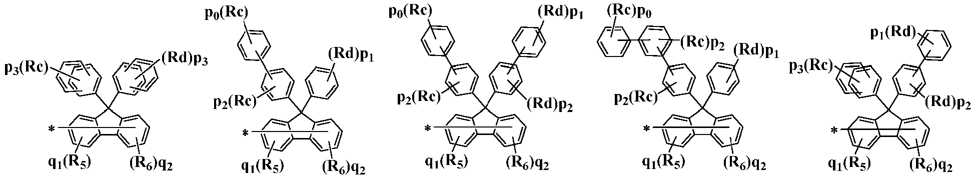 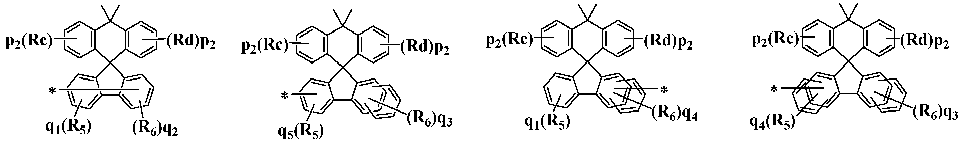 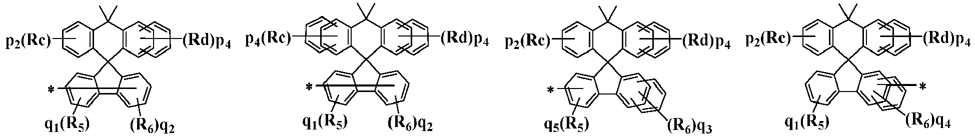 where p₀ is selected from 1, 2, 3, 4, or 5; p₁ is selected from 1, 2, 3, 4, or 5; p₂ is selected from 1, 2, 3, or 4; p₃ is selected from 1, 2, 3, 4, 5, 6, or 7; p₄ is selected from 1, 2, 3, 4, 5, or 6; q₁ is selected from 0, 1, 2, 3, or 4; q₂ is selected from 0, 1, 2, 3, or 4; q₃ is selected from 0, 1, 2, 3, 4, 5, or 6; q₄ is selected from 0, 1, 2, 3, 4, or 5; and q₅ is selected from 0, 1, 2, or 3.

More preferably, Ar₂ is selected from one of the following groups: 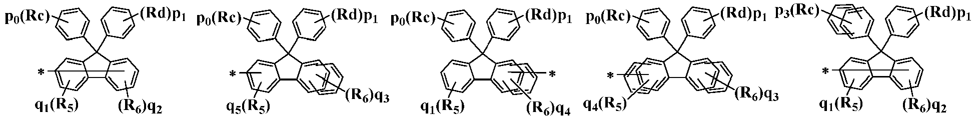 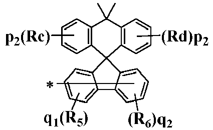 where p₀ is selected from 1, 2, 3, 4, or 5; p₁ is selected from 1, 2, 3, 4, or 5; p₂ is selected from 1, 2, 3, or 4; p₃ is selected from 1, 2, 3, 4, 5, 6, or 7; p₄ is selected from 1, 2, 3, 4, 5, or 6; q₁ is selected from 0, 1, 2, 3, or 4; q₂ is selected from 0, 1, 2, 3, or 4; q₃ is selected from 0, 1, 2, 3, 4, 5, or 6; q₄ is selected from 0, 1, 2, 3, 4, or 5; and q₅ is selected from 0, 1, 2, or 3.

In the present disclosure, R_{c} and R_{d} are each identically or differently selected from one of hydrogen, halogen, cyano, nitro, substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted t-butyl, substituted or unsubstituted pentyl, substituted or unsubstituted hexyl, substituted or unsubstituted heptyl, substituted or unsubstituted octyl, substituted or unsubstituted nonyl, substituted or unsubstituted decyl, substituted or unsubstituted cyclopropyl, substituted or unsubstituted cyclobutyl, substituted or unsubstituted cyclopentyl, substituted or unsubstituted cyclohexyl, substituted or unsubstituted adamantyl, substituted or unsubstituted norbornyl, substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, and substituted or unsubstituted triphenylenyl, or adjacent two R_{c} or two R_{d} are bonded to each other to form a substituted or unsubstituted benzene ring; and
R₅ and R₆ are each identically or differently selected from one of hydrogen, halogen, cyano, nitro, substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted t-butyl, substituted or unsubstituted pentyl, substituted or unsubstituted hexyl, substituted or unsubstituted heptyl, substituted or unsubstituted octyl, substituted or unsubstituted nonyl, substituted or unsubstituted decyl, substituted or unsubstituted cyclopropyl, substituted or unsubstituted cyclobutyl, substituted or unsubstituted cyclopentyl, substituted or unsubstituted cyclohexyl, substituted or unsubstituted adamantyl, substituted or unsubstituted norbornyl, substituted or unsubstituted trimethylsilyl, substituted or unsubstituted triethylsilyl, substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, and substituted or unsubstituted triphenylenyl, or adjacent two R₅ or two R₆ are bonded to each other to form a substituted or unsubstituted benzene ring.

Further preferably, R₅ and R₆ are each identically or differently selected from one of hydrogen, halogen, cyano, nitro, trifluoromethyl, methyl, ethyl, n-propyl, isopropyl, n-butyl, isobutyl, sec-butyl, t-butyl, pentyl, hexyl, heptyl, octyl, nonyl, decyl, cyclopropyl, cyclobutyl, cyclopentyl, cyclohexyl, adamantyl, norbornyl, trimethylsilyl, triethylsilyl, phenyl, biphenyl, terphenyl, naphthyl, anthryl, phenanthryl, and triphenylenyl.

Preferably, "at least one of Rₐ and R_{b}" includes at least one of Rₐ, at least one of R_{b}, or at least one of Rₐ and at least one of R_{b}.

"At least one" includes one, two, three, four, and more if allowed.

Preferably, at least one of Rₐ and R_{b} is selected from one of substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, and substituted or unsubstituted decyl.

Preferably, at least one of Rₐ and R_{b} is selected from one of methyl, ethyl, n-propyl, isopropyl, n-butyl, isobutyl, sec-butyl, *t*-butyl, pentyl, hexyl, heptyl, octyl, nonyl, and decyl.

More preferably, at least one of Rₐ and R_{b} is selected from one of methyl, ethyl, n-propyl, isopropyl, n-butyl, isobutyl, sec-butyl, and *t*-butyl.

More preferably, at least one of Rₐ and R_{b} is selected from methyl, ethyl, isopropyl, or t-butyl.

Most preferably, at least one of Rₐ and R_{b} is selected from isopropyl or *t*-butyl.

Preferably, "at least one of R_{c} and R_{d}" includes at least one of R_{c}, at least one of R_{d}, or at least one of R_{c} and at least one of R_{d}.

"At least one" includes one, two, three, four, and more if allowed.

Preferably, at least one of R_{c} and R_{d} is selected from one of substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, and substituted or unsubstituted decyl.

Preferably, at least one of R_{c} and R_{d} is selected from one of methyl, ethyl, n-propyl, isopropyl, n-butyl, isobutyl, sec-butyl, *t*-butyl, pentyl, hexyl, heptyl, octyl, nonyl, and decyl.

More preferably, at least one of R_{c} and R_{d} is selected from one of methyl, ethyl, n-propyl, isopropyl, n-butyl, isobutyl, sec-butyl, and *t*-butyl.

More preferably, at least one of R_{c} and R_{d} is selected from methyl, ethyl, isopropyl, or t-butyl.

Most preferably, at least one of R_{c} and R_{d} is selected from isopropyl or t-butyl.

Preferably, Ar is selected from one of the following groups or a combination thereof: 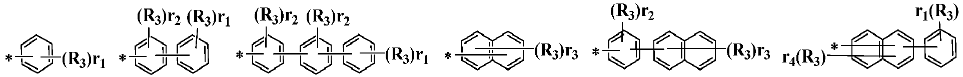 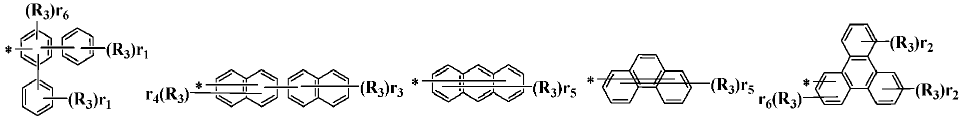 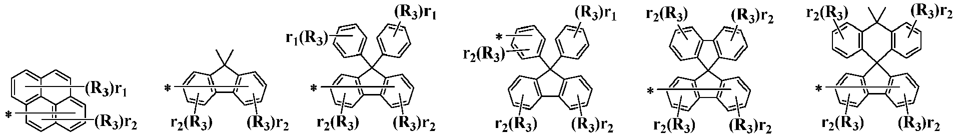
where R₃ is selected from one of hydrogen, cyano, nitro, substituted or unsubstituted C1 to C25 alkyl, substituted or unsubstituted C3 to C25 cycloalkyl, and substituted or unsubstituted C6 to C30 aryl, or adjacent two R₃ are bonded to each other to form a substituted or unsubstituted benzene ring; and
where r₁ is selected from 0, 1, 2, 3, 4, or 5; r₂ is selected from 0, 1, 2, 3, or 4; r₃ is selected from 0, 1, 2, 3, 4, 5, 6, or 7; r₄ is selected from 0, 1, 2, 3, 4, 5, or 6; r₅ is selected from 0, 1, 2, 3, 4, 5, 6, 7, 8, or 9; and r₆ is selected from 0, 1, 2, or 3.

Preferably, Ar is selected from one of the following groups or a combination thereof: 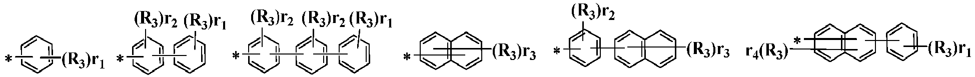 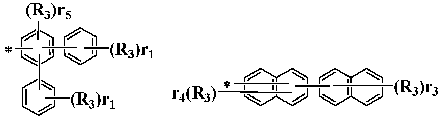 where r₁ is selected from 0, 1, 2, 3, 4, or 5; r₂ is selected from 0, 1, 2, 3, or 4; r₃ is selected from 0, 1, 2, 3, 4, 5, 6, or 7; r₄ is selected from 0, 1, 2, 3, 4, 5, or 6; and r₅ is selected from 0, 1, 2, or 3.

In the present disclosure, R₃ is selected from one of hydrogen, cyano, nitro, substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, substituted or unsubstituted decyl, substituted or unsubstituted cyclopropyl, substituted or unsubstituted cyclobutyl, substituted or unsubstituted cyclopentyl, substituted or unsubstituted cyclohexyl, substituted or unsubstituted adamantyl, substituted or unsubstituted norbornyl, substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, and substituted or unsubstituted triphenylenyl, or adjacent two R₃ are bonded to form a substituted or unsubstituted benzene ring.

Further preferably, Ar is selected from one of the following groups or a combination thereof: 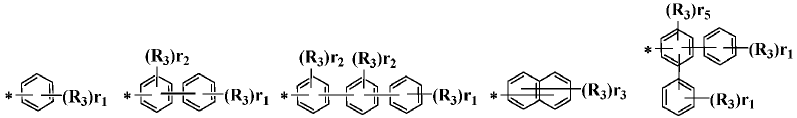
where R₃ is selected from one of hydrogen, cyano, nitro, trifluoromethyl, methyl, ethyl, n-propyl, isopropyl, n-butyl, isobutyl, sec-butyl, *t*-butyl, pentyl, hexyl, heptyl, octyl, nonyl, decyl, cyclopropyl, cyclobutyl, cyclopentyl, cyclohexyl, adamantyl, norbornyl, phenyl, biphenyl, terphenyl, naphthyl, anthryl, phenanthryl, and triphenylenyl; and
where r₁ is selected from 0, 1, 2, 3, 4, or 5; r₂ is selected from 0, 1, 2, 3, or 4; r₃ is selected from 0, 1, 2, 3, 4, 5, 6, or 7; and r₅ is selected from 0, 1, 2, or 3.

Preferably, L, L₁, and L₂ are independently selected from one of a single bond and the following groups or a combination thereof: 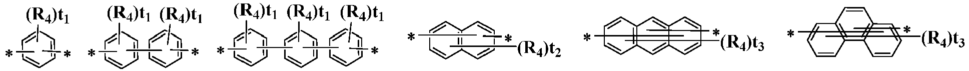 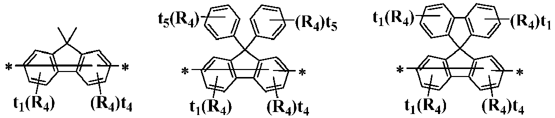
where R₄ is selected from one of hydrogen, halogen, cyano, nitro, substituted or unsubstituted C1 to C25 alkyl, substituted or unsubstituted C3 to C25 cycloalkyl, and substituted or unsubstituted C6 to C30 aryl; and
where t₁ is selected from 0, 1, 2, 3, or 4; t₂ is selected from 0, 1, 2, 3, 4, 5, or 6; t₃ is selected from 0, 1, 2, 3, 4, 5, 6, 7, or 8; t₄ is selected from 0, 1, 2, or 3; and t₅ is selected from 0, 1, 2, 3, 4, or 5.

Preferably, R₄ is selected from one of hydrogen, halogen, cyano, nitro, trifluoromethyl, substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, substituted or unsubstituted decyl, substituted or unsubstituted cyclopropyl, substituted or unsubstituted cyclobutyl, substituted or unsubstituted cyclopentyl, substituted or unsubstituted cyclohexyl, substituted or unsubstituted adamantyl, substituted or unsubstituted norbornyl, substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, and substituted or unsubstituted triphenylenyl.

More preferably, L, L₁, and L₂ are independently selected from one of a single bond and the following groups or a combination thereof: 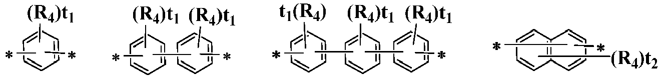
where R₄ is selected from one of hydrogen, halogen, cyano, nitro, trifluoromethyl, methyl, ethyl, n-propyl, isopropyl, n-butyl, isobutyl, sec-butyl, *t*-butyl, pentyl, hexyl, heptyl, octyl, nonyl, decyl, cyclopropyl, cyclobutyl, cyclopentyl, cyclohexyl, adamantyl, norbornyl, phenyl, biphenyl, terphenyl, naphthyl, anthryl, phenanthryl, and triphenylenyl; and
where t₁ is selected from 0, 1, 2, 3, or 4; and t₂ is selected from 0, 1, 2, 3, 4, 5, or 6.

More preferably, L₁ and L₂ are independently selected from a single bond.

Preferably, the fluorene-containing triarylamine compound is selected from any one of the following structures:

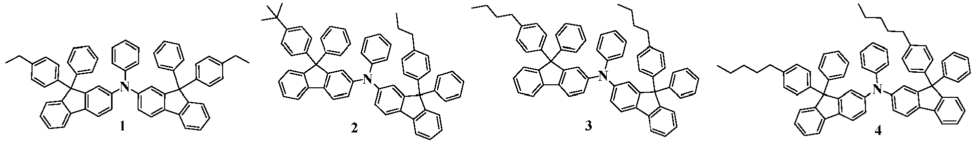

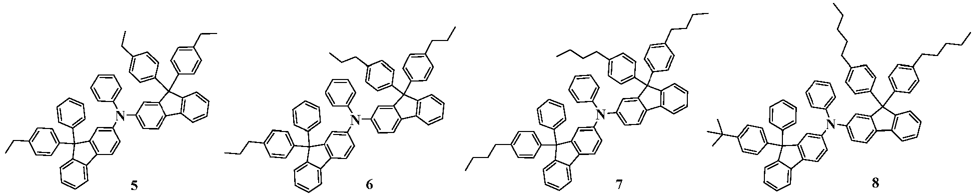

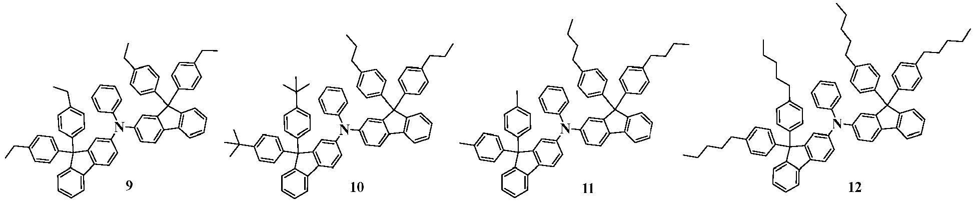

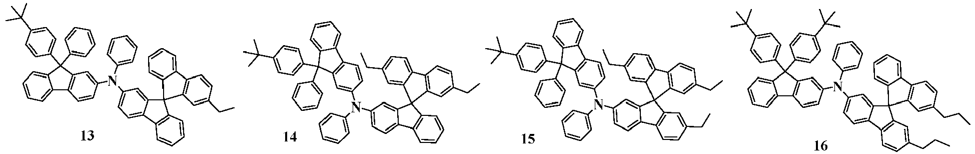

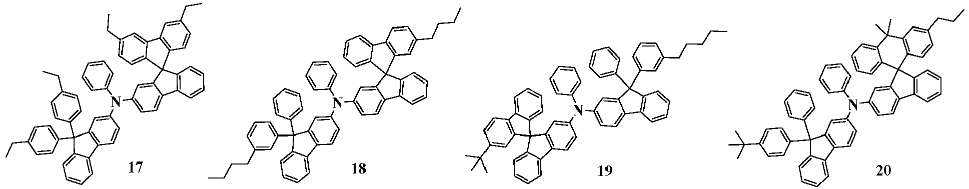

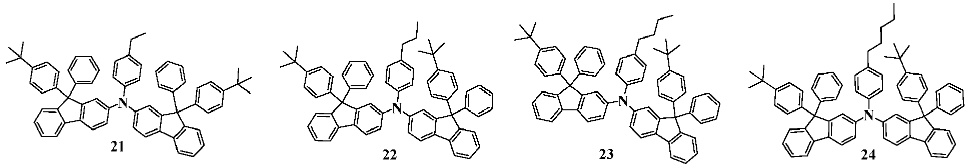

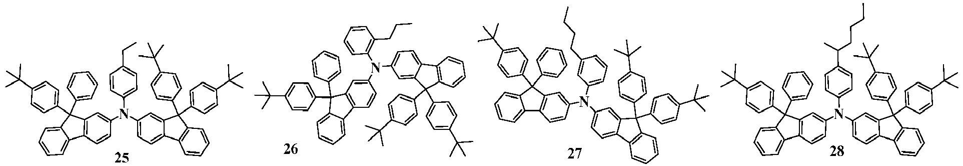

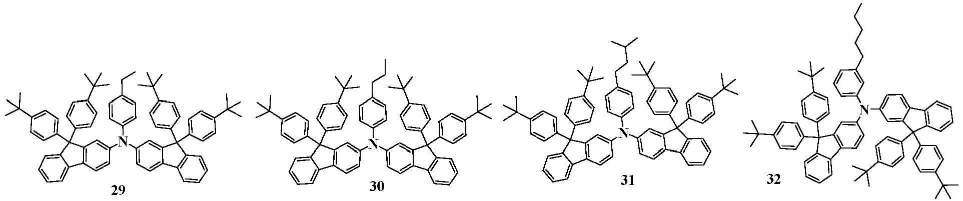

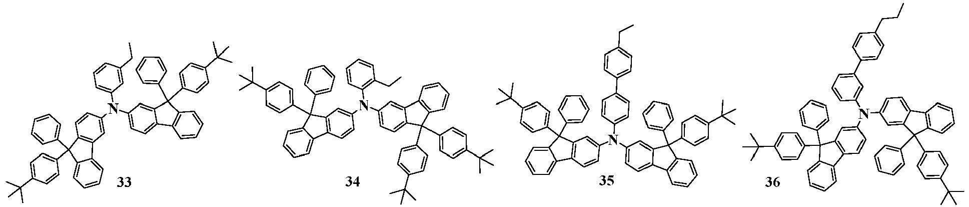

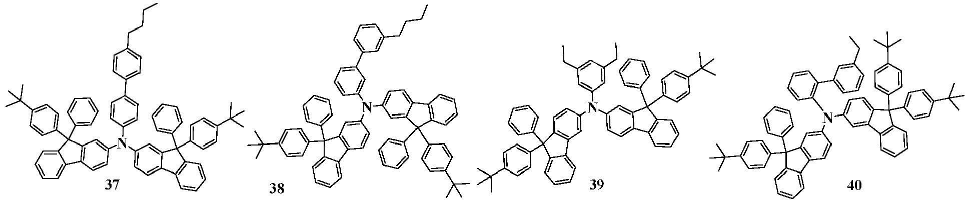

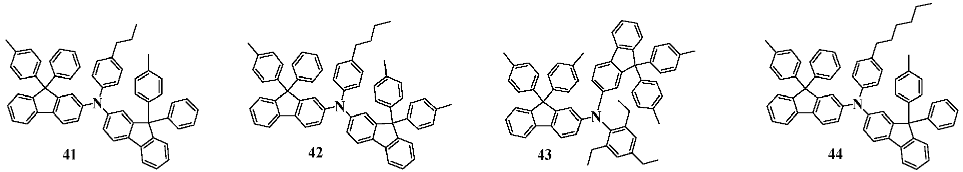

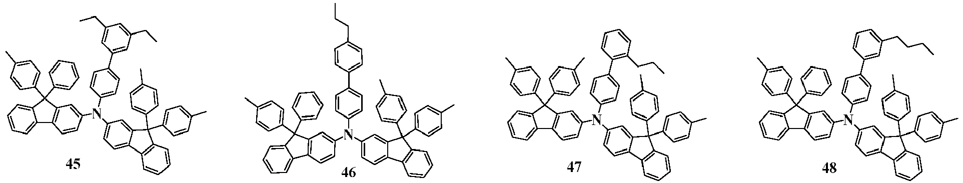

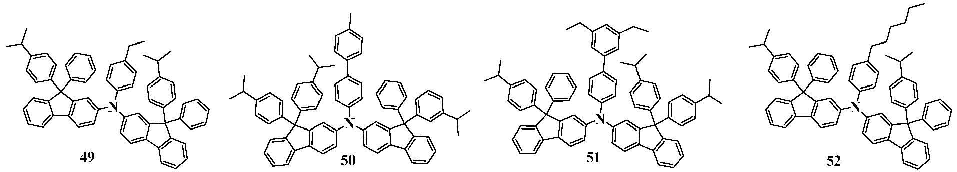

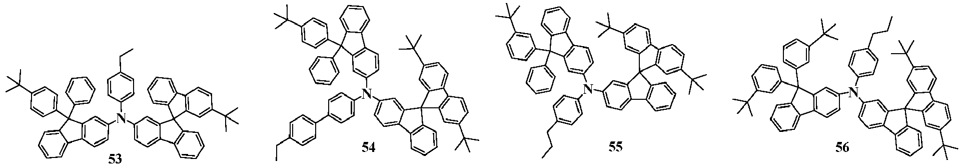

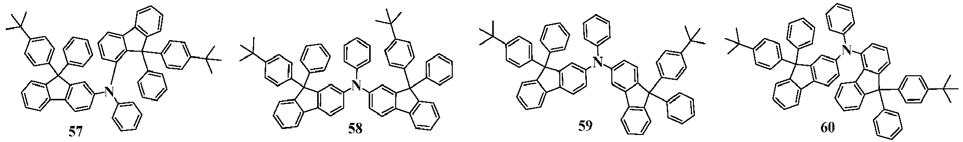

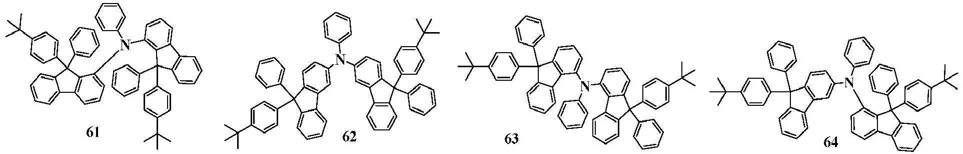

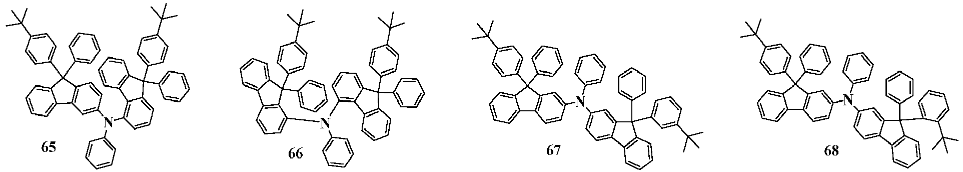

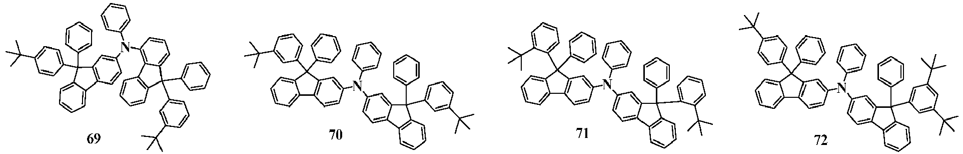

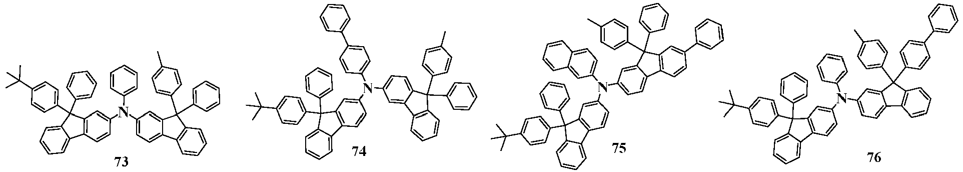

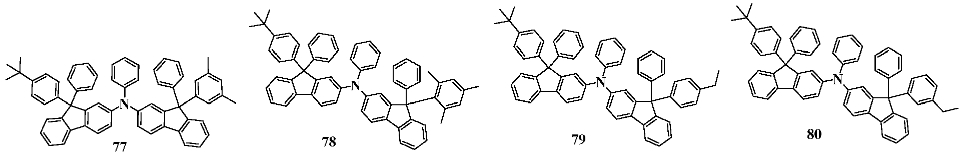

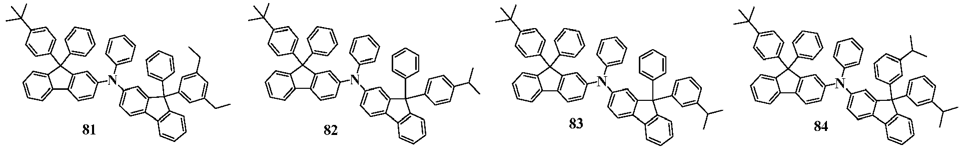

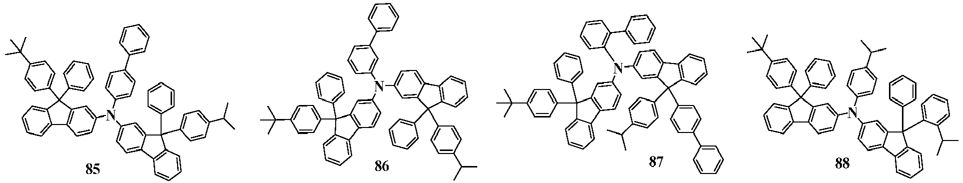

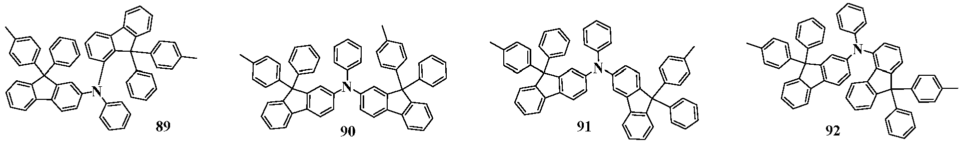

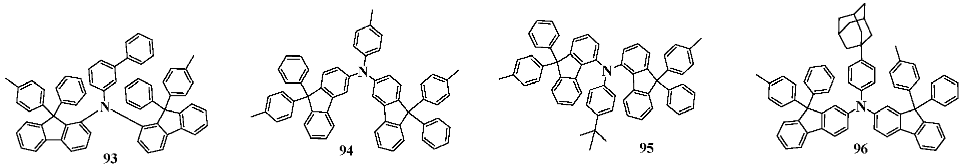

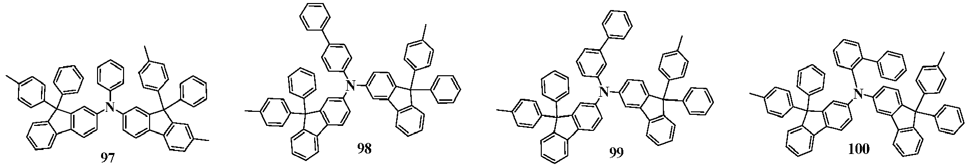

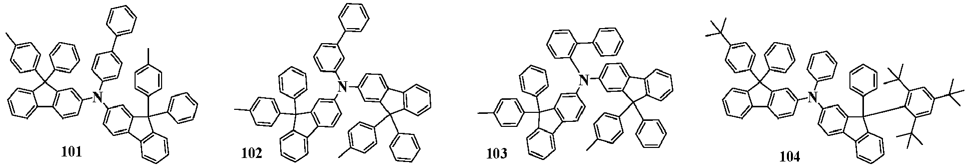

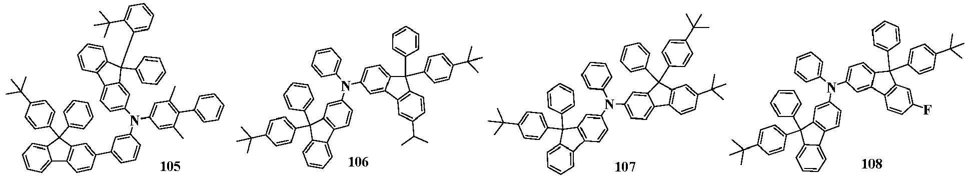

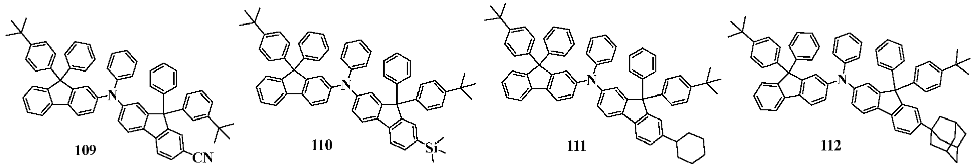

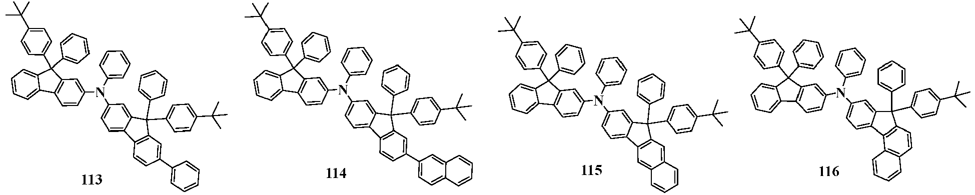

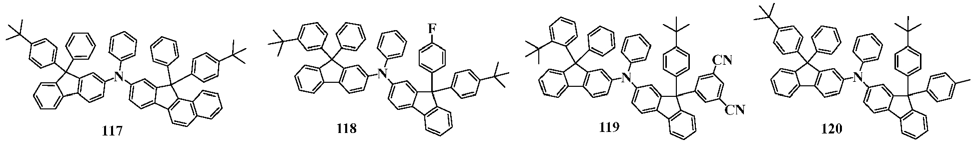

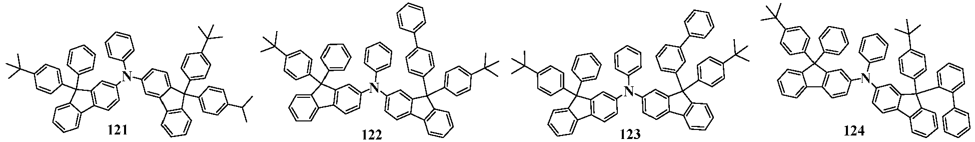

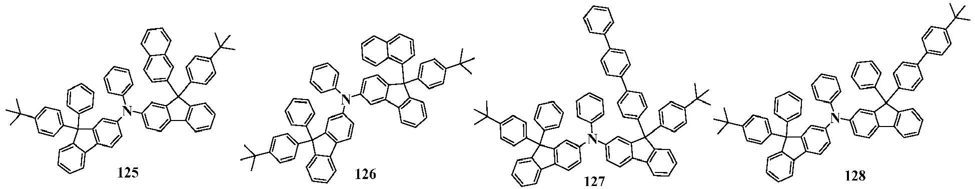

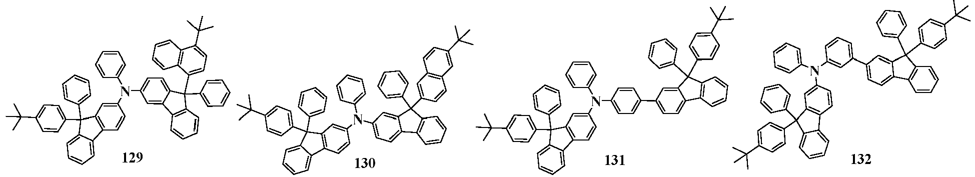

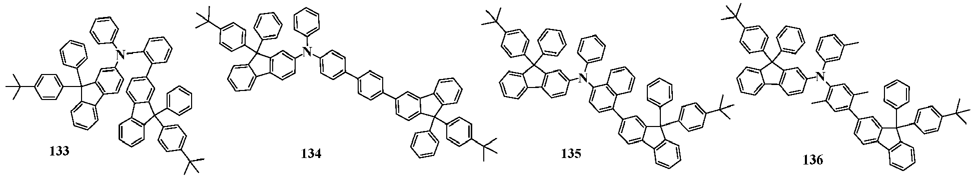

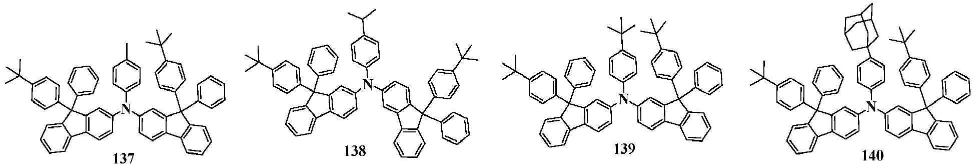

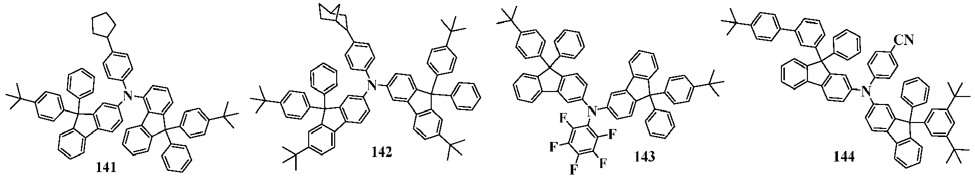

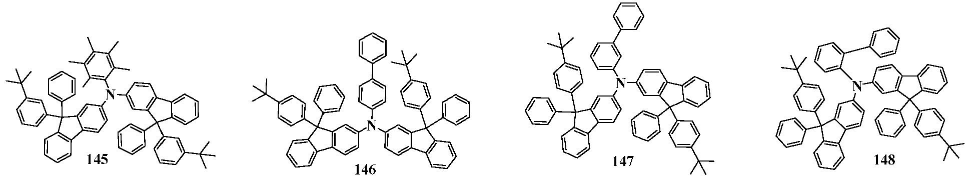

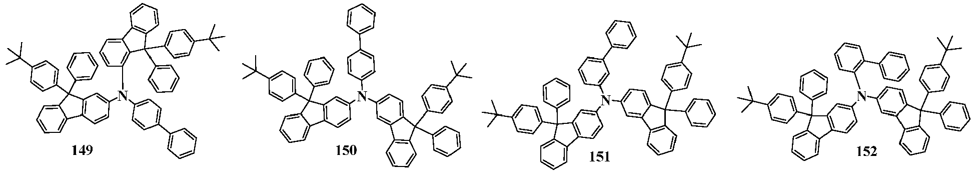

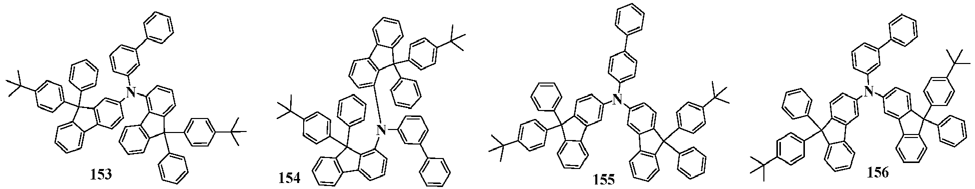

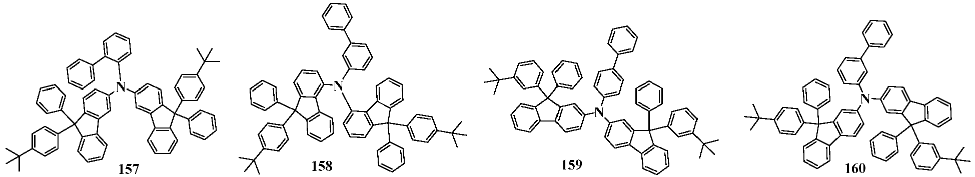

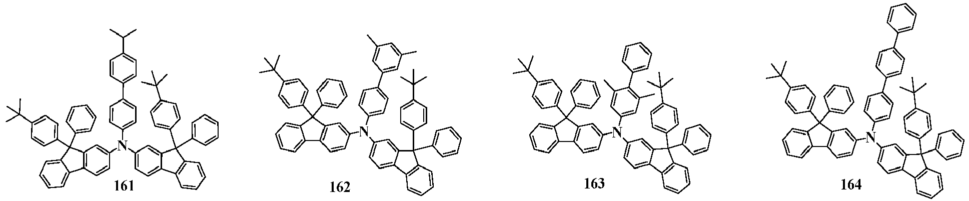

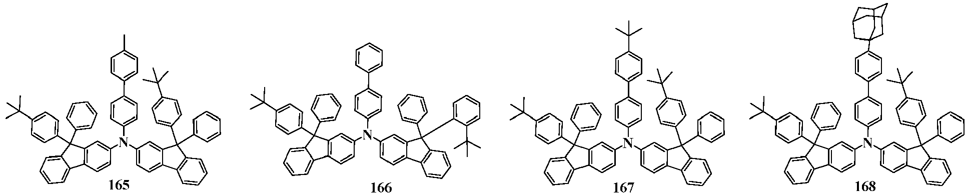

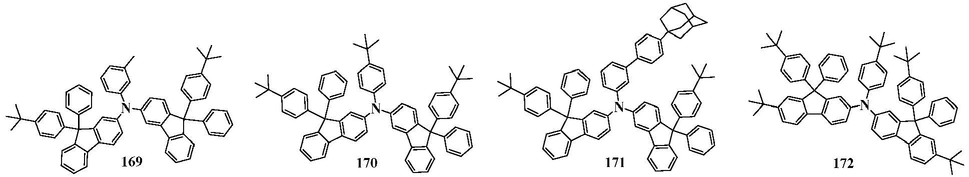

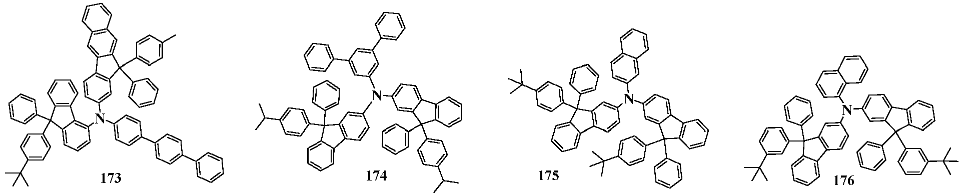

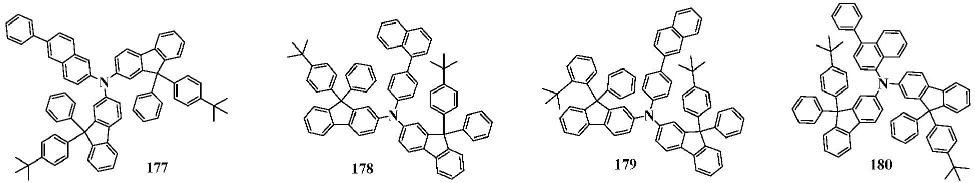

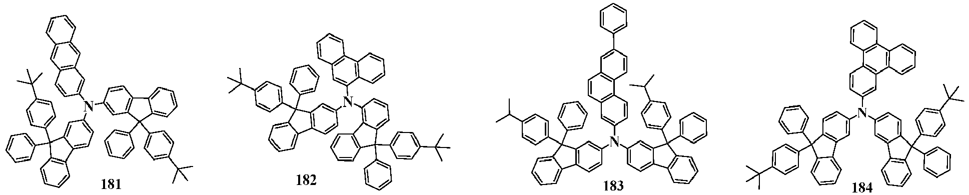

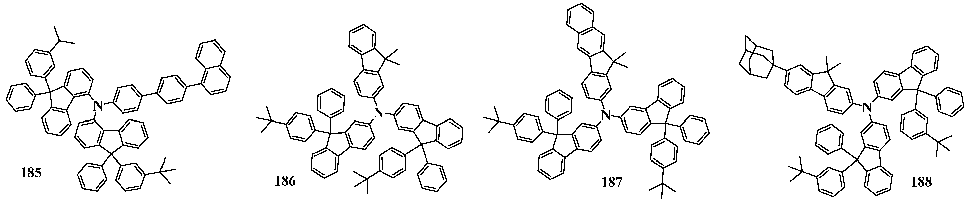

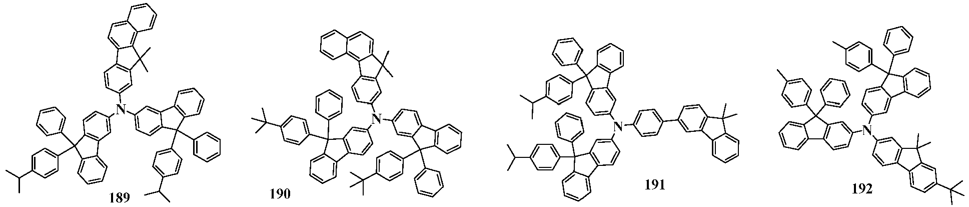

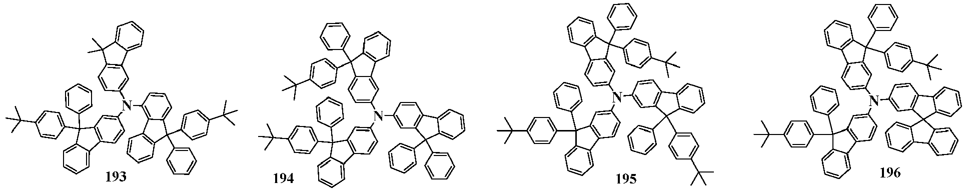

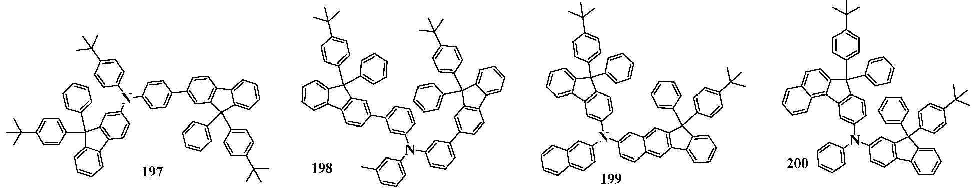

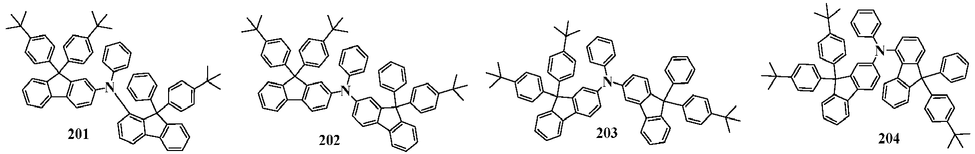

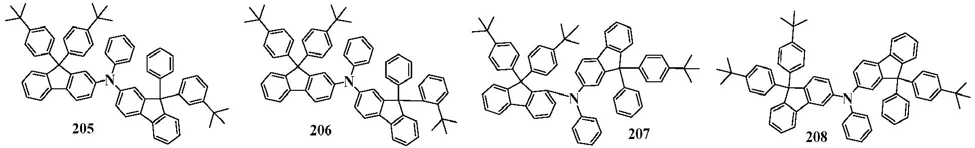

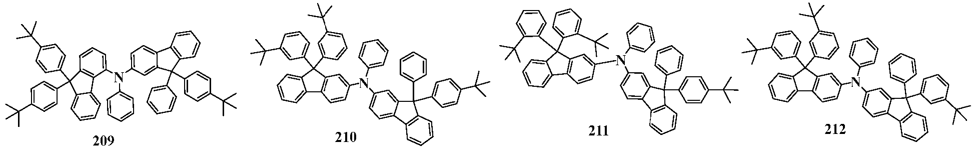

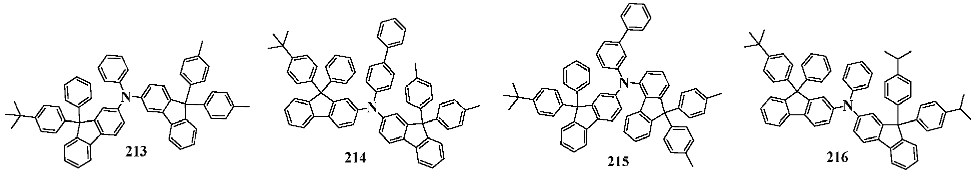

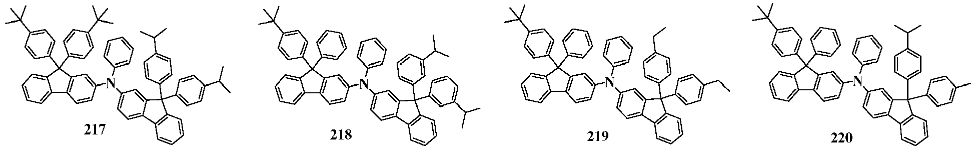

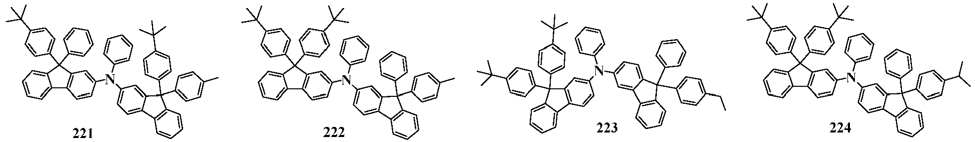

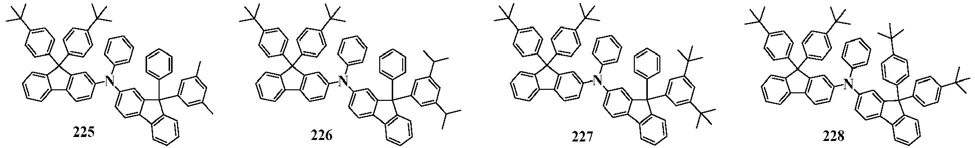

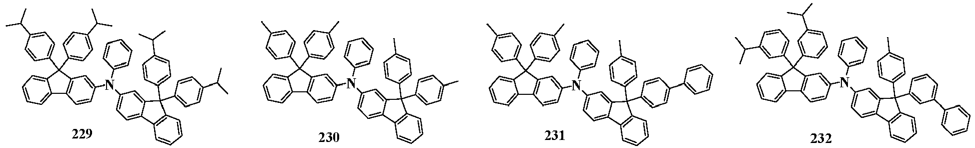

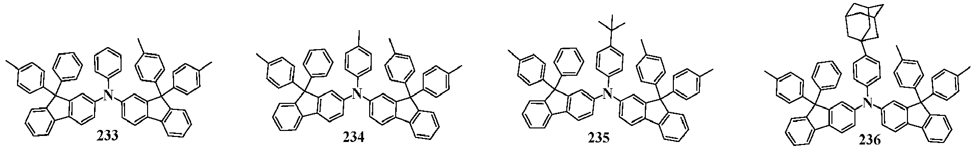

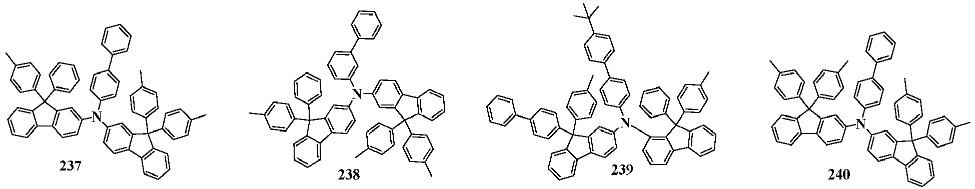

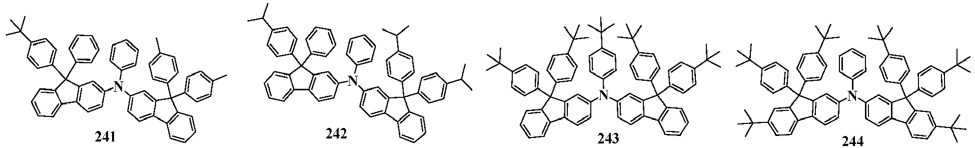

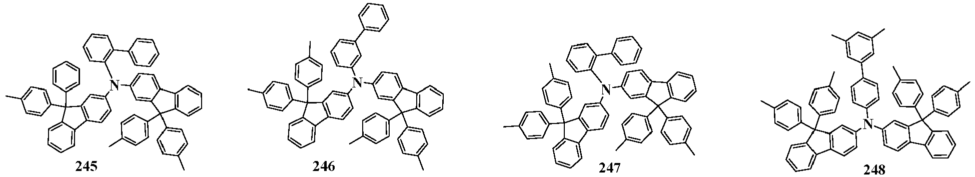

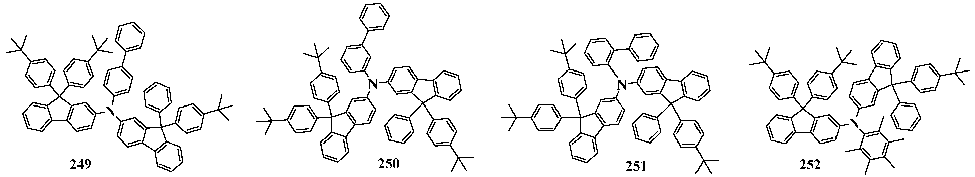

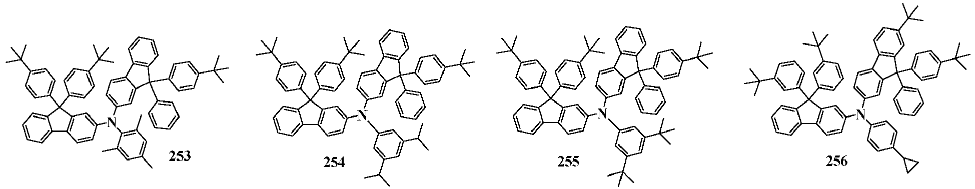

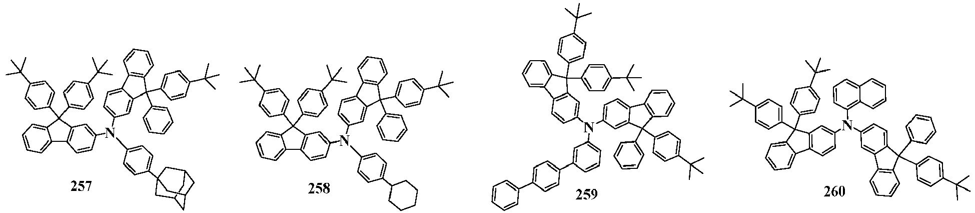

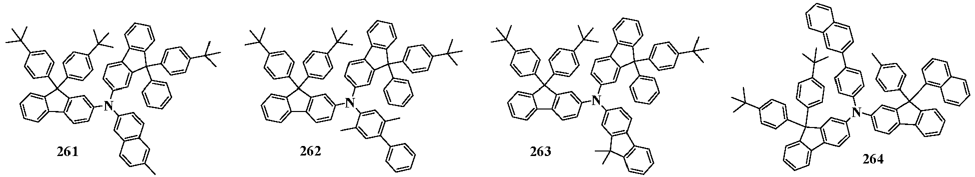

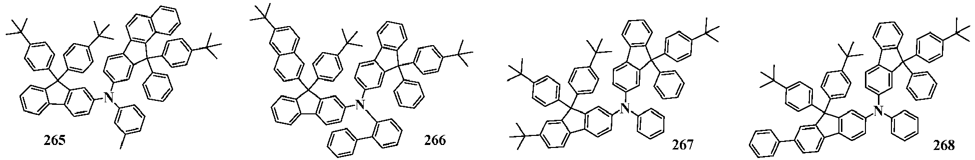

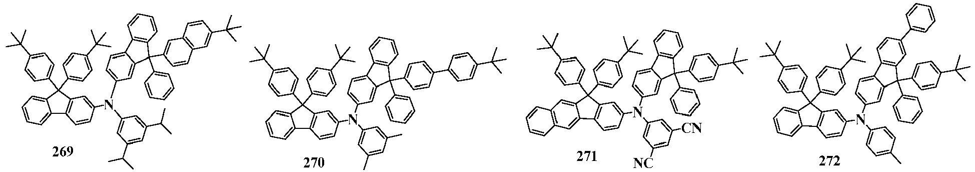

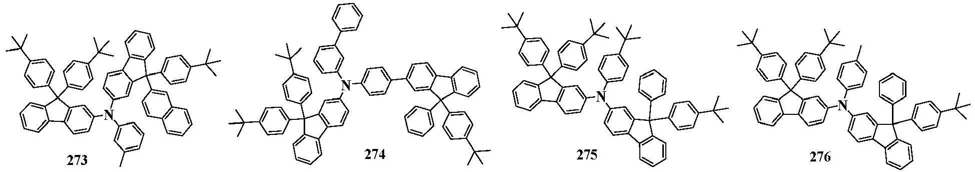

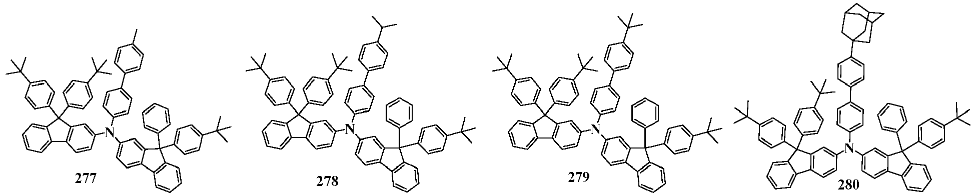

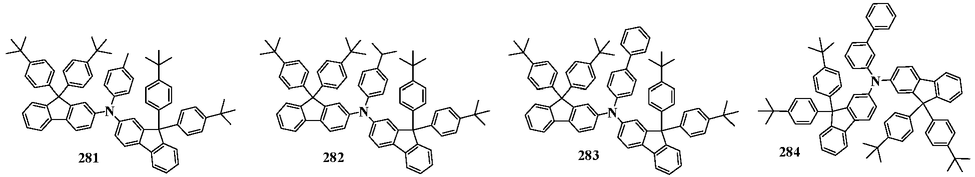

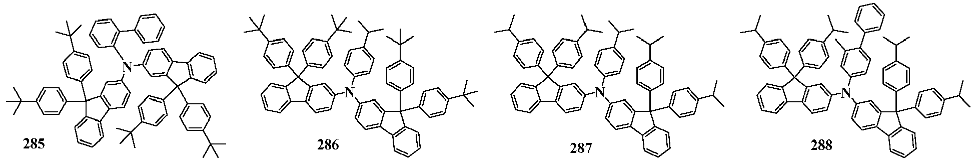

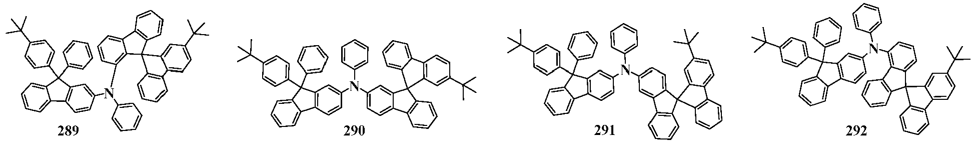

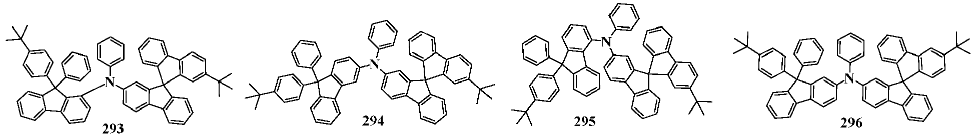

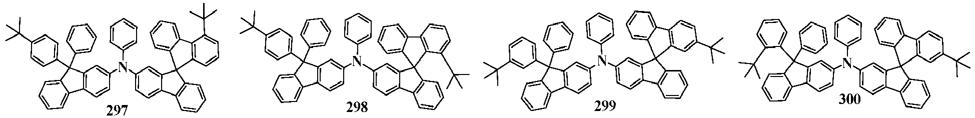

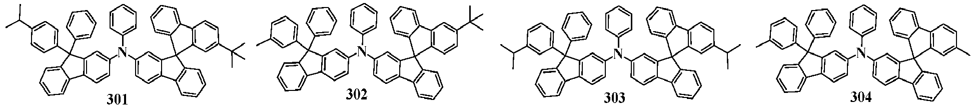

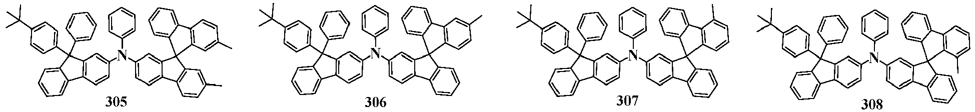

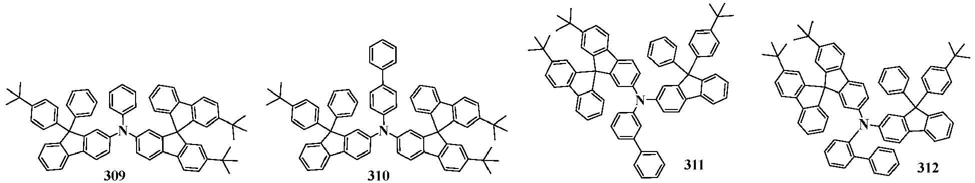

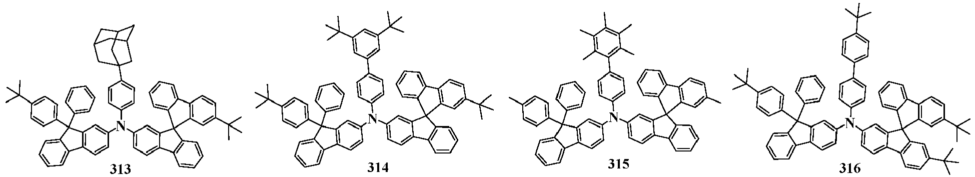

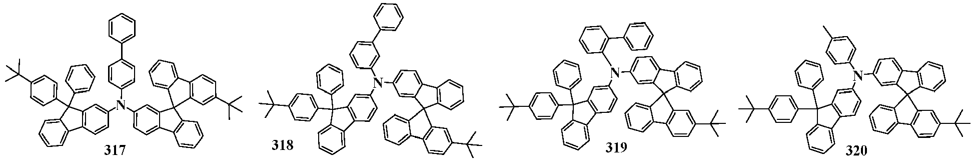

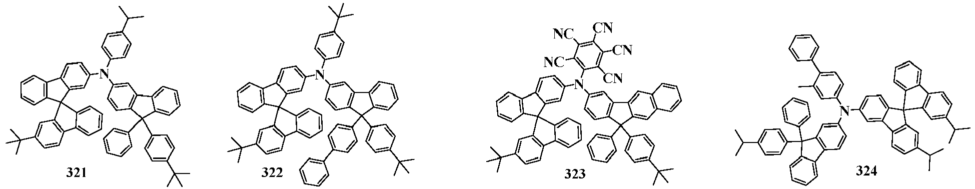

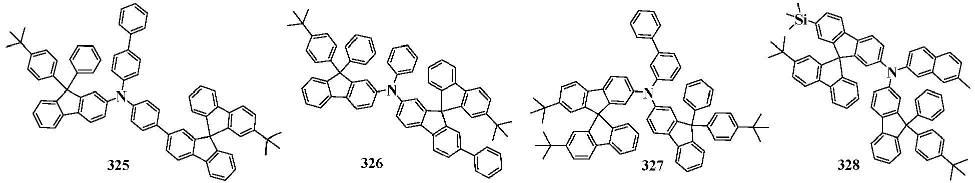

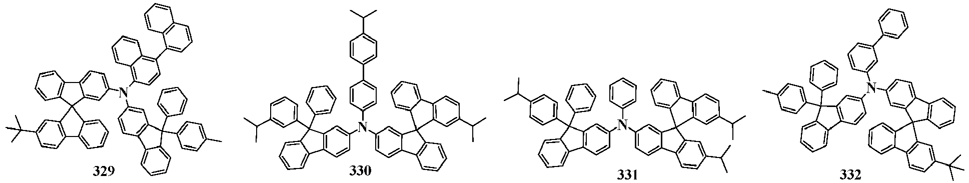

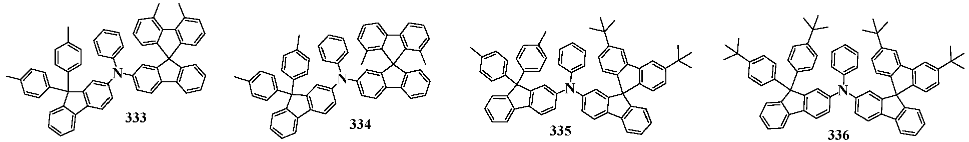

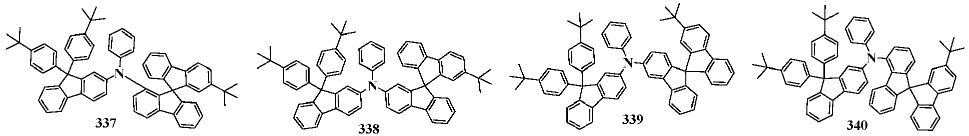

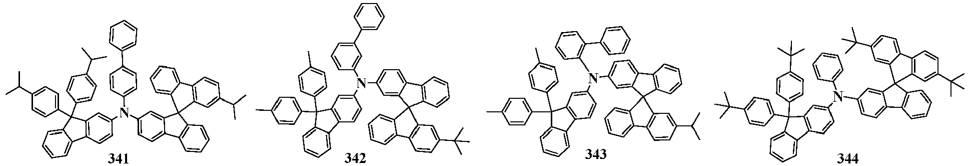

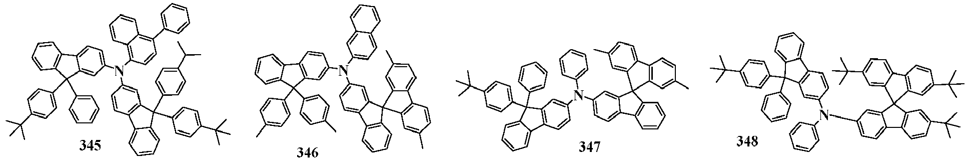

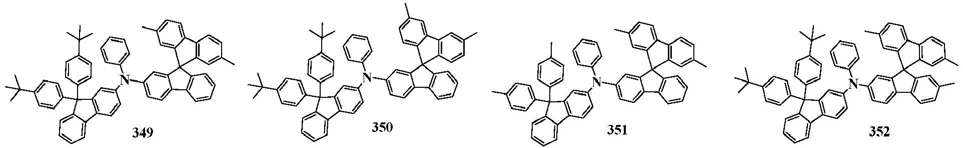

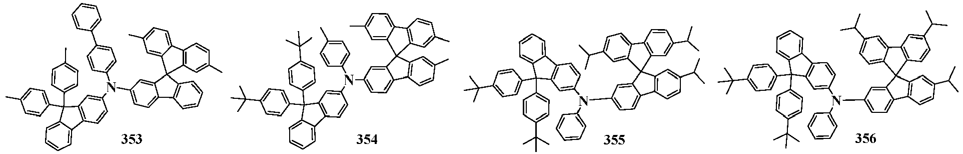

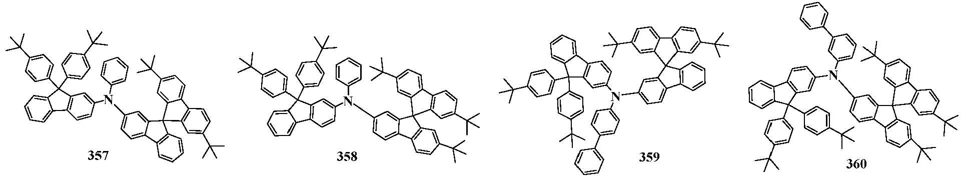

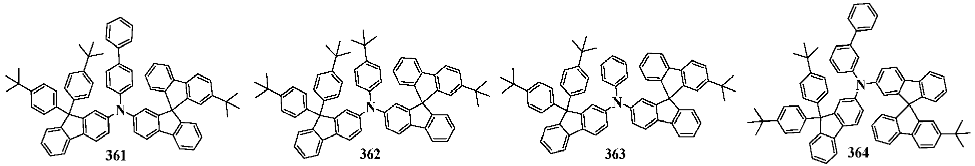

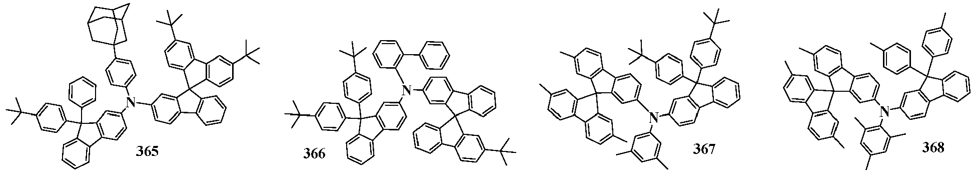

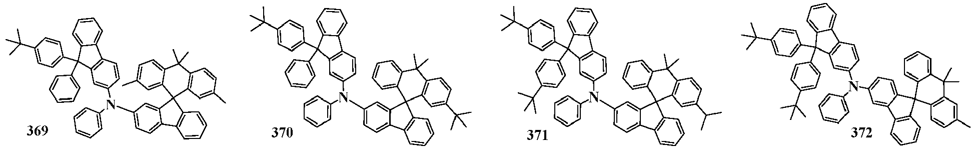

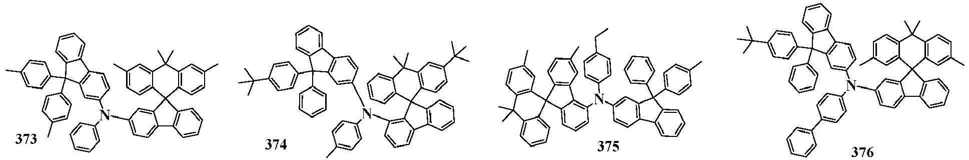

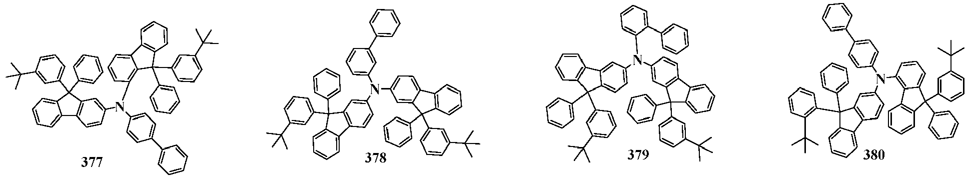

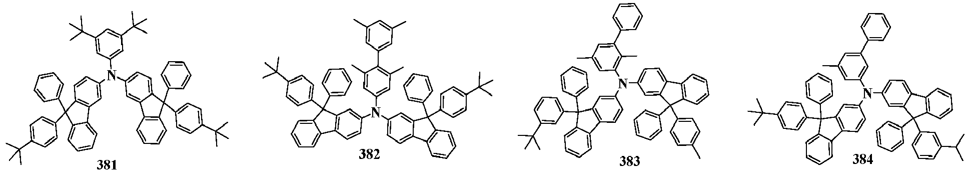

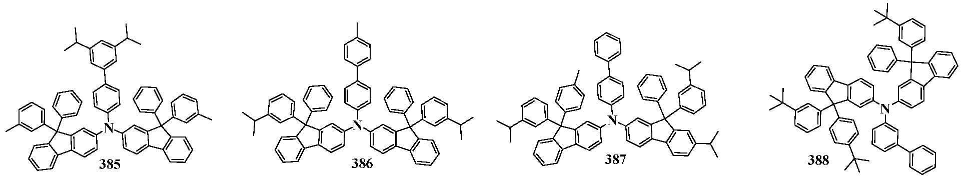

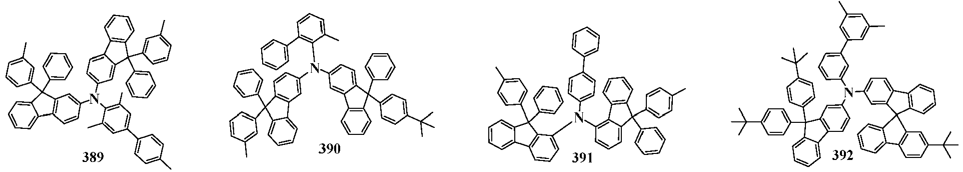

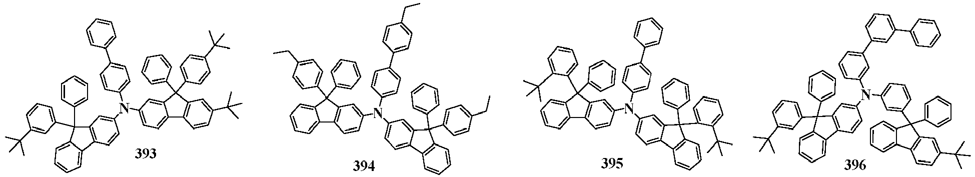

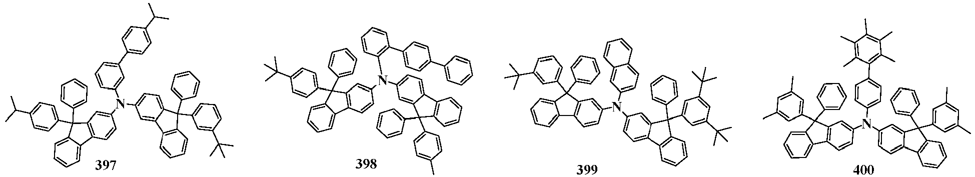

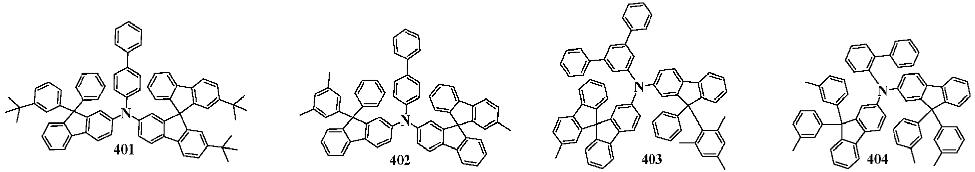

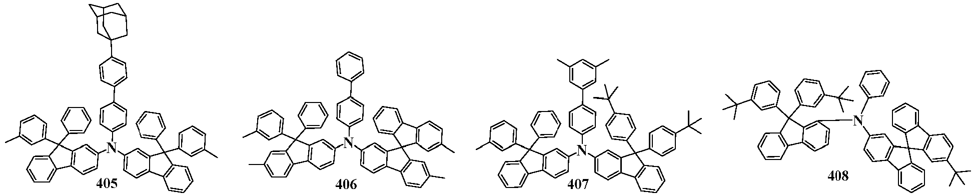

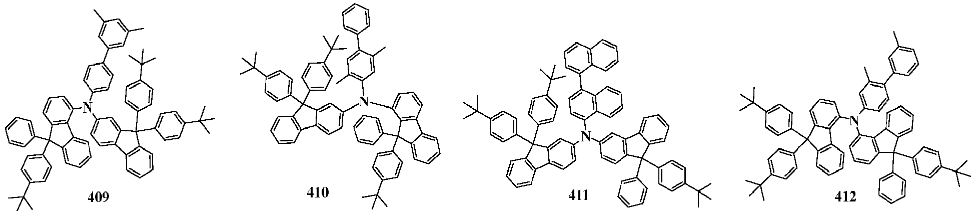

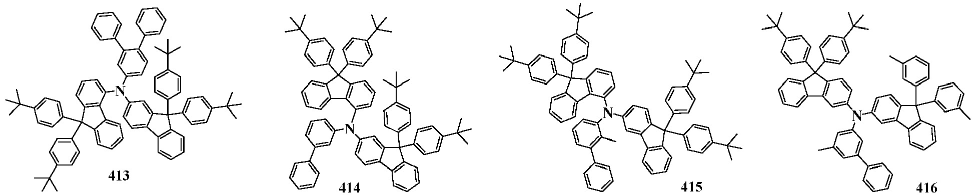

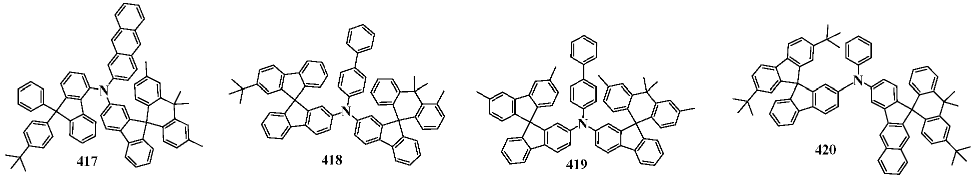

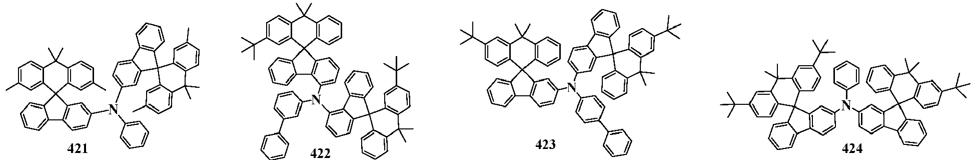

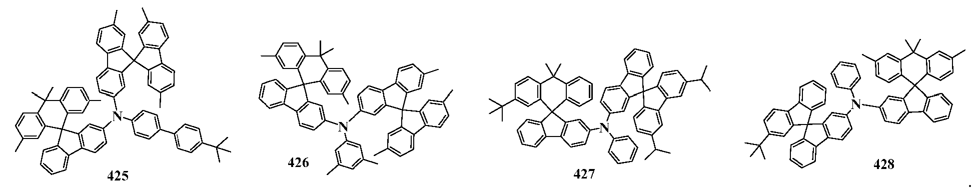

Some specific chemical structures of the fluorene-containing triarylamine compound of the present disclosure represented by Formula 1 are exemplified above. However, the present disclosure is not limited to the exemplified chemical structures. Any structures based on the structure represented by Formula 1 and having substituents which are the groups defined above are included.

Additionally, the present disclosure further provides an organic electroluminescent device. The organic electroluminescent device includes an anode, a cathode, and an organic layer, where the organic layer includes the preceding fluorene-containing triarylamine compound of the present disclosure.

Preferably, the organic electroluminescent device includes an anode, a cathode, and an organic layer, where the organic layer is disposed between the anode and the cathode, and the organic layer includes the preceding fluorene-containing triarylamine compound of the present disclosure.

Preferably, the organic electroluminescent device includes an anode, a cathode, and an organic layer, where the organic layer is disposed between the anode and the cathode, the organic layer includes a hole transporting layer, and the hole transporting layer includes the preceding fluorene-containing triarylamine compound of the present disclosure.

Preferably, the organic layer includes a hole transporting layer, the hole transporting layer includes a first hole transporting layer and/or a second hole transporting layer, the first hole transporting layer is disposed between the anode and a light-emitting layer, the second hole transporting layer is disposed between the first hole transporting layer and the light-emitting layer, and the first hole transporting layer and/or the second hole transporting layer include the preceding fluorene-containing triarylamine compound of the present disclosure.

A function layer of the organic electroluminescent device of the present disclosure may include at least one of the following function layers: a hole injection layer, a hole transporting layer, a luminescence assistance layer, an electron blocking layer, a light-emitting layer, a hole blocking layer, an electron transporting layer, an electron injection layer, a capping layer, etc. Any function layers with hole injection and/or transporting properties, function layers with electron injection and/or transporting properties, function layers with a light-emitting property, or function layers with a light extraction property are to be included. Each function layer may include a single film or multiple films. Each film may be composed of only one material or multiple materials.

A material of each film in the organic electroluminescent device is not particularly limited in the present disclosure, which may be a substance known in the art. The above-mentioned organic function layers of the organic electroluminescent device and electrodes on two sides of the device are separately described below.

The anode of the present disclosure preferably uses a material with a relatively high work function and may be a single-layer structure or a multi-layer composite structure. The anode includes, but is not limited to, the following materials: a metal oxide, a metal or an alloy thereof, a stacked material, and a conductive polymer. Specific examples may include, but are not limited to, gold (Au), platinum (Pt), aluminum (Al), indium zinc oxide (IZO), indium tin oxide (ITO), indium tin oxide/silver/indium tin oxide (ITO/Ag/ITO), and polyaniline.

The hole injection layer of the present disclosure preferably uses a material with a good hole injection ability, including, but not limited to, a triarylamine compound, a porphyrin compound, a styrene compound, polythiophene and derivatives thereof, and a phthalocyanine derivative. Specific examples of a hole injection material may include, but are not limited to, *N,N'*-bis[4-di(m-tolyl)aminophenyl]-*N,N'*-diphenylbenzidine (DNTPD), 4,4',4"-tris(N-(1-naphthyl)-*N-*phenylamino)triphenylamine (1-TNATA), 4,4',4'-tris[2-naphthylphenylamino]triphenylamine (2-TNATA), 1,4,5,8,9,11-hexaazatriphenylenehexacarbonitrile (HAT-CN), molybdenum trioxide (MoO₃), and poly(4-vinyl-triphenylamine) (PVTPA).

The hole transporting layer of the present disclosure preferably uses a material with relatively good hole transporting performance, including, but not limited to, an arylamine derivative, a carbazole derivative, and a polymer. Specific examples of a hole transporting material may include, but are not limited to, *N4*,*N4*'-bis(biphenyl-4-yl)-*N4*,*N4*'-diphenylbiphenyl-4,4'-diamine (TPD-10), *N,N'*-diphenyl-*N,N'*-(1-naphthalenyl)-1,1'-biphenyl-4,4'-diamine (NPB), and 1,3,5-tris(9-carbazolyl)benzene (TCB). The fluorene-containing triarylamine compound of Formula 1 in the present disclosure is preferred.

The electron blocking layer of the present disclosure preferably uses a material with a relatively good hole transporting ability and electron blocking ability. The electron blocking layer includes, but is not limited to, an arylamine derivative and a carbazole derivative. Specific examples may include, but are not limited to, *N,N*-bis(naphthalen-1-yl)-*N,N-*bis(phenyl)benzidine (NPD) and *N,N-*bis([1,1'-biphenyl]-4-)-(9H-carbazol-9-yl)-[1,1'-biphenyl]-4-amine.

The light-emitting layer of the present disclosure includes a host material and a doped material. A doping proportion of the host material and the doped material may be determined according to a material used. Generally, the doping proportion of the doped material is 0.01% to 20%, preferably 0.1% to 15%, and more preferably 1% to 10%.

The host material of the light-emitting layer requires not only a bipolar charge transporting property but also a proper energy level to effectively transfer excitation energy to a guest light-emitting material. The host material includes, but is not limited to, a heterocyclic compound, an aromatic amine compound, a fused aromatic ring derivative, a metal complex, and a silicon-containing compound. Specific examples may include, but are not limited to, 4,4'-bis(carbazol-9-yl)biphenyl (CBP), 1,3-bis(*N*-carbazolyl)benzene (MCP), 1,3,5-tris(carbazol-9-yl)benzene (TCP), 9,10-di(2-naphthyl)anthracene (ADN), 2-methyl-9,10-bis(naphthalen-2-yl)anthracene (MADN), and tris(8-hydroxyquinolinato)aluminum (Alq₃).

The doped material of the present disclosure may use a fluorescent compound including a pyrene derivative, a fluoranthene derivative, an aromatic amine derivative, and the like or may use a phosphorescent material, for example, but not limited to, a metal complex such as an iridium complex and a platinum complex. Specific examples may include, but are not limited to, tris(2-phenylpyridine)iridium (Ir(ppy)₃), tris[2-(3-methyl-2-pyridinyl)phenyl]iridium (Ir(3mppy)₃), bis(2-phenylpyridine)(acetylacetonate)iridium (Ir(ppy)₂(acac)), bis(1-phenylisoquinoline)(acetylacetonate)iridium (Ir(piq)₂(acac)), tris(1-phenyl-isoquinoline) iridium(III) (Ir(piq)₃), and 2,5,8,11-tetra-*tert*-butylperylene (TBPe).

The hole blocking layer of the present disclosure preferably uses a material with a relatively good electron transporting ability and hole blocking ability. The hole blocking layer includes, but is not limited to, a metal complex and a heteroaromatic compound. Specific examples may include, but are not limited to, bis(2-methyl-8-quinolinolato-N1,O8)-(1,1'-biphenyl-4-olato)aluminum (BAlq) and 2,9-dimethyl-4,7-diphenyl-1,10-phenanthroline (BCP).

The electron transporting layer of the present disclosure preferably uses a material with relatively good stability and a relatively high electron mobility, including, but not limited to, a metal complex, a heteroaromatic compound, and a polymer. Specific examples may include, but are not limited to, tris(8-hydroxyquinolinato)aluminum (Alq₃), tris(4-methyl-8-hydroxyquinoline)aluminum (Al(4-Mq)₃), bis(10-hydroxybenzo[*h*]quinoline)beryllium (Bepq₂), 4,4'-bis(4,6-diphenyl-1,3,5-triazin-2-yl)biphenyl (BTB), 2,9-bis(naphthalen-2-yl)-4,7-diphenyl-1,10-phenanthroline (NBphen), and 2-(4-biphenyl)-5-(4-*t*-butylphenyl)-1,3,4-oxadiazole (PBD).

The electron injection layer of the present disclosure preferably uses a material with a relatively good electron injection ability, including, but not limited to, a metal, a metal compound, and a metal oxide. Specific examples may include, but are not limited to, calcium (Ca), ytterbium (Yb), lithium fluoride (LiF), 8-hydroxyquinolinolato-lithium (LiQ), calcium fluoride (CaF₂), rubidium acetate (CH₃COORb), and lithium oxide (Li₂O).

The cathode of the present disclosure preferably uses a material with good conductive performance and a relatively low work function, including, but not limited to, a metal and a metal alloy. Specific examples of a cathode material may include, but are not limited to, aluminum (Al), silver (Ag), gold (Au), calcium (Ca), strontium (Sr), lithium (Li), magnesium (Mg), a magnesium-silver alloy (Mg/Ag), and a lithium-aluminum alloy (Li/Al).

The capping layer of the present disclosure preferably uses a material with a relatively high glass transition temperature and excellent light extraction performance, including, but not limited to, an arylamine derivative, a metal compound, and a carbazole derivative. Specific examples may include, but are not limited to, tris(8-hydroxyquinolinato)aluminum (Alq₃) and 4,4'-bis(carbazol-9-yl)biphenyl (CBP).

A method for preparing the films in the organic electroluminescent device of the present disclosure is not particularly limited, which may use, but is not limited to, a vacuum evaporation method, a sputtering method, a spin coating method, a spraying method, a screen printing method, and a laser transfer printing method.

The organic electroluminescent device of the present disclosure is mainly applied to the field of information display technology and the field of lighting and is widely applied to various information displays in the aspect of information display, such as a mobile phone, a tablet computer, a flat-panel television, a smart watch, VR, an in-vehicle system, a digital camera, and a wearable device.

### Synthesis Example

Raw materials and reagents: The raw materials or reagents used in the following synthesis examples are not particularly limited in the present disclosure and may be commercially available products or be prepared by preparation methods well-known to those skilled in the art. The raw materials and reagents used in the present disclosure are all reagent pure.

Instruments: G2-Si quadrupole tandem time-of-flight high-resolution mass spectrometer (Waters Co., Britain); Vario EL cube elemental analyzer (Elementar Co., Germany).

The method for preparing the fluorene-containing triarylamine compound of the present disclosure represented by Formula 1 is not particularly limited, which may use a conventional method well-known to those skilled in the art. For example, a carbon-nitrogen coupling reaction is carried out. The fluorene-containing triarylamine compound of the present disclosure represented by Formula 1 may be prepared by using the synthesis routes shown below.

Synthesis route 1: When Ar₁-L₁-Xn is different from Ar₂-L₂-Xn, the reaction route is as follows:

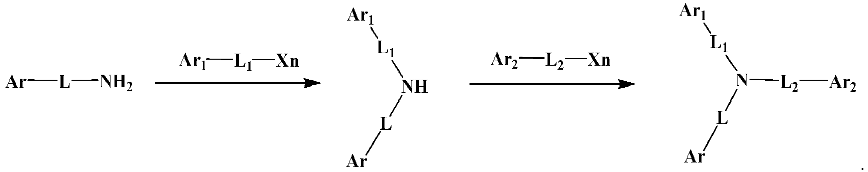

Synthesis route 2: When Ar₁-L₁-Xn is the same as Ar₂-L₂-Xn, the reaction route is as follows: 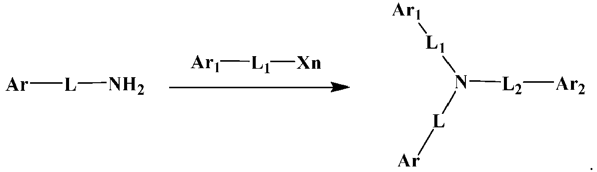 Xn is halogen. For example, Xn is identically or differently selected from Cl, Br, or I.

### Synthesis Example 1: Preparation of Intermediate A-50

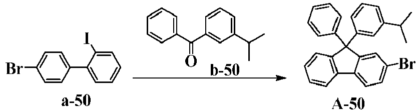

a-50 (35.90 g, 100.00 mmol) was dissolved in 190.00 mL of anhydrous tetrahydrofuran. Under nitrogen, the temperature of the solution was kept at -78 °C, and a solution of n-butyl lithium in hexane (40.00 mL, 2.5M) was slowly added dropwise to the solution and stirred for 2.5 h after the addition. b-50 (22.41 g, 100.00 mmol) was dissolved in 250.00 mL of tetrahydrofuran and slowly added dropwise. The reaction solution was kept at -78 °C and stirred for 2 h, heated to room temperature, and stirred overnight. The solvent was removed under reduced pressure. The residue was dissolved in glacial acetic acid (150.00 mL) and added with a concentrated hydrochloric acid solution (15.00 mL, 31%). The mixture was reacted for 6 h under a reflux condition and stirred overnight at room temperature. After the reaction was completed, the solution was added with water and extracted with dichloromethane. The organic phases were combined, washed with water, and dried over anhydrous magnesium sulfate. The solvent was removed under reduced pressure. The system was recrystallized from isopropanol to obtain Intermediate A-50 (30.76 g, with a yield of 70%) with a purity ≥ 99.82% by HPLC. Mass spectrometry (m/z): 438.0970 (Calcd.: 438.0983).

### Synthesis Example 2: Preparation of Intermediate B-50

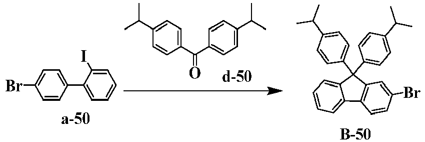

According to the same preparation method in Synthesis Example 1, b-50 was replaced with equimolar d-50 to obtain Intermediate B-50 (32.74 g) with a purity ≥ 99.72% by HPLC. Mass spectrometry (m/z): 480.1469 (Calcd.: 480.1453).

### Synthesis Example 3: Preparation of Intermediate A-144

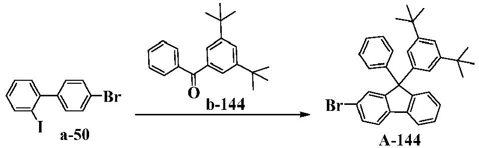

According to the same preparation method in Synthesis Example 1, b-50 was replaced with equimolar b-144 to obtain Intermediate A-144 (32.61g) with a purity ≥ 99.83% by HPLC. Mass spectrometry (m/z): 508.1754 (Calcd.: 508.1766).

### Synthesis Example 4: Preparation of Intermediate A-166

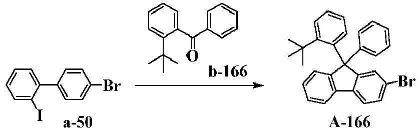

According to the same preparation method in Synthesis Example 1, b-50 was replaced with equimolar b-166 to obtain Intermediate A-166 (31.29 g) with a purity ≥ 99.78% by HPLC. Mass spectrometry (m/z): 452.1159 (Calcd.: 452.1140).

### Synthesis Example 5: Preparation of Intermediate A-173

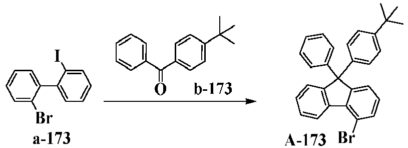

According to the same preparation method in Synthesis Example 1, a-50 and b-50 were replaced with equimolar a-173 and b-173 respectively to obtain Intermediate A-173 (31.74 g) with a purity ≥ 99.70% by HPLC. Mass spectrometry (m/z): 452.1157 (Calcd.: 452.1140).

### Synthesis Example 6: Preparation of Intermediate A-174

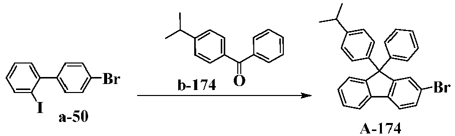

According to the same preparation method in Synthesis Example 1, b-50 was replaced with equimolar b-174 to obtain Intermediate A-174 (31.20 g) with a purity ≥ 99.72% by HPLC. Mass spectrometry (m/z): 438.0970 (Calcd.: 438.0983).

### Synthesis Example 7: Preparation of Intermediate A-185

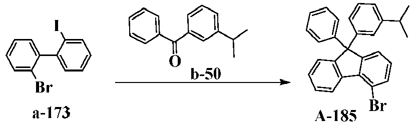

According to the same preparation method in Synthesis Example 1, a-50 was replaced with equimolar a-173 to obtain Intermediate A-185 (30.32 g) with a purity ≥ 99.79% by HPLC. Mass spectrometry (m/z): 438.0974 (Calcd.: 438.0983).

### Synthesis Example 8: Preparation of Intermediate A-256

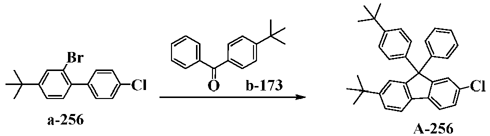

According to the same preparation method in Synthesis Example 1, a-50 and b-50 were replaced with equimolar a-256 and b-173 respectively to obtain Intermediate A-256 (30.70 g) with a purity ≥ 99.81% by HPLC. Mass spectrometry (m/z): 464.2282 (Calcd.: 464.2271).

### Synthesis Example 9: Preparation of Intermediate A-345

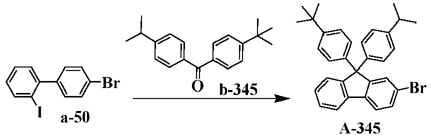

According to the same preparation method in Synthesis Example 1, b-50 was replaced with equimolar b-345 to obtain Intermediate A-345 (32.21 g) with a purity ≥ 99.76% by HPLC. Mass spectrometry (m/z): 494.1622 (Calcd.: 494.1609).

### Synthesis Example 10: Preparation of Compound 50

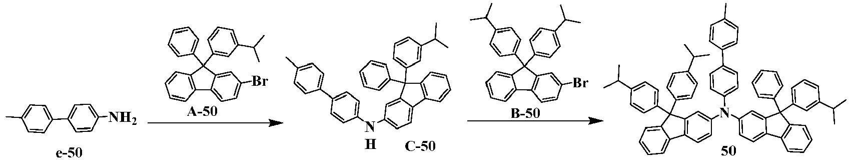

### Preparation of Intermediate C-50:

Under nitrogen protection, e-50 (9.16 g, 50.00 mmol), A-50 (21.97 g, 50.00 mmol), sodium *tert*-butoxide (7.21 g, 75.00 mmol), Pd(OAc)₂ (0.11 g, 0.50 mmol), BINAP (0.39 g, 0.60 mmol), and 400 mL of toluene were added in sequence to a reaction flask. The mixture was stirred, and the system was refluxed for 4 h. After the reaction was completed, the system was cooled to room temperature, added with distilled water, and extracted with dichloromethane and stood still to separate layers. The organic layer was collected, dried over anhydrous magnesium sulfate, filtered, and distilled under reduced pressure to concentrate the filtrate. The filtrate was cooled to crystallize and suction-filtered. The obtained solid was recrystallized from toluene/ethanol (with a volume ratio of 8:1) to obtain Intermediate C-50 (20.59 g, with a yield of 76%) with a purity ≥ 99.89% by HPLC. Mass spectrometry (m/z): 541.2761 (Calcd.: 541.2770).

### Preparation of Compound 50

Under nitrogen protection, Intermediate C-50 (16.25 g, 30.00 mmol), Intermediate B-50 (14.44 g, 30.00 mmol), sodium *tert*-butoxide (4.32 g, 45.00 mmol), Pd₂(dba)₃ (0.27 g, 0.30 mmol), X-Phos (0.29 g, 0.60 mmol), and 200 mL of toluene were added in sequence to a reaction flask. The mixture was stirred, and the system was refluxed for 5 h. After the reaction was completed, the system was cooled to room temperature, added with distilled water, and extracted with dichloromethane and stood still to separate layers. The organic layer was collected, dried over anhydrous magnesium sulfate, filtered, and distilled under reduced pressure to concentrate the filtrate. The filtrate was cooled to crystallize and suction-filtered. The obtained solid was recrystallized from toluene to obtain Compound 50 (20.35 g, with a yield of 72%) with a purity ≥ 99.95% by HPLC. Mass spectrometry (m/z): 941.4978 (Calcd.: 941.4961). Theoretical element content (%) of C₇₂H₆₃N: C, 91.77; H, 6.74; N, 1.49. Measured element content (%): C, 91.80; H, 6.70; N, 1.51.

### Synthesis Example 11: Preparation of Compound 58

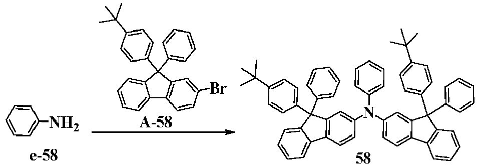

Under nitrogen protection, e-58 (2.79 g, 30.00 mmol), Intermediate A-58 (27.21 g, 60.00 mmol), sodium tert-butoxide (4.32 g, 45.00 mmol), Pd₂(dba)₃ (0.27 g, 0.30 mmol), tri-*tert*-butyl phosphine (0.12 g, 0.60 mmol), and 300 mL of toluene were added in sequence to a reaction flask. The mixture was stirred, and the system was refluxed for 5 h. After the reaction was completed, the system was cooled to room temperature, added with distilled water, and extracted with dichloromethane and stood still to separate layers. The organic layer was collected, dried over anhydrous magnesium sulfate, filtered, and distilled under reduced pressure to concentrate the filtrate. The filtrate was cooled to crystallize and suction-filtered. The obtained solid was recrystallized from toluene to obtain Compound 58 (18.86 g, with a yield of 75%) with a purity ≥ 99.93% by HPLC. Mass spectrometry (m/z): 837.4348 (Calcd.: 837.4335). Theoretical element content (%) of C₆₄H₅₅N: C, 91.71; H, 6.61; N, 1.67. Measured element content (%): C, 91.69; H, 6.58; N, 1.71.

### Synthesis Example 12: Preparation of Compound 75

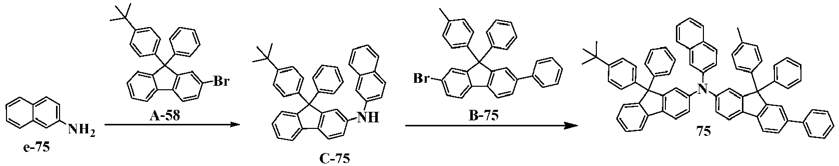

According to the same preparation method in Synthesis Example 10, e-50, A-50, and B-50 were replaced with equimolar e-75, A-58, and B-75 respectively to obtain Compound 75 (19.09 g) with a purity ≥ 99.92% by HPLC. Mass spectrometry (m/z): 921.4320 (Calcd.: 921.4335). Theoretical element content (%) of C₇₁H₅₅N: C, 92.47; H, 6.01; N, 1.52. Measured element content (%): C, 92.50; H, 6.03; N, 1.47.

### Synthesis Example 13: Preparation of Compound 94

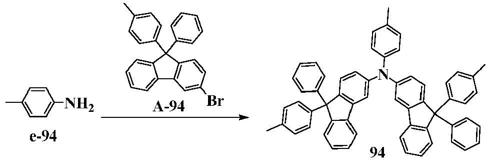

According to the same preparation method in Synthesis Example 11, e-58 and A-58 were replaced with equimolar e-94 and A-94 respectively to obtain Compound 94 (17.97 g) with a purity ≥ 99.94% by HPLC. Mass spectrometry (m/z): 767.3566 (Calcd.: 767.3552). Theoretical element content (%) of C₅₉H₄₅N: C, 92.27; H, 5.91; N, 1.82. Measured element content (%): C, 92.29; H, 5.87; N, 1.87.

### Synthesis Example 14: Preparation of Compound 139

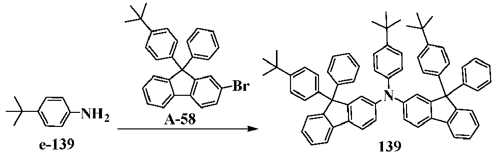

According to the same preparation method in Synthesis Example 11, e-58 was replaced with equimolar e-139 to obtain Compound 139 (20.12 g) with a purity ≥ 99.94% by HPLC. Mass spectrometry (m/z): 893.4969 (Calcd.: 893.4961). Theoretical element content (%) of C₆₈H₆₃N: C, 91.33; H, 7.10; N, 1.57. Measured element content (%): C, 91.28; H, 7.08; N, 1.62.

### Synthesis Example 15: Preparation of Compound 144

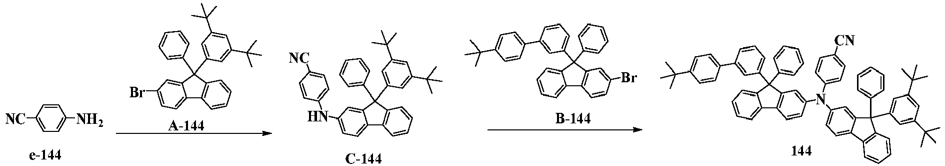

According to the same preparation method in Synthesis Example 10, e-50, A-50, and B-50 were replaced with equimolar e-144, A-144, and B-144 respectively to obtain Compound 144 (19.71 g) with a purity ≥ 99.96% by HPLC. Mass spectrometry (m/z): 994.5242 (Calcd.: 994.5226). Theoretical element content (%) of C₇₅H₆₆N₂: C, 90.50; H, 6.68; N, 2.81. Measured element content (%): C, 90.54; H, 6.63; N, 2.83.

### Synthesis Example 16: Preparation of Compound 163

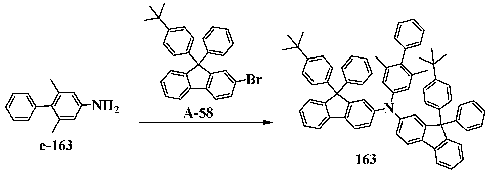

According to the same preparation method in Synthesis Example 11, e-58 was replaced with equimolar e-163 to obtain Compound 163 (21.20 g) with a purity ≥ 99.94% by HPLC. Mass spectrometry (m/z): 941.4943 (Calcd.: 941.4961). Theoretical element content (%) of C₇₂H₆₃N: C, 91.77; H, 6.74; N, 1.49. Measured element content (%): C, 91.72; H, 6.76; N, 1.51.

### Synthesis Example 17: Preparation of Compound 166

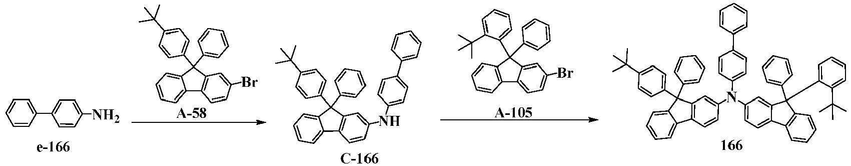

According to the same preparation method in Synthesis Example 10, e-50, A-50, and B-50 were replaced with equimolar e-166, A-58, and A-105 respectively to obtain Compound 166 (20.02 g) with a purity ≥ 99.91% by HPLC. Mass spectrometry (m/z): 913.4656 (Calcd.: 913.4648). Theoretical element content (%) of C₇₀H₅₉N: C, 91.96; H, 6.51; N, 1.53. Measured element content (%): C, 91.98; H, 6.46; N, 1.56.

### Synthesis Example 18: Preparation of Compound 173

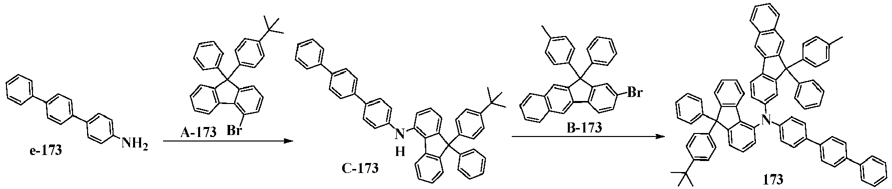

According to the same preparation method in Synthesis Example 10, e-50, A-50, and B-50 were replaced with equimolar e-173, A-173, and B-173 respectively to obtain Compound 173 (20.07 g) with a purity ≥ 99.98% by HPLC. Mass spectrometry (m/z): 997.4630 (Calcd.: 997.4648). Theoretical element content (%) of C₇₇H₅₉N: C, 92.64; H, 5.96; N, 1.40. Measured element content (%): C, 92.67; H, 5.92; N, 1.38.

### Synthesis Example 19: Preparation of Compound 174

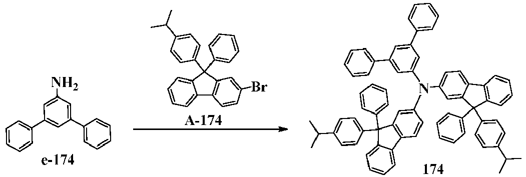

According to the same preparation method in Synthesis Example 11, e-58 and A-58 were replaced with equimolar e-174 and A-174 respectively to obtain Compound 174 (20.50 g) with a purity ≥ 99.93% by HPLC. Mass spectrometry (m/z): 961.4658 (Calcd.: 961.4648). Theoretical element content (%) of C₇₄H₅₉N: C, 92.36; H, 6.18; N, 1.46. Measured element content (%): C, 92.34; H, 6.14; N, 1.43.

### Synthesis Example 20: Preparation of Compound 177

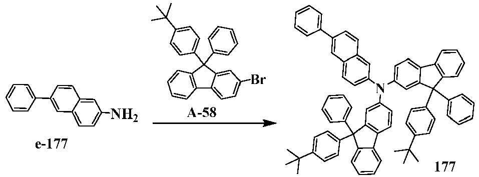

According to the same preparation method in Synthesis Example 11, e-58 was replaced with equimolar e-177 to obtain Compound 177 (19.67 g) with a purity ≥ 99.97% by HPLC. Mass spectrometry (m/z): 963.4820 (Calcd.: 963.4804). Theoretical element content (%) of C₇₄H₆₁N: C, 92.17; H, 6.38; N, 1.45. Measured element content (%): C, 92.21; H, 6.40; N, 1.40.

### Synthesis Example 21: Preparation of Compound 182

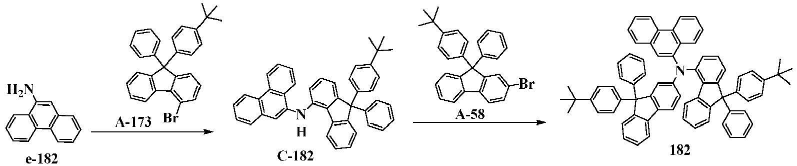

According to the same preparation method in Synthesis Example 10, e-50, A-50, and B-50 were replaced with equimolar e-182, A-173, and A-58 respectively to obtain Compound 182 (19.70 g) with a purity ≥ 99.93% by HPLC. Mass spectrometry (m/z): 937.4659 (Calcd.: 937.4648). Theoretical element content (%) of C₇₂H₅₉N: C, 92.17; H, 6.34; N, 1.49. Measured element content (%): C, 92.15; H, 6.38; N, 1.52.

### Synthesis Example 22: Preparation of Compound 185

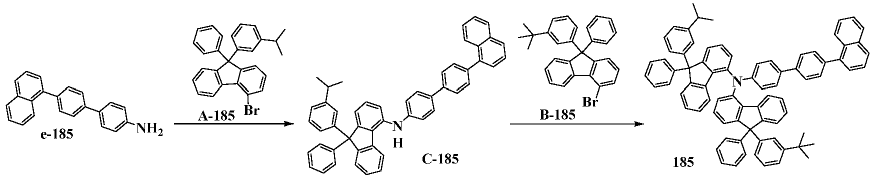

According to the same preparation method in Synthesis Example 10, e-50, A-50, and B-50 were replaced with equimolar e-185, A-185, and B-185 respectively to obtain Compound 185 (20.01 g) with a purity ≥ 99.97% by HPLC. Mass spectrometry (m/z): 1025.4941 (Calcd.: 1025.4961). Theoretical element content (%) of C₇₉H₆₃N: C, 92.45; H, 6.19; N, 1.36. Measured element content (%): C, 92.43; H, 6.16; N, 1.40.

### Synthesis Example 23: Preparation of Compound 186

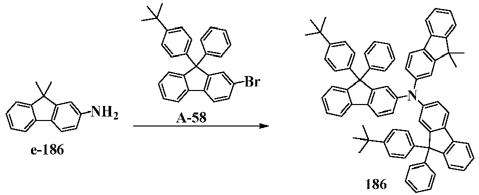

According to the same preparation method in Synthesis Example 11, e-58 was replaced with equimolar e-186 to obtain Compound 186 (19.75 g) with a purity ≥ 99.94% by HPLC. Mass spectrometry (m/z): 953.4971 (Calcd.: 953.4961). Theoretical element content (%) of C₇₃H₆₃N: C, 91.88; H, 6.65; N, 1.47. Measured element content (%): C, 91.92; H, 6.61; N, 1.50.

### Synthesis Example 24: Preparation of Compound 202

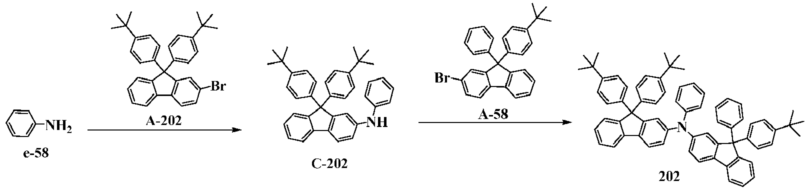

According to the same preparation method in Synthesis Example 10, e-50, A-50, and B-50 were replaced with equimolar e-58, A-202, and A-58 respectively to obtain Compound 202 (19.85 g) with a purity ≥ 99.93% by HPLC. Mass spectrometry (m/z): 893.4943 (Calcd.: 893.4961). Theoretical element content (%) of C₆₈H₆₃N: C, 91.33; H, 7.10; N, 1.57. Measured element content (%): C, 91.31; H, 7.08; N, 1.53.

### Synthesis Example 25: Preparation of Compound 239

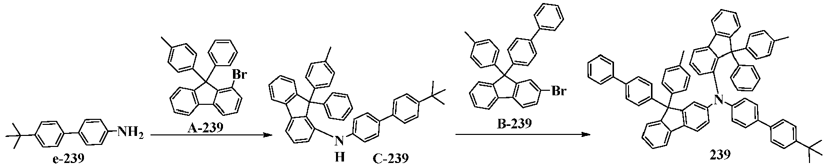

According to the same preparation method in Synthesis Example 10, e-50, A-50, and B-50 were replaced with equimolar e-239, A-239, and B-239 respectively to obtain Compound 239 (19.63 g) with a purity ≥ 99.91% by HPLC. Mass spectrometry (m/z): 961.4661 (Calcd.: 961.4648). Theoretical element content (%) of C₇₄H₅₉N: C, 92.36; H, 6.18; N, 1.46. Measured element content (%): C, 92.40; H, 6.20; N, 1.41.

### Synthesis Example 26: Preparation of Compound 246

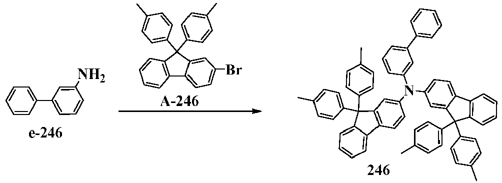

According to the same preparation method in Synthesis Example 11, e-58 and A-58 were replaced with equimolar e-246 and A-246 respectively to obtain Compound 246 (19.57 g) with a purity ≥ 99.96% by HPLC. Mass spectrometry (m/z): 857.4038 (Calcd.: 857.4022). Theoretical element content (%) of C₆₆H₅₁N: C, 92.38; H, 5.99; N, 1.63. Measured element content (%): C, 92.35; H, 5.95; N, 1.67.

### Synthesis Example 27: Preparation of Compound 250

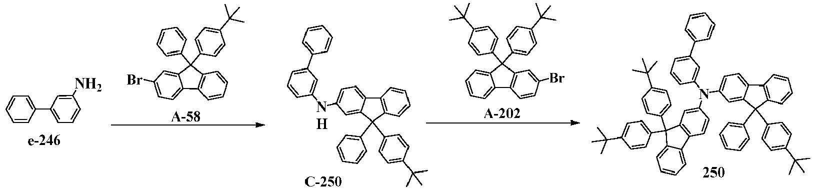

According to the same preparation method in Synthesis Example 10, e-50, A-50, and B-50 were replaced with equimolar e-246, A-58, and A-202 respectively to obtain Compound 250 (21.54 g) with a purity ≥ 99.91% by HPLC. Mass spectrometry (m/z): 969.5282 (Calcd.: 969.5274). Theoretical element content (%) of C₇₄H₆₇N: C, 91.60; H, 6.96; N, 1.44. Measured element content (%): C, 91.56; H, 6.94; N, 1.49.

### Synthesis Example 28: Preparation of Compound 256

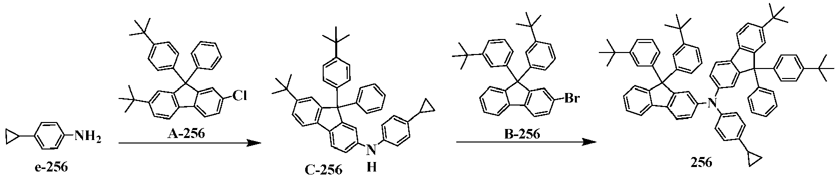

According to the same preparation method in Synthesis Example 10, e-50, A-50, and B-50 were replaced with equimolar e-256, A-256, and B-256 respectively to obtain Compound 256 (20.50 g) with a purity ≥ 99.94% by HPLC. Mass spectrometry (m/z): 989.5909 (Calcd.: 989.5900). Theoretical element content (%) of C₇₅H₇₅N: C, 90.95; H, 7.63; N, 1.41. Measured element content (%): C, 90.90; H, 7.59; N, 1.39.

### Synthesis Example 29: Preparation of Compound 257

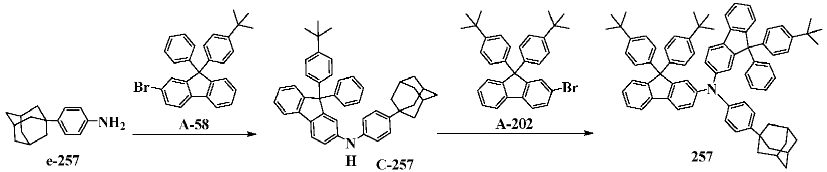

According to the same preparation method in Synthesis Example 10, e-50, A-50, and B-50 were replaced with equimolar e-257, A-58, and A-202 respectively to obtain Compound 257 (20.36 g) with a purity ≥ 99.95% by HPLC. Mass spectrometry (m/z): 1027.6044 (Calcd.: 1027.6056). Theoretical element content (%) of C₇₈H₇₇N: C, 91.09; H, 7.55; N, 1.36. Measured element content (%): C, 91.05; H, 7.57; N, 1.40.

### Synthesis Example 30: Preparation of Compound 264

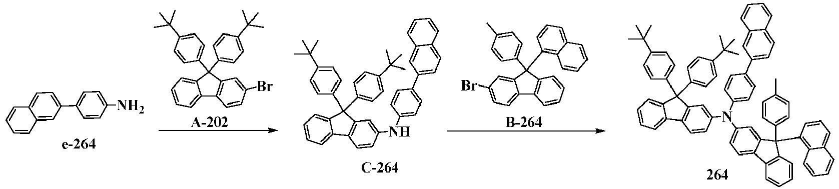

According to the same preparation method in Synthesis Example 10, e-50, A-50, and B-50 were replaced with equimolar e-264, A-202, and B-264 respectively to obtain Compound 264 (20.05 g) with a purity ≥ 99.92% by HPLC. Mass spectrometry (m/z): 1027.5106 (Calcd.: 1027.5117). Theoretical element content (%) of C₇₉H₆₅N: C, 92.27; H, 6.37; N, 1.36. Measured element content (%): C, 92.24; H, 6.35; N, 1.38.

### Synthesis Example 31: Preparation of Compound 285

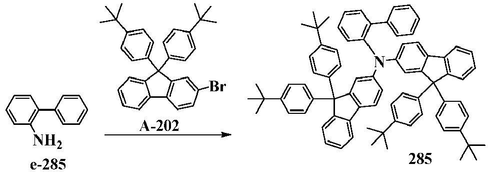

According to the same preparation method in Synthesis Example 11, e-58 and A-58 were replaced with equimolar e-285 and A-202 respectively to obtain Compound 285 (21.56 g) with a purity ≥ 99.96% by HPLC. Mass spectrometry (m/z): 1025.5914 (Calcd.: 1025.5900). Theoretical element content (%) of C₇₈H₇₅N: C, 91.27; H, 7.37; N, 1.36. Measured element content (%): C, 91.22; H, 7.40; N, 1.38.

### Synthesis Example 32: Preparation of Compound 345

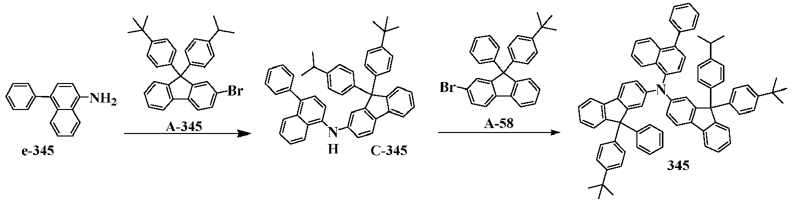

According to the same preparation method in Synthesis Example 10, e-50, A-50, and B-50 were replaced with equimolar e-345, A-345, and A-58 respectively to obtain Compound 345 (20.53 g) with a purity ≥ 99.95% by HPLC. Mass spectrometry (m/z): 1005.5289 (Calcd.: 1005.5274). Theoretical element content (%) of C₇₇H₆₇N: C, 91.90; H, 6.71; N, 1.39. Measured element content (%): C, 91.86; H, 6.68; N, 1.44.

### Synthesis Example 33: Preparation of Compound 392

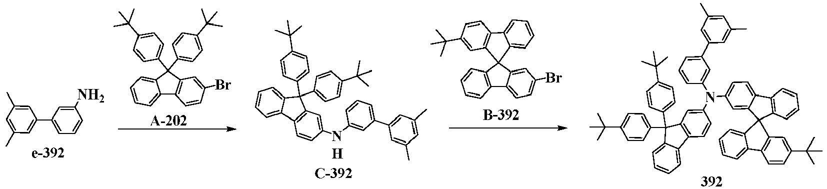

According to the same preparation method in Synthesis Example 10, e-50, A-50, and B-50 were replaced with equimolar e-392, A-202, and B-392 respectively to obtain Compound 392 (20.03 g) with a purity ≥ 99.95% by HPLC. Mass spectrometry (m/z): 995.5448 (Calcd.: 995.5430). Theoretical element content (%) of C₇₆H₆₉N: C, 91.61; H, 6.98; N, 1.41. Measured element content (%): C, 91.57; H, 6.96; N, 1.46.

### Synthesis Example 34: Preparation of Compound 417

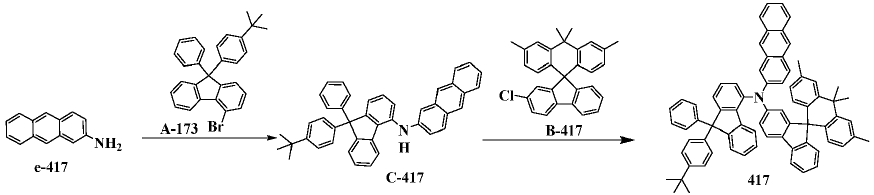

According to the same preparation method in Synthesis Example 10, e-50, A-50, and B-50 were replaced with equimolar e-417, A-173, and B-417 respectively to obtain Compound 417 (19.67 g) with a purity ≥ 99.96% by HPLC. Mass spectrometry (m/z): 949.4636 (Calcd.: 949.4648). Theoretical element content (%) of C₇₃H₅₉N: C, 92.27; H, 6.26; N, 1.47. Measured element content (%): C, 92.22; H, 6.30; N, 1.45.

### Device Example

In the present disclosure, an ITO/Ag/ITO glass substrate was ultrasonically cleaned twice with a 5% glass cleaning solution, 20 minutes each time, and then ultrasonically cleaned twice with deionized water, 10 minutes each time. The ITO/Ag/ITO glass substrate was ultrasonically cleaned for 20 minutes with acetone and isopropyltone in sequence and dried at 120 °C. All organic materials were sublimated, each with a purity above 99.99%.

Test software, a computer, a K2400 digital source meter manufactured by Keithley, USA, and a PR788 spectral scanning photometer manufactured by Photo Research, USA were combined into a combined IVL test system to test the drive voltage, luminescence efficiency, and CIE color coordinates of an organic electroluminescent device. The lifetime was tested by using an M6000 OLED lifetime test system from McScience. The test was conducted in an atmospheric environment and at room temperature.

### Example 1: Preparation of organic electroluminescent device 1

2-TNATA was deposited on an ITO/Ag/ITO anode through vacuum evaporation as a hole injection layer with a thickness of 58 nm; Compound 50 of the present disclosure was deposited on the hole injection layer through vacuum evaporation as a hole transporting layer with a thickness of 110 nm; a host material RH-1 and a doped material RD-1 were deposited on the hole transporting layer through vacuum evaporation as a light-emitting layer with a thickness of 21 nm, where the doping ratio of RH-1:RD-1 was 98:2 (wt%); BTB:LiQ = 50:50 (wt%) were deposited on the light-emitting layer through vacuum evaporation as an electron transporting layer with a thickness of 28 nm; LiF was deposited on the electron transporting layer through vacuum evaporation as an electron injection layer with a thickness of 1.1 nm; Mg:Ag = 1:9 were deposited on the electron injection layer through vacuum evaporation as a cathode with a thickness of 12 nm; and CP-1 was deposited on the cathode through vacuum evaporation as a capping layer with a thickness of 65 nm.

### Examples 2 to 25: Preparation of organic electroluminescent devices 2 to 25

Compound 50 in the hole transporting layer of Example 1 was replaced with Compound 58, Compound 75, Compound 94, Compound 139, Compound 144, Compound 163, Compound 166, Compound 173, Compound 174, Compound 177, Compound 182, Compound 185, Compound 186, Compound 202, Compound 239, Compound 246, Compound 250, Compound 256, Compound 257, Compound 264, Compound 285, Compound 345, Compound 392, and Compound 417 separately, with the other steps the same, so as to obtain organic electroluminescent devices 2 to 25.

### Comparative Examples 1 and 2: Preparation of comparative organic electroluminescent devices 1 and 2

Compound 50 in the hole transporting layer of Example 1 was replaced with R-1 and R-2 separately, with the other steps the same, so as to obtain comparative organic electroluminescent devices 1 and 2.

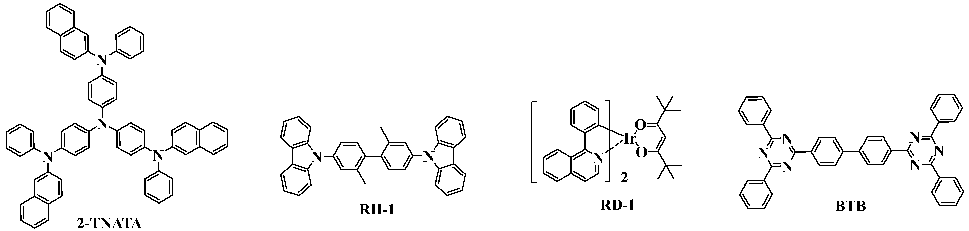

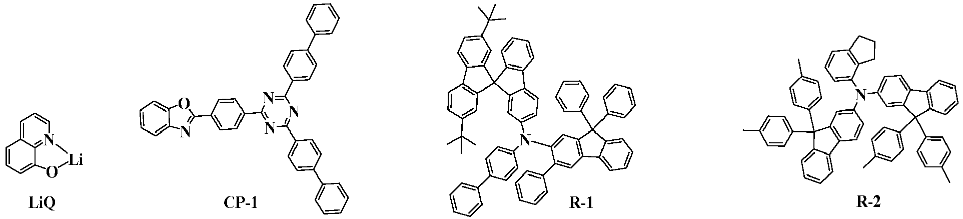

The test results of the luminescence characteristics of the organic electroluminescent devices prepared in Examples 1 to 25 and Comparative Examples 1 and 2 of the present disclosure are shown in Table 1.

**Table 1 Test data of the luminescence characteristics of the organic electroluminescent devices**

| Example | Material of the Hole Transporting Layer | Voltage [V] | Efficiency [cd/A] (@10mA/cm²) | Lifetime [T95, h] (@10mA/cm²) |
|---|---|---|---|---|
| Example 1 | Compound 50 | 4.4 | 40.24 | 204.1 |
| Example 2 | Compound 58 | 4.4 | 40.51 | 204.5 |
| Example 3 | Compound 75 | 4.5 | 39.00 | 201.4 |
| Example 4 | Compound 94 | 4.4 | 40.70 | 205.0 |
| Example 5 | Compound 139 | 4.4 | 40.79 | 205.2 |
| Example 6 | Compound 144 | 4.6 | 38.21 | 200.3 |
| Example 7 | Compound 163 | 4.4 | 40.59 | 204.7 |
| Example 8 | Compound 166 | 4.4 | 40.38 | 204.3 |
| Example 9 | Compound 173 | 4.5 | 38.75 | 201.0 |
| Example 10 | Compound 174 | 4.5 | 39.73 | 203.0 |
| Example 11 | Compound 177 | 4.5 | 39.60 | 202.7 |
| Example 12 | Compound 182 | 4.5 | 39.07 | 201.5 |
| Example 13 | Compound 185 | 4.5 | 39.24 | 202.0 |
| Example 14 | Compound 186 | 4.5 | 39.38 | 202.2 |
| Example 15 | Compound 202 | 4.4 | 40.08 | 203.7 |
| Example 16 | Compound 239 | 4.5 | 39.15 | 201.7 |
| Example 17 | Compound 246 | 4.4 | 39.88 | 203.3 |
| Example 18 | Compound 250 | 4.4 | 40.16 | 204.0 |
| Example 19 | Compound 256 | 4.5 | 38.84 | 201.2 |
| Example 20 | Compound 257 | 4.4 | 39.97 | 203.5 |
| Example 21 | Compound 264 | 4.5 | 38.63 | 200.8 |
| Example 22 | Compound 285 | 4.4 | 39.80 | 203.1 |
| Example 23 | Compound 345 | 4.5 | 39.51 | 202.4 |
| Example 24 | Compound 392 | 4.6 | 38.44 | 200.6 |
| Example 25 | Compound 417 | 4.6 | 38.06 | 200.0 |
| Comparative Example 1 | R-1 | 5.0 | 28.54 | 146.8 |
| Comparative Example 2 | R-2 | 4.9 | 30.05 | 152.5 |

As can be seen from Table 1, compared with comparative devices 1 and 2, the organic electroluminescent devices each including the fluorene-containing triarylamine compound of Formula 1 in the present disclosure in the hole transporting layer have a lower drive voltage, higher luminescence efficiency, and a longer service lifetime, and the devices have better performance.

### Example 26: Preparation of organic electroluminescent device 26

HAT-CN was deposited on an ITO/Ag/ITO anode through vacuum evaporation as a hole injection layer with a thickness of 15 nm; NPB was deposited on the hole injection layer through vacuum evaporation as a first hole transporting layer with a thickness of 80 nm; Compound 50 of the present disclosure was deposited on the first hole transporting layer through vacuum evaporation as a second hole transporting layer with a thickness of 40 nm; a host material GH-1 and a doped material GD-1 were deposited on the second hole transporting layer through vacuum evaporation as a light-emitting layer with a thickness of 22 nm, where the doping ratio of GH-1:GD-1 was 92:8 (wt%); BCP:LiQ = 50:50 (wt%) were deposited on the light-emitting layer through vacuum evaporation as an electron transporting layer with a thickness of 29 nm; LiF was deposited on the electron transporting layer through vacuum evaporation as an electron injection layer with a thickness of 1.1 nm; Mg:Ag = 1:9 were deposited on the electron injection layer through vacuum evaporation as a cathode with a thickness of 13 nm; and CP-1 was deposited on the cathode through vacuum evaporation as a capping layer with a thickness of 72 nm.

### Examples 27 to 50: Preparation of organic electroluminescent devices 27 to 50

Compound 50 in the second hole transporting layer of Example 26 was replaced with Compound 58, Compound 75, Compound 94, Compound 139, Compound 144, Compound 163, Compound 166, Compound 173, Compound 174, Compound 177, Compound 182, Compound 185, Compound 186, Compound 202, Compound 239, Compound 246, Compound 250, Compound 256, Compound 257, Compound 264, Compound 285, Compound 345, Compound 392, and Compound 417 separately, with the other steps the same, so as to obtain organic electroluminescent devices 27 to 50.

### Comparative Examples 3 and 4: Preparation of comparative organic electroluminescent devices 3 and 4

Compound 50 in the second hole transporting layer of Example 26 was replaced with R-3 and R-4 separately, with the other steps the same, so as to obtain comparative organic electroluminescent devices 3 and 4.

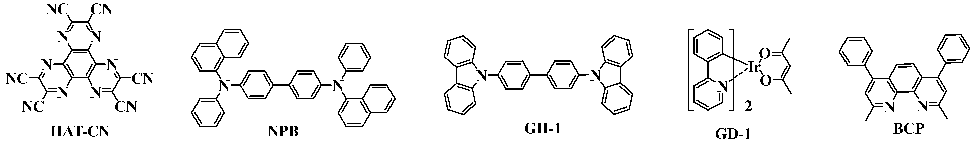

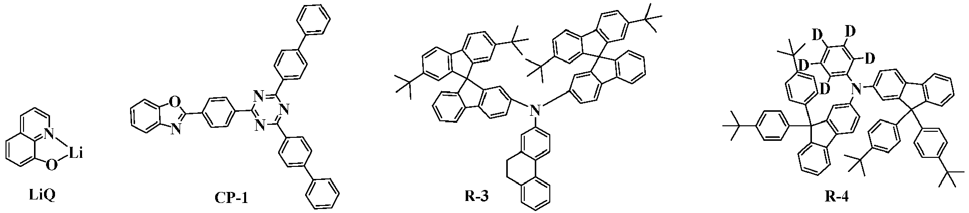

The test results of the luminescence characteristics of the organic electroluminescent devices prepared in Examples 26 to 50 and Comparative Examples 3 and 4 of the present disclosure are shown in Table 2.

**Table 2 Test data of the luminescence characteristics of the organic electroluminescent devices**

| Example | Material of the Second Hole Transporting Layer | Voltage [V] | Efficiency [cd/A] (@10mA/cm²) | Lifetime [T97, h] (@10mA/cm²) |
|---|---|---|---|---|
| Example 26 | Compound 50 | 3.9 | 46.00 | 219.3 |
| Example 27 | Compound 58 | 3.9 | 46.20 | 219.8 |
| Example 28 | Compound 75 | 4.0 | 44.71 | 216.5 |
| Example 29 | Compound 94 | 3.9 | 46.38 | 220.2 |
| Example 30 | Compound 139 | 3.9 | 46.44 | 220.3 |
| Example 31 | Compound 144 | 4.2 | 44.15 | 215.4 |
| Example 32 | Compound 163 | 3.9 | 46.29 | 220.0 |
| Example 33 | Compound 166 | 3.9 | 46.12 | 219.6 |
| Example 34 | Compound 173 | 4.1 | 44.46 | 216.0 |
| Example 35 | Compound 174 | 3.9 | 45.40 | 218.1 |
| Example 36 | Compound 177 | 3.9 | 45.28 | 217.9 |
| Example 37 | Compound 182 | 4.0 | 44.80 | 216.7 |
| Example 38 | Compound 185 | 4.0 | 45.00 | 217.1 |
| Example 39 | Compound 186 | 4.0 | 45.10 | 217.4 |
| Example 40 | Compound 202 | 3.9 | 45.85 | 218.8 |
| Example 41 | Compound 239 | 4.0 | 44.87 | 216.9 |
| Example 42 | Compound 246 | 3.9 | 45.60 | 218.5 |
| Example 43 | Compound 250 | 3.9 | 45.94 | 219.0 |
| Example 44 | Compound 256 | 4.1 | 44.53 | 216.2 |
| Example 45 | Compound 257 | 3.9 | 45.72 | 218.7 |
| Example 46 | Compound 264 | 4.1 | 44.38 | 215.8 |
| Example 47 | Compound 285 | 3.9 | 45.49 | 218.3 |
| Example 48 | Compound 345 | 4.0 | 45.15 | 217.6 |
| Example 49 | Compound 392 | 4.1 | 44.24 | 215.5 |
| Example 50 | Compound 417 | 4.2 | 44.02 | 215.0 |
| Comparative Example 3 | R-3 | 4.8 | 32.55 | 165.7 |
| Comparative Example 4 | R-4 | 4.4 | 39.50 | 190.8 |

As can be seen from Table 2, compared with comparative devices 3 and 4, the organic electroluminescent devices each including the fluorene-containing triarylamine compound of Formula 1 in the present disclosure in the second hole transporting layer have a lower drive voltage, higher luminescence efficiency, and a longer service lifetime, and the devices have better performance.

It is to be noted that although the present disclosure has been particularly described through particular embodiments, those of ordinary skill in the art can make various modifications in form or detail without departing from the principle of the present disclosure and such modifications also fall within the scope of the present disclosure.

## Claims

1. A fluorene-containing triarylamine compound, which is represented by the following Formula 1: 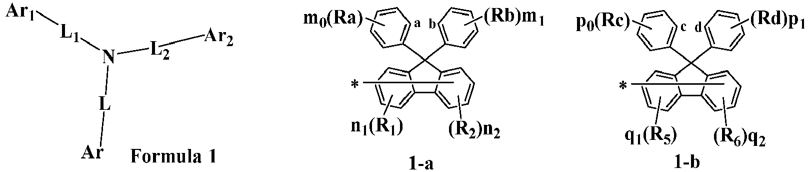 wherein Ar₁ is selected from a group represented by Formula 1-a;
"* " represents a linkage site to L₁;
Rₐ and R_{b} are each identically or differently selected from one of hydrogen, halogen, cyano, nitro, substituted or unsubstituted C1 to C25 alkyl, substituted or unsubstituted C3 to C25 cycloalkyl, and substituted or unsubstituted C6 to C60 aryl or a combination thereof, or adjacent two Rₐ or two R_{b} are bonded to each other to form a substituted or unsubstituted ring;
at least one of Rₐ and R_{b} is selected from substituted or unsubstituted C1 to C25 alkyl;
carbon atoms corresponding to a and b are separate, connected via a single bond, or bonded into a ring;
R₁ and R₂ are each identically or differently selected from one of hydrogen, halogen, cyano, nitro, substituted or unsubstituted C1 to C25 alkyl, substituted or unsubstituted silyl, substituted or unsubstituted C3 to C25 cycloalkyl, and substituted or unsubstituted C6 to C60 aryl, or adjacent two R₁ or two R₂ are bonded to each other to form a substituted or unsubstituted ring;
m₀ is selected from 1, 2, 3, 4, or 5; m₁ is selected from 1, 2, 3, 4, or 5;
n₁ is selected from 0, 1, 2, 3, or 4; n₂ is selected from 0, 1, 2, 3, or 4;
Ar₂ is selected from a group represented by Formula 1-b;
R_{c} and R_{d} are each identically or differently selected from one of hydrogen, halogen, cyano, nitro, substituted or unsubstituted C1 to C25 alkyl, substituted or unsubstituted C3 to C25 cycloalkyl, and substituted or unsubstituted C6 to C60 aryl or a combination thereof, or adjacent two R_{c} or two R_{d} are bonded to each other to form a substituted or unsubstituted ring;
at least one of R_{c} and R_{d} is selected from substituted or unsubstituted C1 to C25 alkyl;
carbon atoms corresponding to c and d are separate or bonded into a ring;
R₅ and R₆ are each identically or differently selected from one of hydrogen, halogen, cyano, nitro, substituted or unsubstituted C1 to C25 alkyl, substituted or unsubstituted silyl, substituted or unsubstituted C3 to C25 cycloalkyl, and substituted or unsubstituted C6 to C60 aryl, or adjacent two R₅ or two R₆ are bonded to each other to form a substituted or unsubstituted ring;
p₀ is selected from 1, 2, 3, 4, or 5; p₁ is selected from 1, 2, 3, 4, or 5;
q₁ is selected from 0, 1, 2, 3, or 4; q₂ is selected from 0, 1, 2, 3, or 4;
Ar is selected from one of substituted or unsubstituted phenyl, substituted or unsubstituted biphenyl, substituted or unsubstituted terphenyl, substituted or unsubstituted naphthyl, substituted or unsubstituted anthryl, substituted or unsubstituted phenanthryl, substituted or unsubstituted triphenylenyl, substituted or unsubstituted pyrenyl, substituted or unsubstituted 9,9-dimethylfluorenyl, substituted or unsubstituted 9,9-diphenylfluorenyl, substituted or unsubstituted 9,9-spirobifluorenyl, and substituted or unsubstituted spiroanthrafluorenyl; the substituent in the "substituted or unsubstituted" is selected from one of cyano, nitro, substituted or unsubstituted C1 to C25 alkyl, substituted or unsubstituted C3 to C25 cycloalkyl, and substituted or unsubstituted C6 to C30 aryl, or adjacent two groups are bonded to each other to form a substituted or unsubstituted benzene ring; and
L, L₁, and L₂ are independently selected from one of a single bond and substituted or unsubstituted C6 to C30 arylene.

2. The fluorene-containing triarylamine compound according to claim 1, wherein the fluorene-containing triarylamine compound is selected from one of Formulas 1-1 to 1-5: 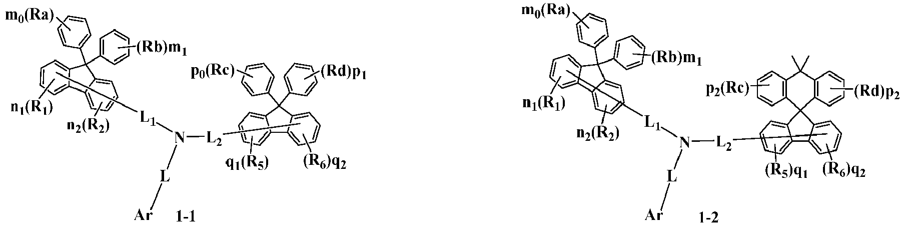 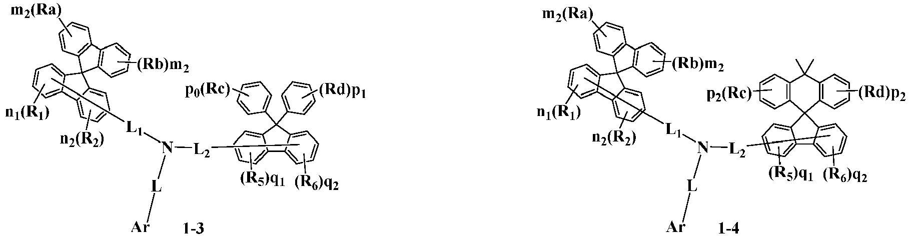 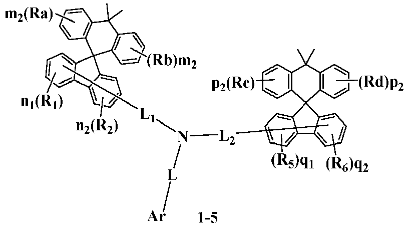 wherein m₀ is selected from 1, 2, 3, 4, or 5; m₁ is selected from 1, 2, 3, 4, or 5; m₂ is selected from 1, 2, 3, or 4; p₀ is selected from 1, 2, 3, 4, or 5; p₁ is selected from 1, 2, 3, 4, or 5; and p₂ is selected from 1, 2, 3, or 4.

3. The fluorene-containing triarylamine compound according to claim 1, wherein Ar₁ is selected from one of the following groups: 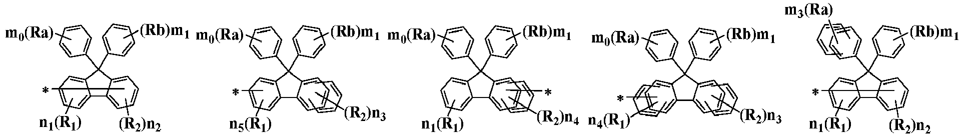 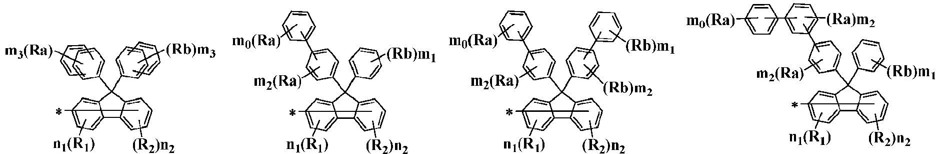 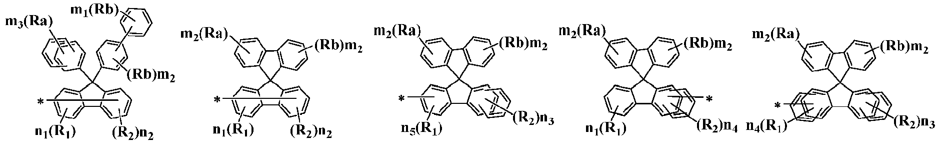 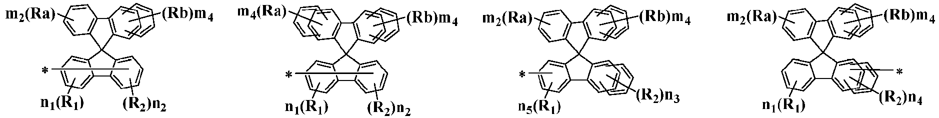 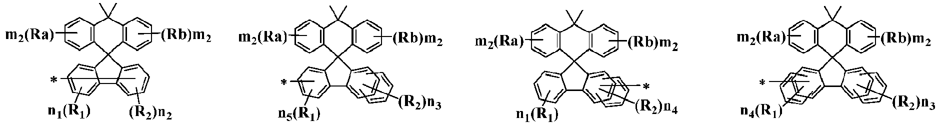 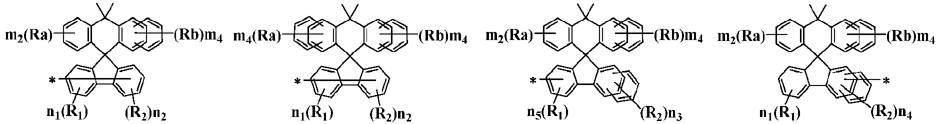 wherein m₀ is selected from 1, 2, 3, 4, or 5; m₁ is selected from 1, 2, 3, 4, or 5; m₂ is selected from 1, 2, 3, or 4; m₃ is selected from 1, 2, 3, 4, 5, 6, or 7; m₁ is selected from 1, 2, 3, 4, 5, or 6; n₁ is selected from 0, 1, 2, 3, or 4; n₂ is selected from 0, 1, 2, 3, or 4; n₃ is selected from 0, 1, 2, 3, 4, 5, or 6; n₄ is selected from 0, 1, 2, 3, 4, or 5; and n₅ is selected from 0, 1, 2, or 3.

4. The fluorene-containing triarylamine compound according to claim 1, wherein Ar₂ is selected from one of the following groups: 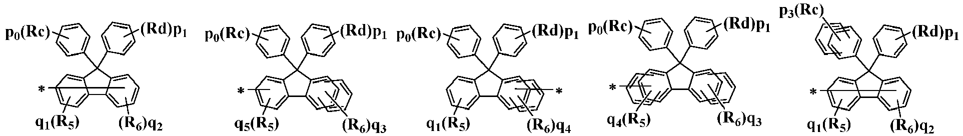 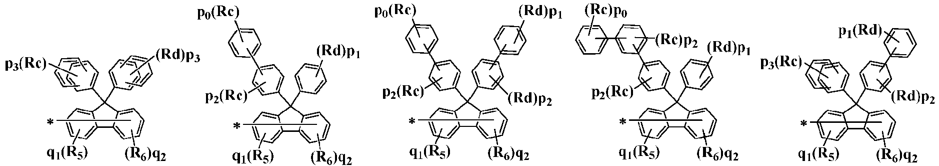 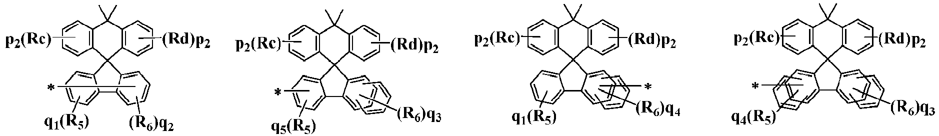 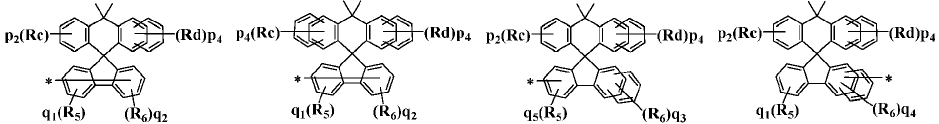 wherein p₀ is selected from 1, 2, 3, 4, or 5; p₁ is selected from 1, 2, 3, 4, or 5; p₂ is selected from 1, 2, 3, or 4; p₃ is selected from 1, 2, 3, 4, 5, 6, or 7; p₄ is selected from 1, 2, 3, 4, 5, or 6; q₁ is selected from 0, 1, 2, 3, or 4; q₂ is selected from 0, 1, 2, 3, or 4; q₃ is selected from 0, 1, 2, 3, 4, 5, or 6; q₄ is selected from 0, 1, 2, 3, 4, or 5; and q₅ is selected from 0, 1, 2, or 3.

5. The fluorene-containing triarylamine compound according to claim 1, wherein at least one of Rₐ and R_{b} is selected from one of substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, and substituted or unsubstituted decyl.

6. The fluorene-containing triarylamine compound according to claim 1, wherein at least one of R_{c} and R_{d} is selected from one of substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, substituted or unsubstituted sec-butyl, substituted or unsubstituted *t*-butyl, substituted or unsubstituted pentyl, substituted or unsubstituted hexyl, substituted or unsubstituted heptyl, substituted or unsubstituted octyl, substituted or unsubstituted nonyl, and substituted or unsubstituted decyl.

7. The fluorene-containing triarylamine compound according to claim 1, wherein Ar is selected from one of the following groups or a combination thereof: 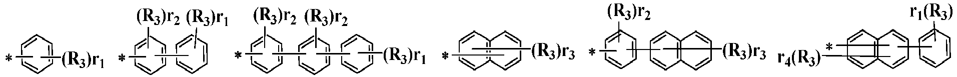 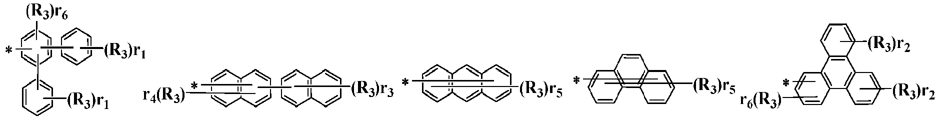 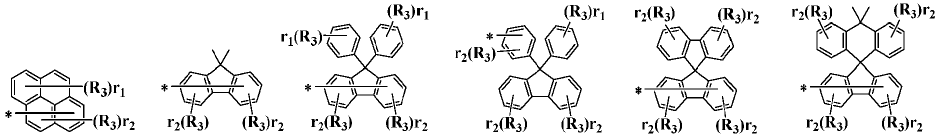
wherein R₃ is selected from one of hydrogen, cyano, nitro, substituted or unsubstituted C1 to C25 alkyl, substituted or unsubstituted C3 to C25 cycloalkyl, and substituted or unsubstituted C6 to C30 aryl, or adjacent two R₃ are bonded to each other to form a substituted or unsubstituted benzene ring; and
wherein r₁ is selected from 0, 1, 2, 3, 4, or 5; r₂ is selected from 0, 1, 2, 3, or 4; r₃ is selected from 0, 1, 2, 3, 4, 5, 6, or 7; r₄ is selected from 0, 1, 2, 3, 4, 5, or 6; r₅ is selected from 0, 1, 2, 3, 4, 5, 6, 7, 8, or 9; and r₆ is selected from 0, 1, 2, or 3.

8. The fluorene-containing triarylamine compound according to claim 1, wherein L, L₁, and L₂ are independently selected from one of a single bond and the following groups or a combination thereof: 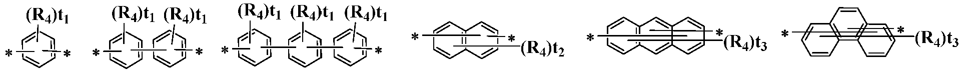 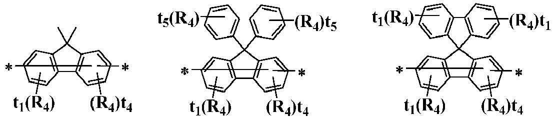
wherein R₄ is selected from one of hydrogen, halogen, cyano, nitro, substituted or unsubstituted C1 to C25 alkyl, substituted or unsubstituted C3 to C25 cycloalkyl, and substituted or unsubstituted C6 to C30 aryl; and
wherein t₁ is selected from 0, 1, 2, 3, or 4; t₂ is selected from 0, 1, 2, 3, 4, 5, or 6; t₃ is selected from 0, 1, 2, 3, 4, 5, 6, 7, or 8; t₄ is selected from 0, 1, 2, or 3; and t₅ is selected from 0, 1, 2, 3, 4, or 5.

9. The fluorene-containing triarylamine compound according to claim 1, wherein the fluorene-containing triarylamine compound is selected from any one of the following structures:

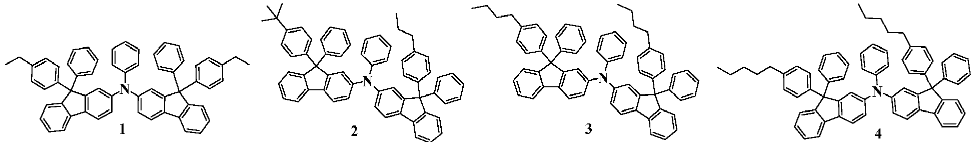

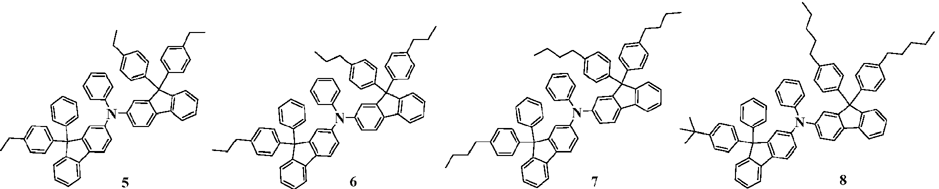

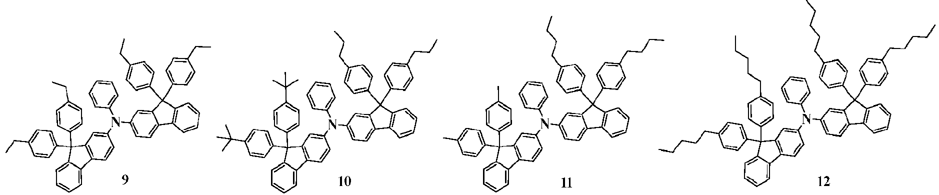

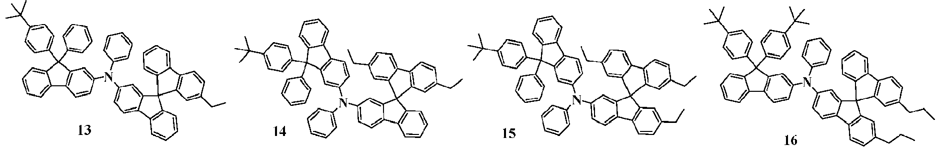

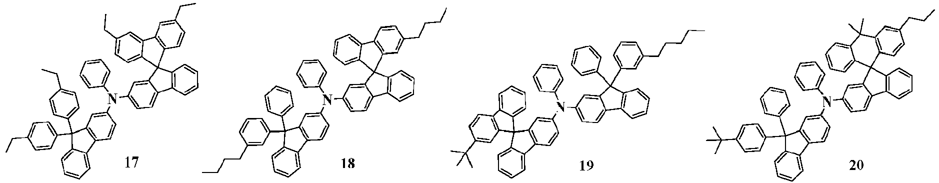

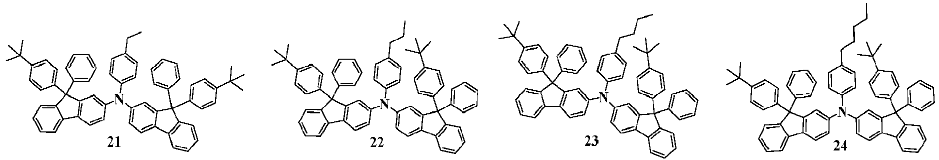

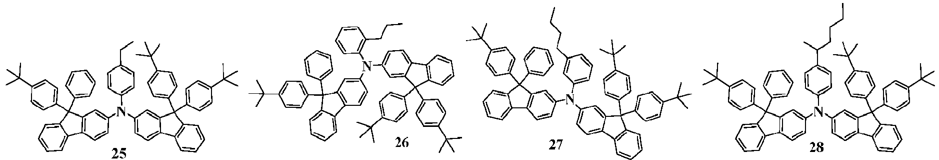

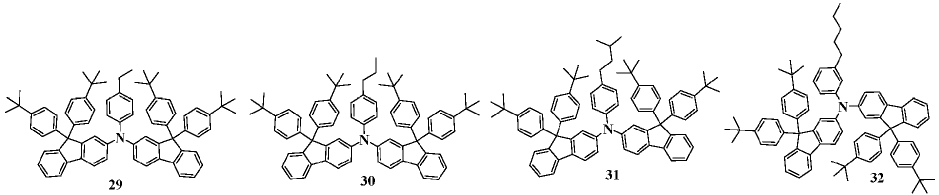

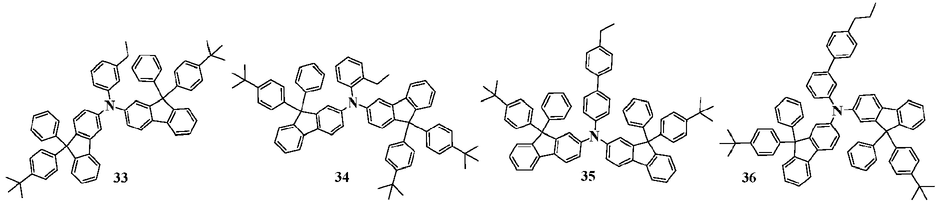

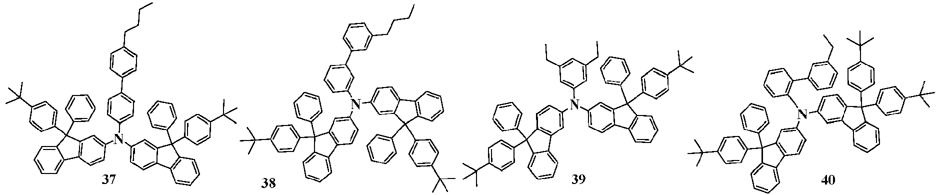

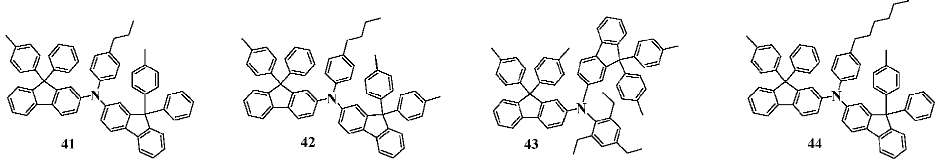

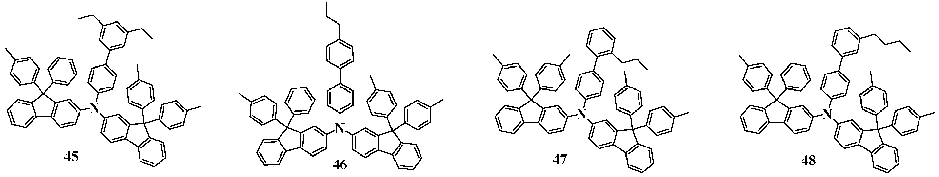

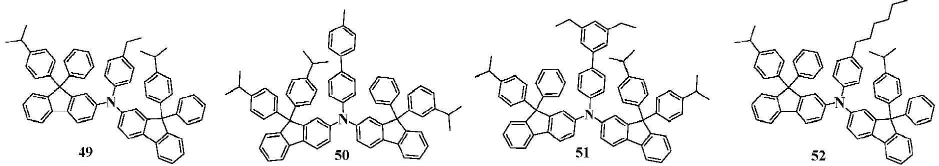

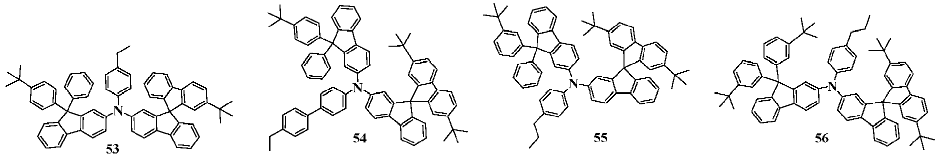

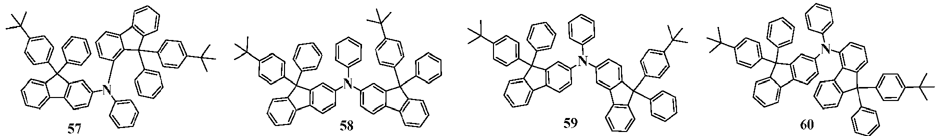

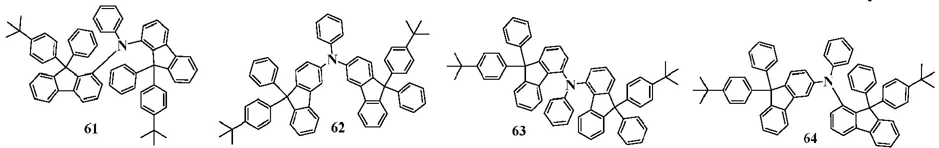

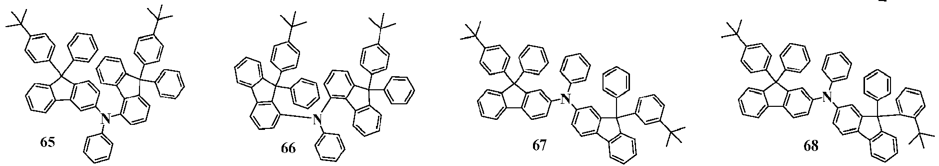

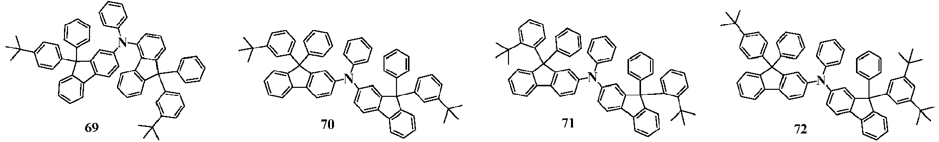

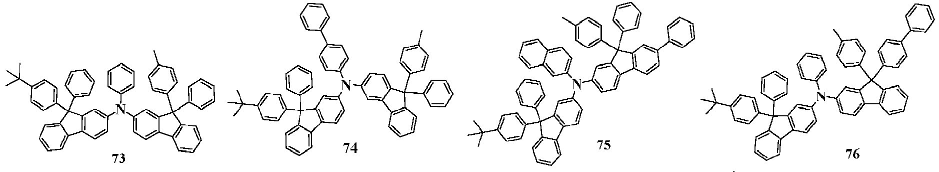

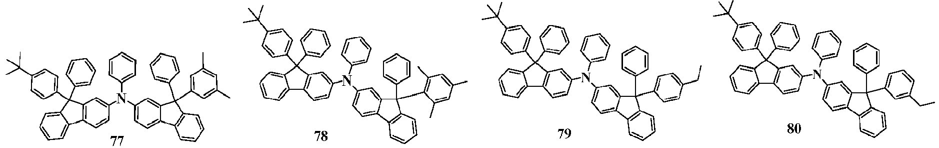

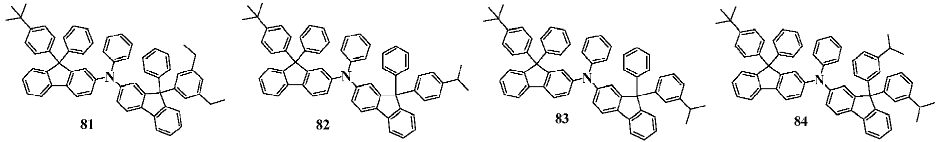

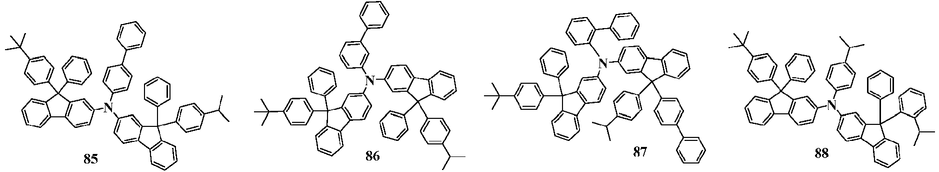

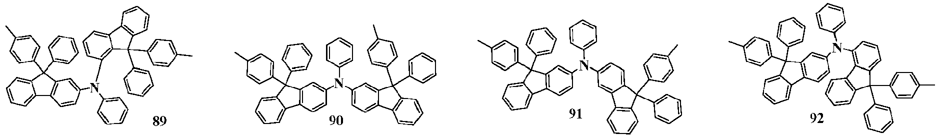

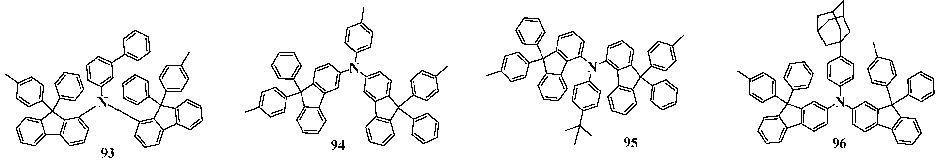

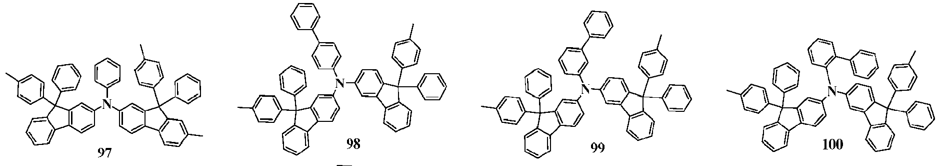

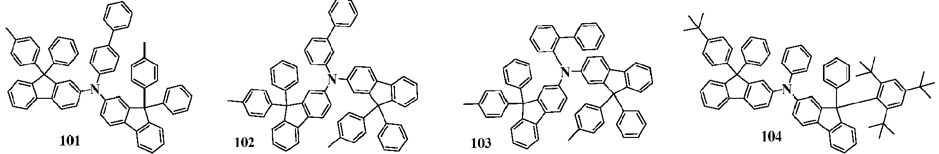

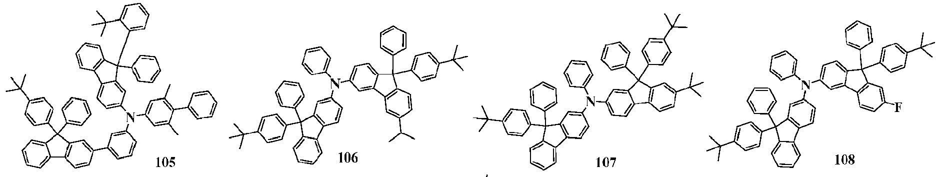

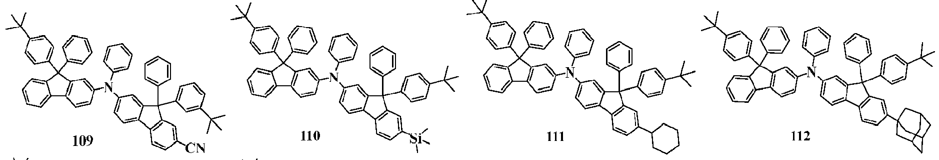

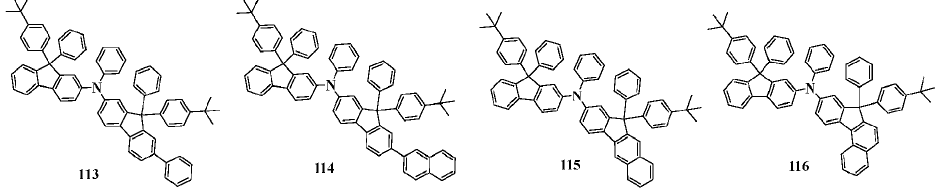

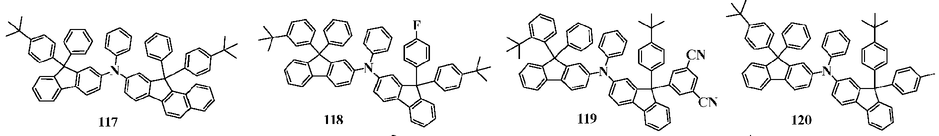

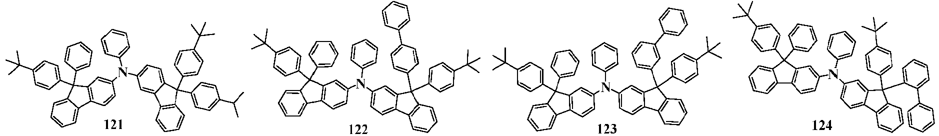

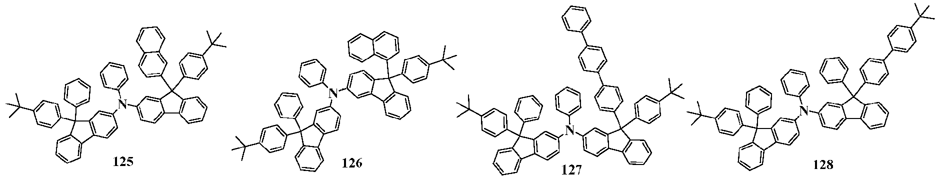

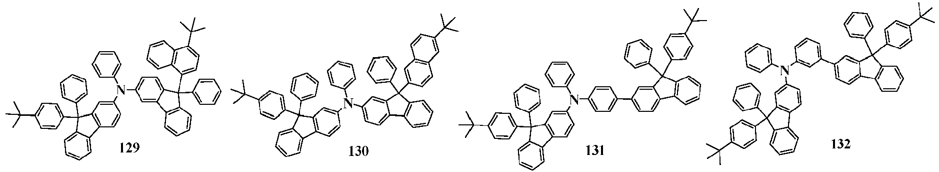

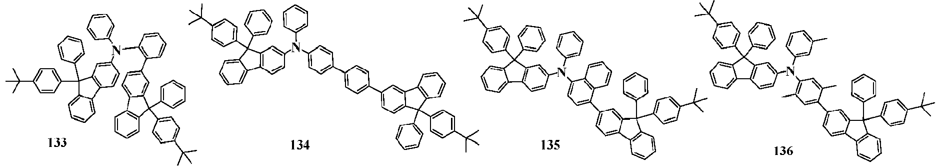

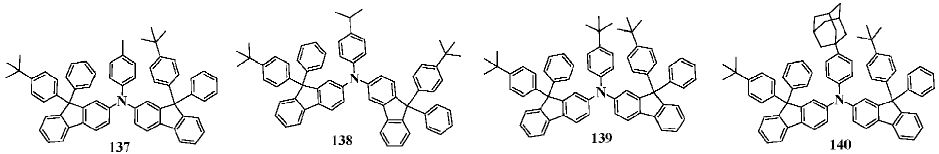

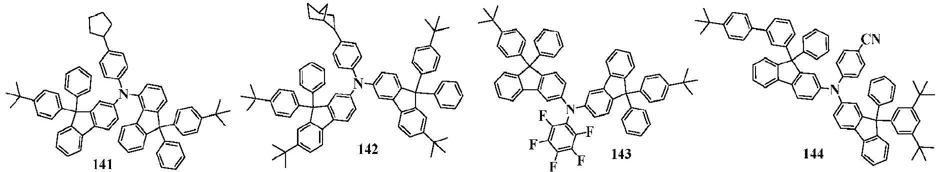

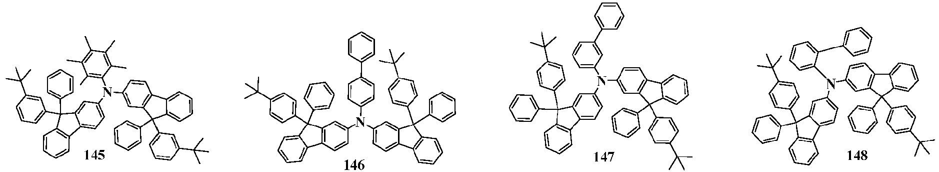

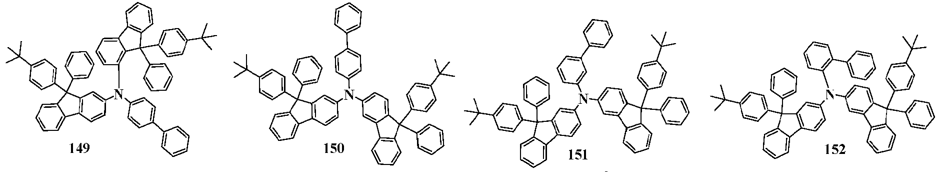

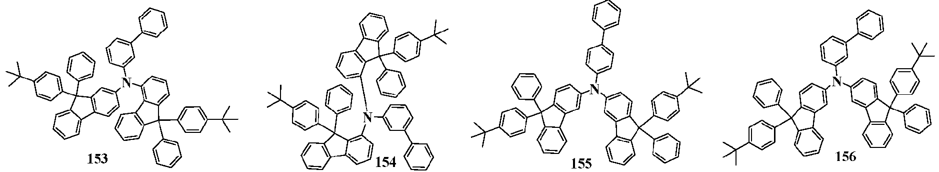

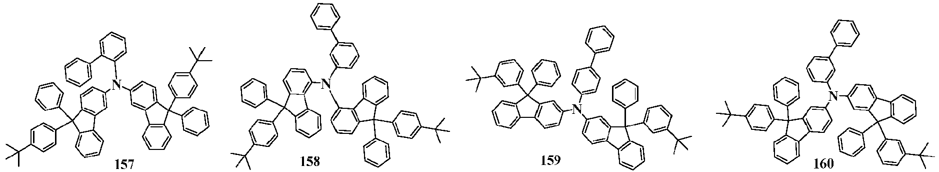

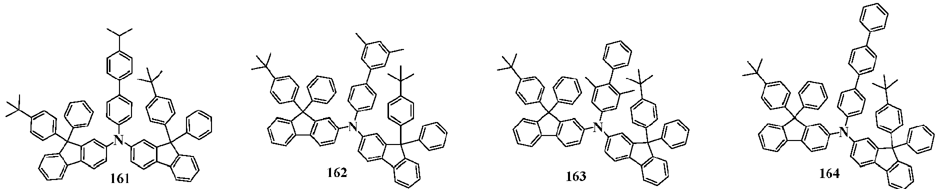

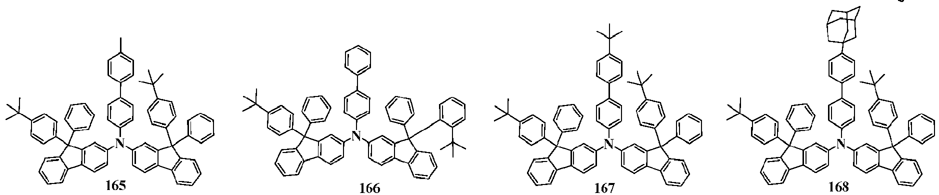

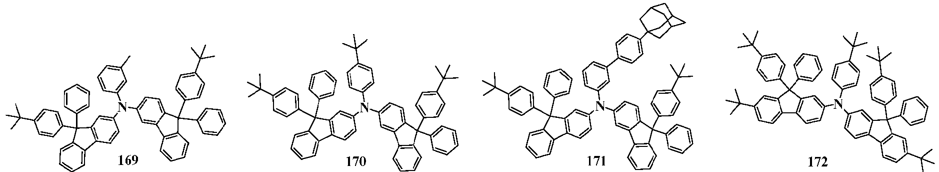

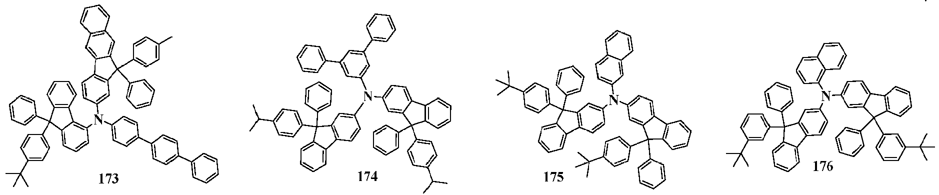

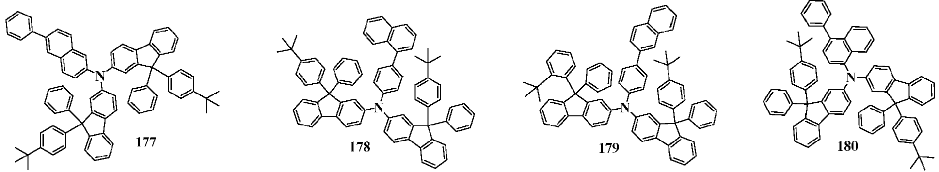

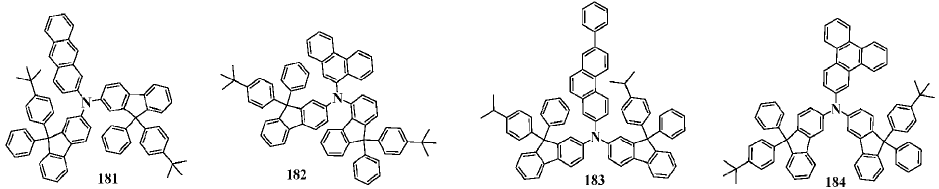

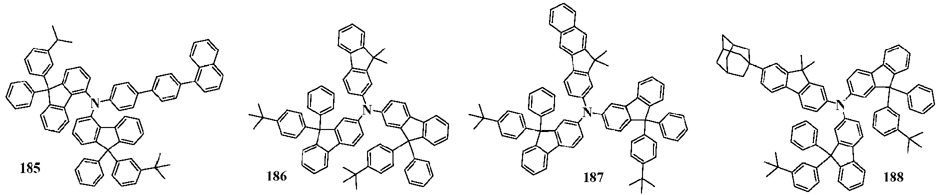

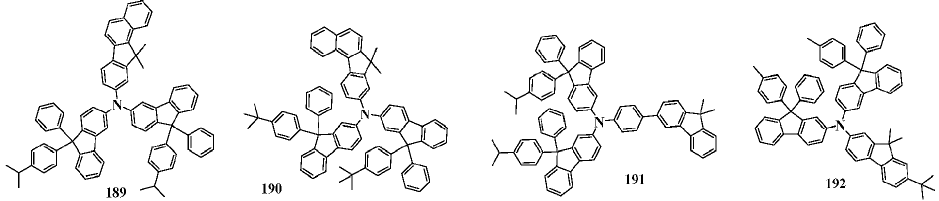

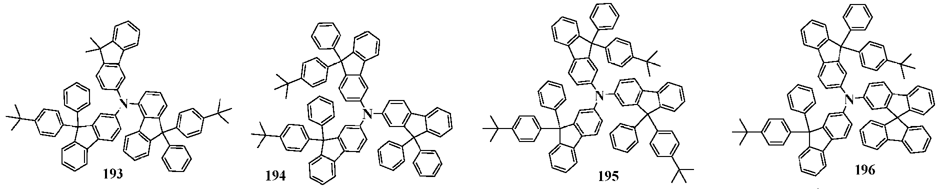

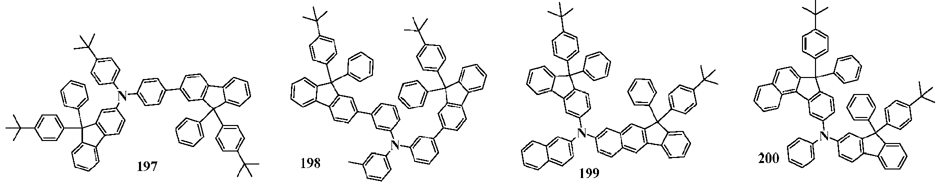

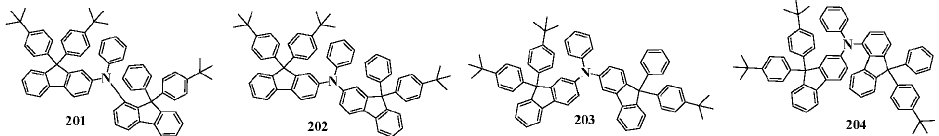

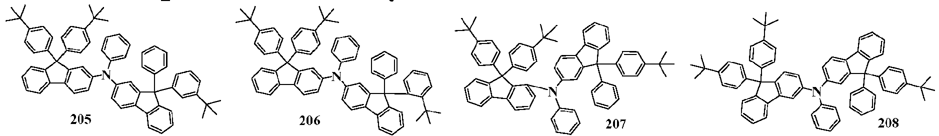

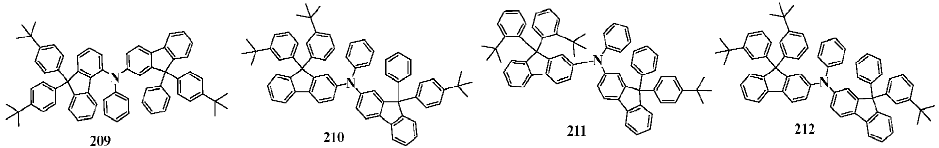

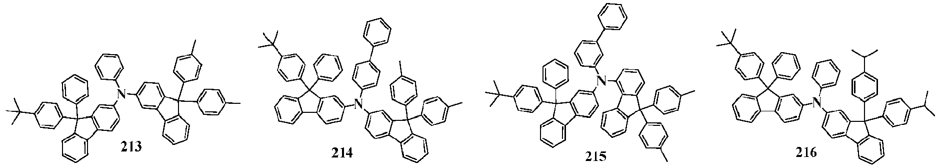

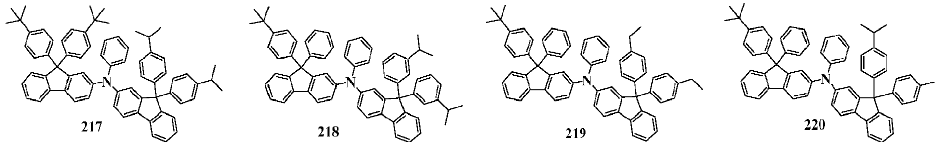

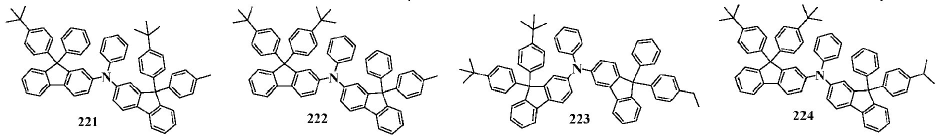

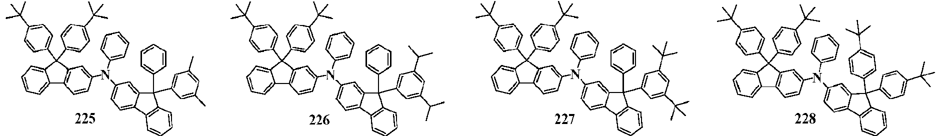

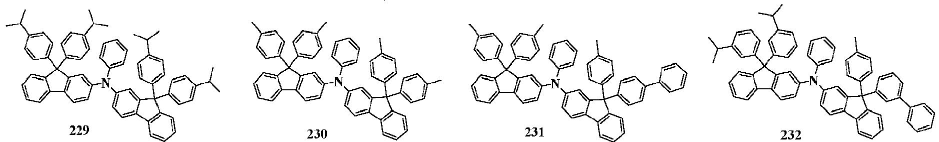

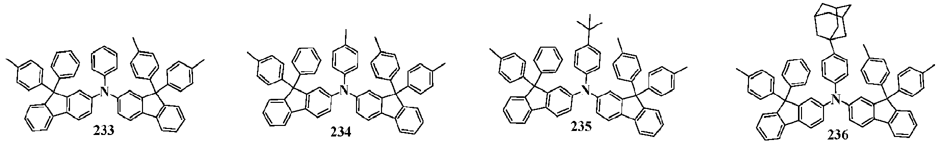

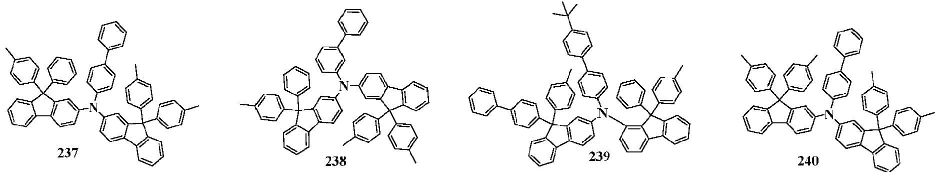

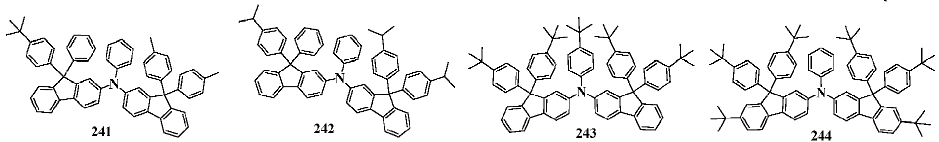

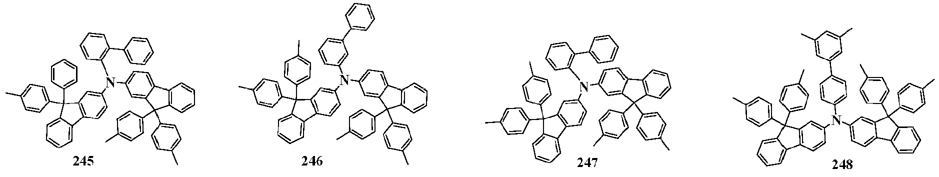

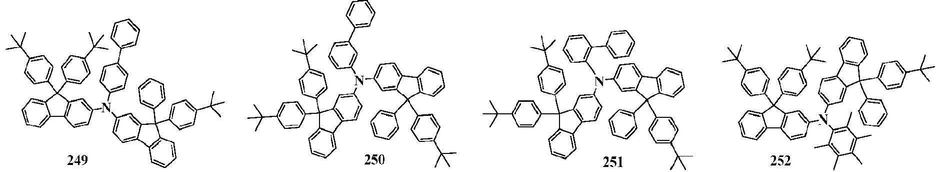

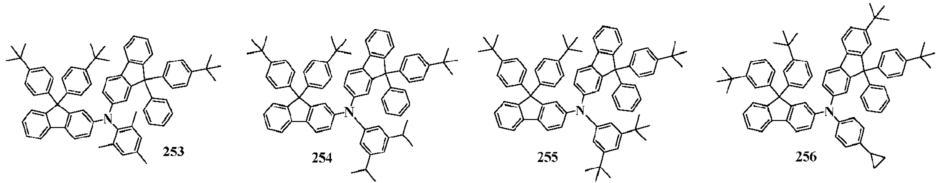

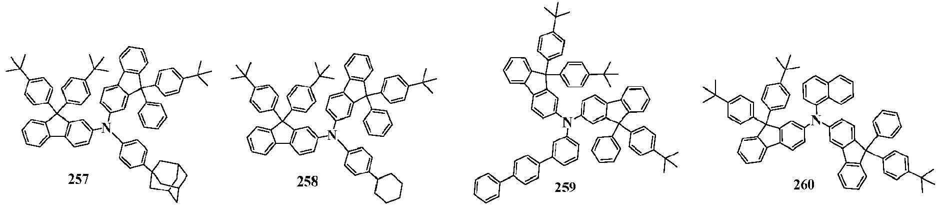

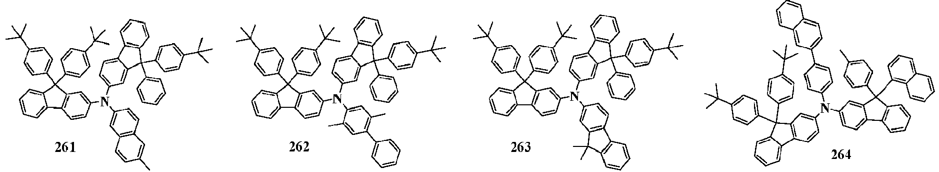

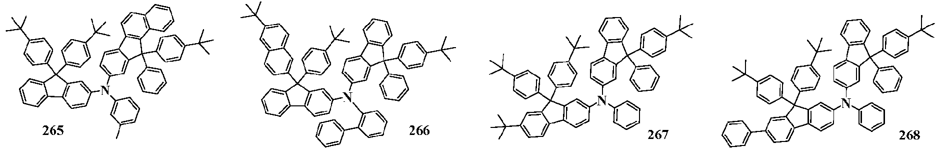

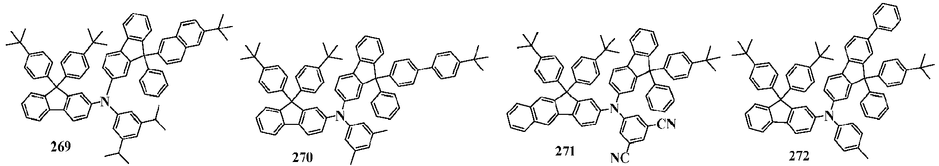

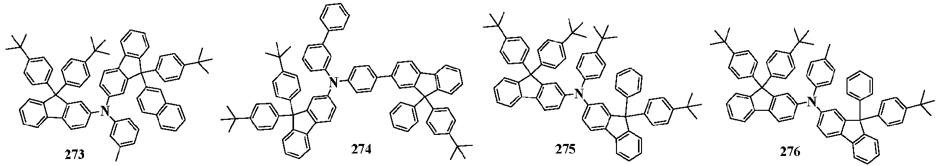

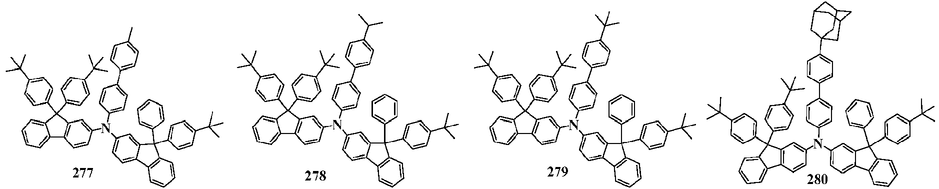

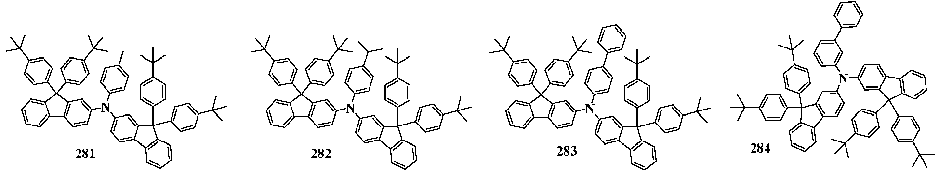

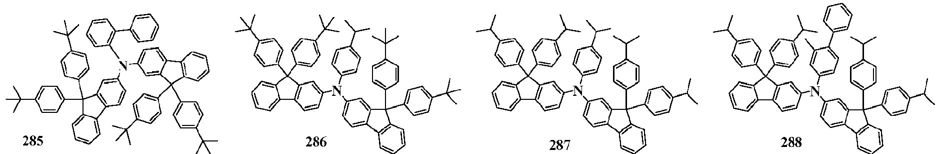

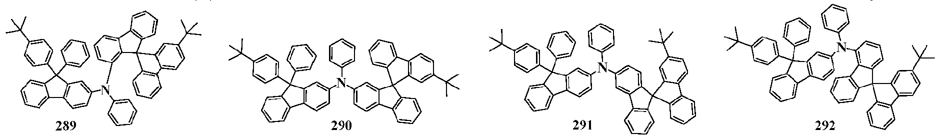

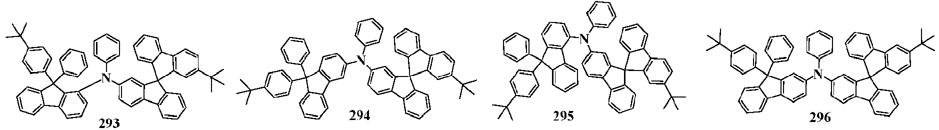

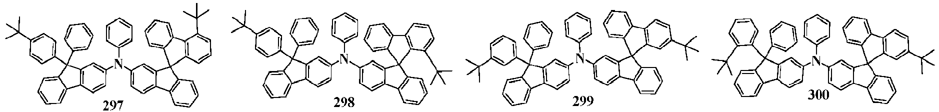

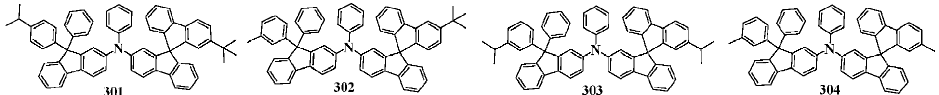

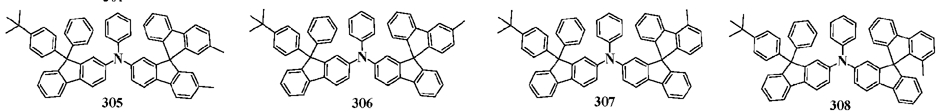

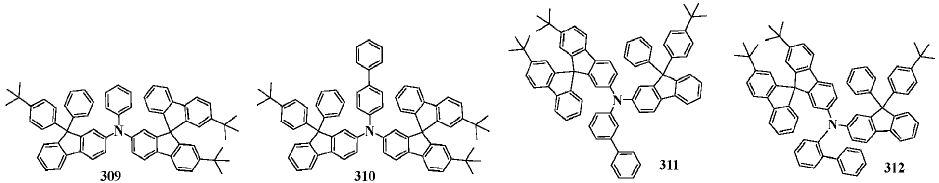

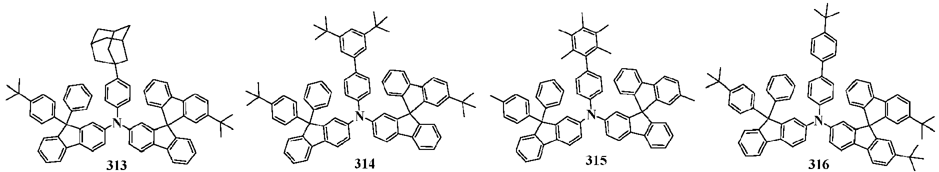

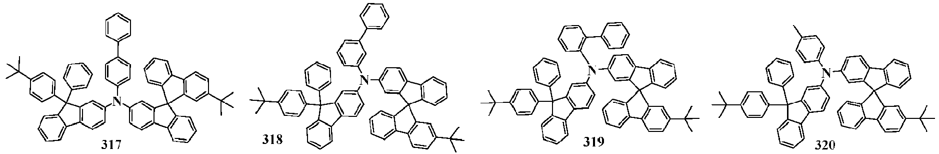

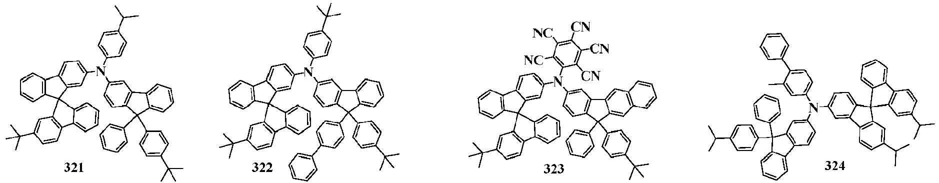

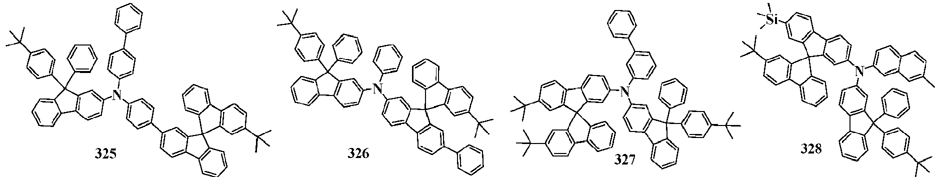

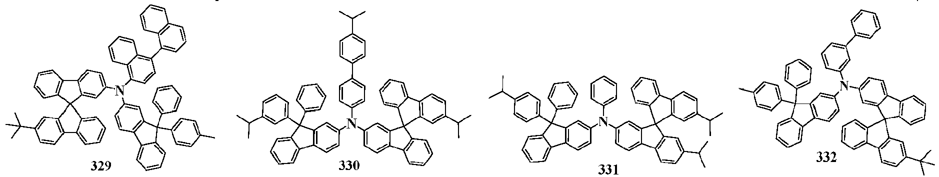

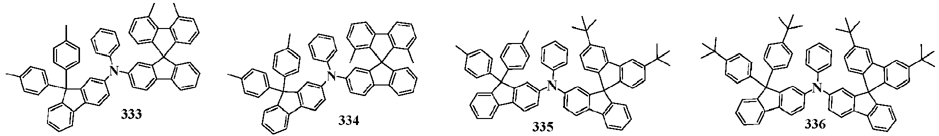

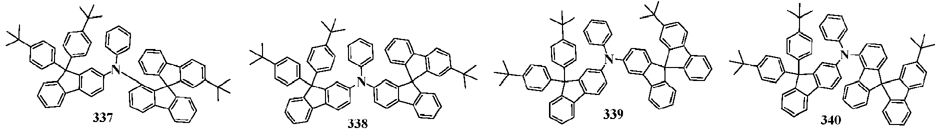

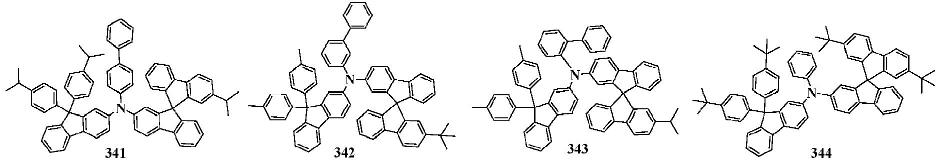

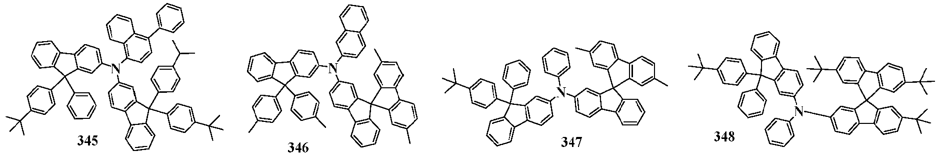

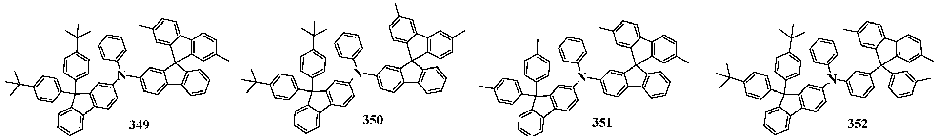

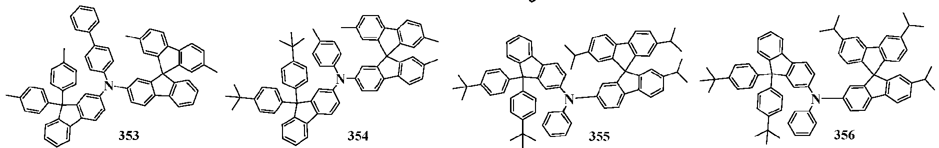

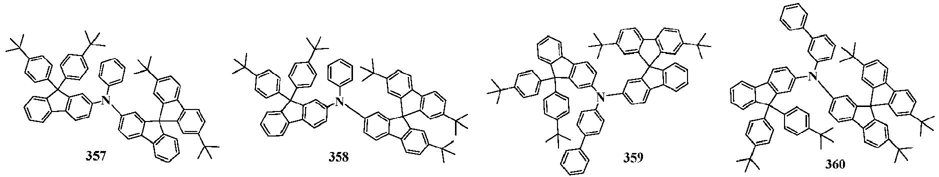

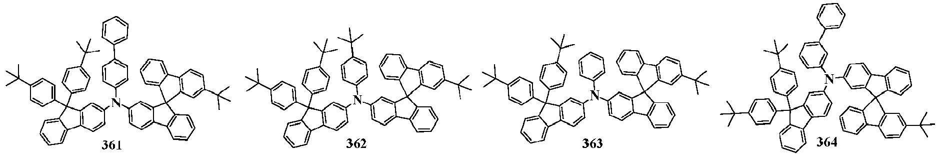

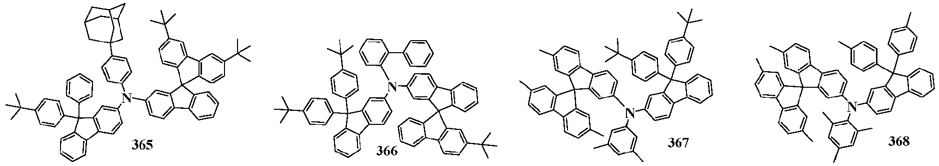

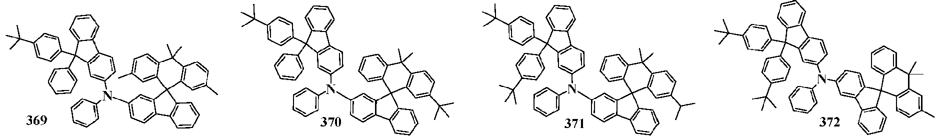

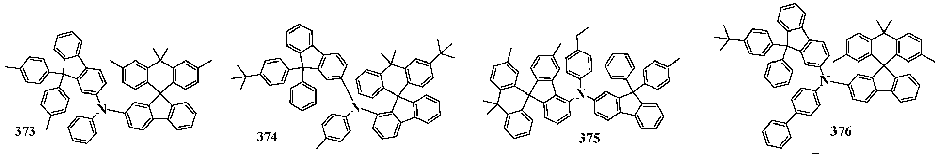

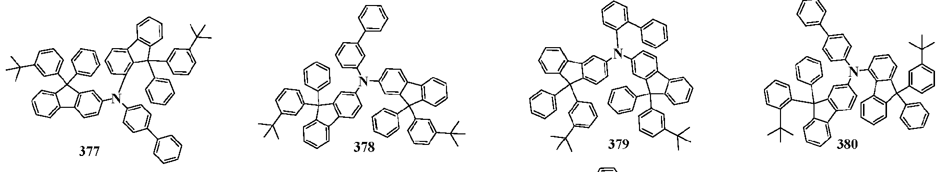

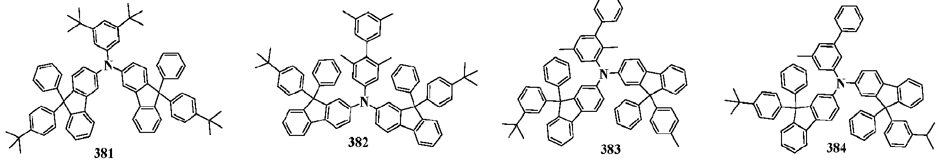

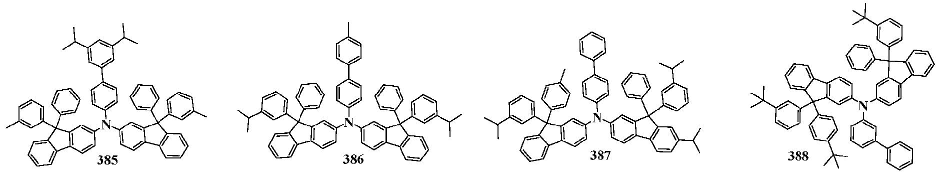

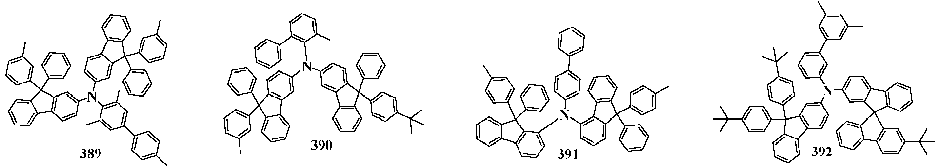

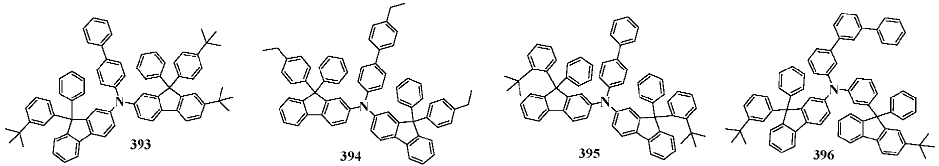

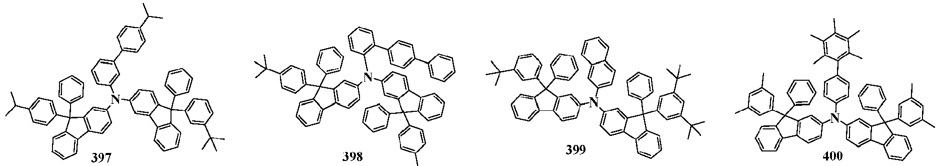

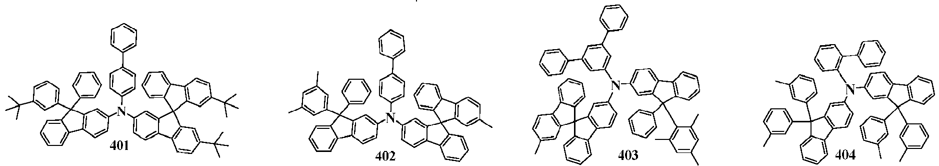

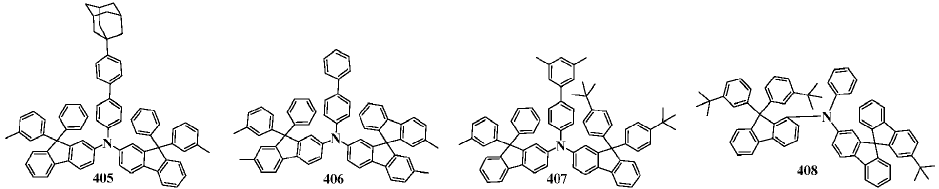

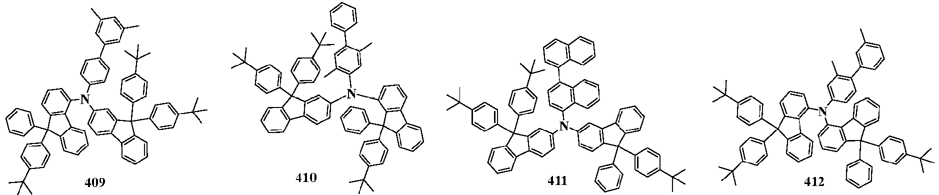

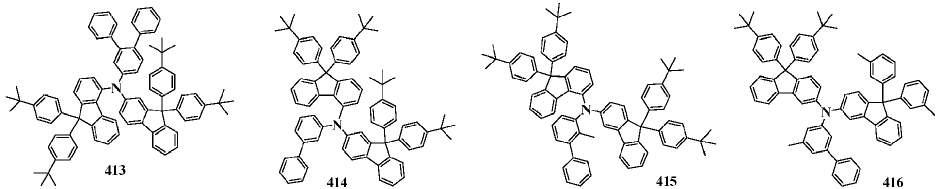

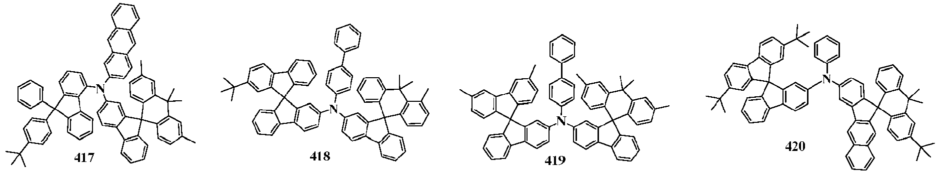

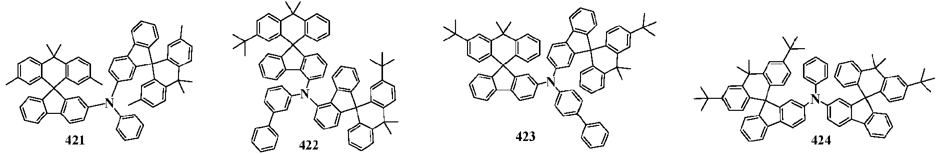

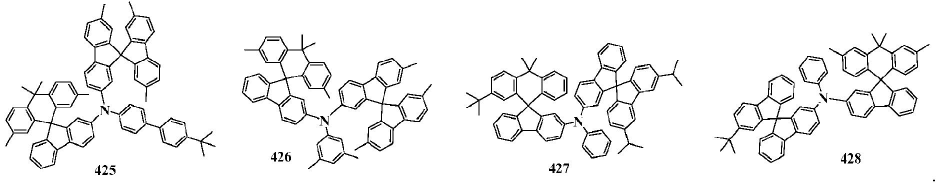

10. An organic electroluminescent device, comprising an anode, a cathode, and an organic layer, wherein the organic layer comprises the fluorene-containing triarylamine compound according to any one of claims 1 to 9.
